(19) Europäisches Patentamt
European Patent Office
Office européen des brevets

(11) Publication number: **0 580 932 A1**

# (12) EUROPEAN PATENT APPLICATION

(21) Application number: **93100536.7**

(22) Date of filing: **15.01.93**

(51) Int. Cl.[5]: **C07D 241/44**, C07D 401/12, C07D 403/12, C07D 409/12, C07D 411/12, C07D 417/12, A01N 43/60

(30) Priority: **29.07.92 PCT/US92/06270**

(43) Date of publication of application: **02.02.94 Bulletin 94/05**

(84) Designated Contracting States: **ES**

(71) Applicant: **E.I. DU PONT DE NEMOURS AND COMPANY**
**1007 Market Street**
**Wilmington Delaware 19898(US)**

(72) Inventor: **Fawzi, Maged Mohamed**
**17 Beech Hill Drive**
**Newark, Delaware 19711(US)**

(74) Representative: **Hildyard, Edward Martin et al**
**Frank B. Dehn & Co.**
**Imperial House**
**15-19 Kingsway**
**London WC2B 6UZ (GB)**

## (54) Herbicidal quinoxalinyloxy ethers.

(57) This invention relates to certain herbicidal quinoxalinyloxy compositions and a method for their use as a general herbicide or as a selective preemergent or postemergent herbicides for controlling the growth of undesired vegetation in crops.

EP 0 580 932 A1

## CROSS-REFERENCE TO RELATED APPLICATION

This application is a continuation-in-part of application U.S. Serial No. 07/850,730 filed March 13, 1992, which was a continutation-in-part of application U.S. Serial No. 07/739,001 filed August 1,1991.

## BACKGROUND OF THE INVENTION

This invention relates to herbicidal quinoxalinyloxy compounds, agriculturally suitable compositions thereof and a method for their use as selective preemergent or postemergent herbicides for controlling the growth of undesired vegetation.

New compounds effective for controlling the growth of undesired vegetation are in constant demand. In the most common situation, such compounds are sought to selectively control the growth of weeds in useful crops such as cotton, rice, corn, wheat and soybeans, to name a few. Unchecked weed growth in such crops can cause significant losses, reducing profit to the farmer and increasing costs to the consumer. In other situations, herbicides are desired which will control all plant growth. There are many products commercially available for these purposes, but the search continues for products which are more effective, less costly and environmentally safe.

EP-A-205,821 discloses herbicidal compounds of the formula:

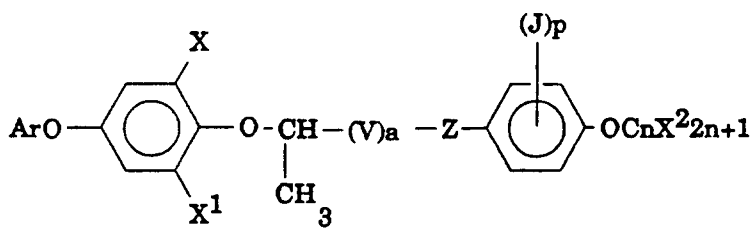

wherein, inter alia

$X^2$ is H, Br, Cl or F with the proviso that at least one $X^2$ is other than -H and that all $X^{2'}$s cannot be -Br or -Cl;

Z (in part) is

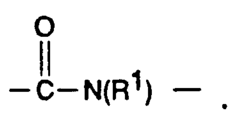

U.S. 4,968,343 discloses similar amides wherein the $OC_nX^2{}_{2n+1}$ group of EP-A-205,821 is replaced with $CF_2L$ wherein L is H, $CF_3$, Br, Cl or F.

EP-A-046,467 discloses herbicidal compounds including:

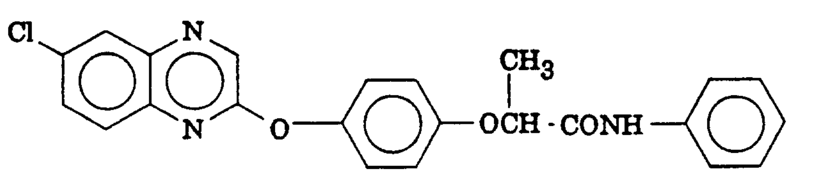

## SUMMARY OF THE INVENTION

The invention comprises novel compounds of Formula I, all optical isomers thereof, all stereoisomers in both racemic and enantiomerically pure form, agriculturally suitable compositions containing them, and their method-of-use as preemergent and/or postemergent herbicides

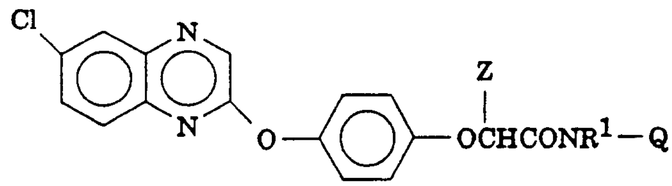

I

wherein:

Q is

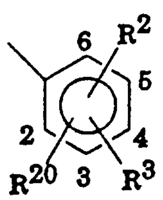 , 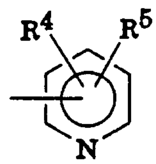 , 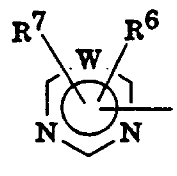 ,

Q-1 Q-2 Q-3

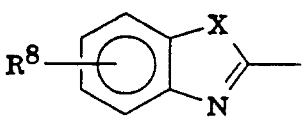 , 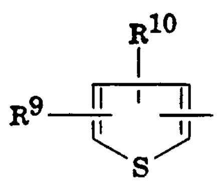 , 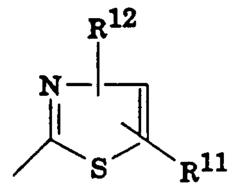 ,

Q-4 Q-5 Q-6

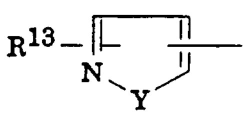 , 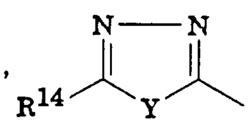 , 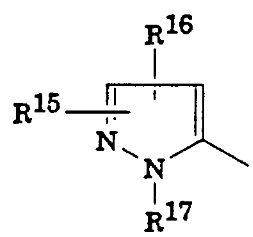 ,

Q-7 Q-8 Q-9

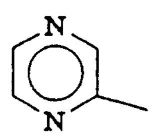 ,

Q-10

$R^1$ is H or $C_1$-$C_4$ alkyl;

$R^2$ is H; halogen; $C_1$-$C_4$ alkyl substituted with up to 3 Halogens; $C_1$-$C_3$ alkoxy; $C_1$-$C_4$ haloalkoxy; CN; $CO_2H$; $CO_2$($C_1$-$C_4$ alkyl);$S(O)_n$($C_1$-$C_3$ alkyl); O-phenyl; $NR^{18}R^{19}$; $SO_2N$-$(CH_3)_2$; or $CH_2CO_2$($C_1$-$C_4$ alkyl);

$R^3$ is H, halogen, $C_1$-$C_4$ alkyl substituted with up to 3 halogens, or $C_1$-$C_3$ alkoxy;

$R^4$ and $R^5$ are independently H; halogen; $C_1$-$C_4$ alkyl optionally substituted with up to 3 halogens; $C_1$-$C_4$ alkoxy; $C_1$-$C_4$ haloalkoxy; $CO_2H$; $CO_2$($C_1$-$C_4$ alkyl); CN; O-phenyl optionally substituted with up to 3 halogens or $CF_3$; or O-benzyl;

| | |
|---|---|
| $R^6$ and $R^7$ | are independently H, $C_1$-$C_4$ alkyl, $C_1$-$C_2$ thioalkyl, $C_1$-$C_3$ alkoxy, $NR^{18}R^{19}$; Cl or Br; |
| $R^8$ | is H, halogen, $C_1$-$C_4$ alkyl optionally substituted with up to 3 halogens, $C_1$-$C_4$ alkoxy, nitro or O-phenyl; |
| $R^9$ and $R^{10}$ | are independently H, $C_1$-$C_4$ alkyl, $CO_2(C_1$-$C_3$ alkyl), phenyl or taken together as $-(CH_2)_4-$ to form a six-membered ring; |
| $R^{11}$ and $R^{12}$ | are independently H; halogen; $C_1$-$C_4$ alkyl optionally substituted with up to 3 halogens or $CO_2(C_1$-$C_2$ alkyl); or phenyl optionally substituted with up to 3 halogens, $CH_3$ or $OCH_3$; |
| $R^{13}$ | is H, $C_1$-$C_4$ alkyl, Cl or Br; |
| $R^{14}$ | is H, $C_1$-$C_4$ alkyl optionally substituted with up to 3 halogens, $S(O)_n(C_1$-$C_4$ alkyl), $S(O)_n$benzyl or phenyl optionally substituted with up to 3 halogens, $CH_3$ or $OCH_3$; |
| $R^{15}$ and $R^{16}$ | are independently H, $C_1$-$C_4$ alkyl, Cl, Br or $CO_2(C_1$-$C_4$ alkyl); |
| $R^{17}$ | is H; $C_1$-$C_4$ alkyl; or phenyl optionally substituted with up to 3 halogens, $CH_3$ or $OCH_3$; |
| $R^{18}$ and $R^{19}$ | are independently H, $C_1$-$C_3$ alkyl or $C_1$-$C_3$ alkenyl; |
| $R^{18}$ and $R^{19}$ | may be taken together as $-(CH_2CH_2OCH_2CH_2)-$ or $-(CH_2)_5-$; |
| $R^{20}$ | is H or halogen; |
| W | is CH or N; |
| X | is O, S or NH; |
| Y | is O or S; |
| Z | is H or $CH_3$; |
| n | is 0, 1 or 2; |

provided that:

(a) when $R^2$ is haloalkoxy or $C_1$-$C_2$ alkyl substituted with up to 3 halogens then $R^2$ is in the 2- or 3-position;

(b) when Q is Q-1 and $R^1$ is H then one of $R^2$, $R^3$ or $R^{20}$ must be other than H;

(c) when Q is Q-1 and $R^3$ and $R^{20}$ are H then $R^2$ cannot be $CF_3$;

(d) when Q is Q-1 and $R^2$, $R^3$ or $R^{20}$ is Cl then the remaining substituent cannot be $OCH_3$;

(e) when Q is Q-1 and $R^3$ and $R^{20}$ are H, then $R^2$ cannot be $SCH_3$;

(f) when $R^3$ is $C_1$-$C_2$ alkyl substituted with up to 3 halogens then $R^3$ is in the 2- or 3-position; and

(g) when Q is Q-1 and $R^2$ and $R^{20}$ are H then $R^3$ cannot be $CF_3$.

In the above definitions, the term "alkyl", used either alone or in compound words such as "haloalkyl", includes straight chain or branched alkyl, e.g., methyl, ethyl, *n*-propyl, isopropyl or the different butyl isomers.

Alkoxy includes methoxy, ethoxy, *n*-propyloxy and isopropyloxy.

The term "halogen", either alone or in compound words such as "haloalkyl", means fluorine, chlorine, bromine or iodine. Further, when used in compound words such as "haloalkyl" said alkyl may be partially or fully substituted with halogen atoms, which may be the same or different. Examples of haloalkyl include $CH_2CH_2F$, $CF_2CF_3$ and $CH_2CHFCl$. The specific substituent positions on the phenyl ring of Q-1 is noted (carbons 2 through 6).

Preferred for reasons including ease of synthesis and/or greater herbicidal efficacy are:

1. Compounds of Formula I wherein the substituents defined above except

| | |
|---|---|
| $R^1$ | is H or $C_1$-$C_3$ alkyl; |
| $R^2$ | is H; F; Cl; Br; $C_1$-$C_2$ alkyl substituted with up to 3 halogens; $C_1$-$C_2$ alkoxy; $C_1$-$C_2$ haloalkoxy; CN; $S(O)_n$ ($C_1$-$C_2$ alkyl); $CO_2$ ($C_1$-$C_2$ alkyl); O-phenyl; $N(CH_3)_2$; or $SO_2N(CH_3)_2$; |
| $R^3$ | is H, F, Cl, Br, $CF_3$ or $C_1$-$C_2$ alkoxy; |
| $R^4$ and $R^5$ | are independently H, F, Cl, Br, $C_1$-$C_2$ alkyl, $CO_2$- ($C_1$-$C_2$ alkyl) or CN. |
| $R^6$ and $R^7$ | are independently H, $C_1$-$C_2$ alkyl $C_1$-$C_2$ alkoxy, Cl or Br; |
| $R^8$ | is H, F, Cl, or nitro; |
| $R^9$ and $R^{10}$ | are independently H, $C_1$-$C_2$ alkyl, $CO_2CH_3$, phenyl or taken together as $-(CH_2)_4-$ to form a 6-membered ring; |
| $R^{11}$ and $R^{12}$ | are independently H, F, Cl, $CF_3$ or phenyl optionally substituted with F, Cl, $CH_3$ or $OCH_3$; |
| $R^{13}$ | is H, $C_1$-$C_2$ alkyl, Cl or Br; |
| $R^{14}$ | is $C_1$-$C_4$ alkyl, $CF_3$ or $SCH_3$; |
| $R^{15}$ and $R^{16}$ | are independently H, $C_1$-$C_2$ alkyl, Cl or Br; |
| $R^{17}$ | is $C_1$-$C_4$ alkyl, $CF_3$ or phenyl optionally substituted with F or Cl. |

2. Compounds of Preferred 1 wherein

Q is Q-1, Q-2, Q-4, Q-6, Q-8 or Q-9.

3. Compounds of Preferred 2 wherein
   - $R^1$ is H or $CH_3$;
   - $R^2$ is H, F, Cl, Br, $CF_3$, $C_1$-$C_2$ alkoxy, $OCF_3$, $OCH_2CF_3$, $NO_2$, $S(O)_nCH_3$, $CO_2(C_1$-$C_2$ alkyl), O-phenyl or $N(CH_3)_2$;
   - $R^3$ is H, F, Cl or $OCH_3$;
   - $R^4$ is H, F, Cl, $CH_3$, $OCH_3$ or $CO_2(C_1$-$C_2$ alkyl);
   - $R^{14}$ is $C_1$-$C_4$ alkyl or $CF_3$.
4. Compounds of Preferred 3 wherein
   - Q is Q-1.

Specifically Preferred for reasons of greatest ease of synthesis and/or greater herbicidal efficacy are the compounds of Preferred 4 which are:

- (R)-2-[4-[(6-chloro-2-quinoxalinyl)oxy]phenoxy]-N-(2,4-difluorophenyl)propanamide;
- (R)-2-[4-[(6-chloro-2-quinoxalinyl)oxy]phenoxy]-N-(3,5-dichlorophenyl)propanamide;
- (R)-N-(4-chloro-2-fluorophenyl)-2-[4-[(6-chloro-2-quinoxalinyl)oxy]phenoxy]propanamide;
- (R)-2-[4-[(6-chloro-2-quinoxalinyl)oxy]phenoxy]-N-(3,4-dichlorophenyl)propanamide;
- (R)-2-[4-[(6-chloro-2-quinoxalinyl)oxy]phenoxy]-N-methyl-N-phenylpropanamide;
- 2-[4-[(6-chloro-2-quinoxalinyl)oxy]phenoxy]-N-(2-fluorophenyl)acetamide;
- N-(4-chloro-2-fluorophenyl)-2-[4-[(6-chloro-2-quinoxalinyl)oxy]phenoxy]acetamide;
- (R)-N-(4-bromophenyl)-2-[4-[(6-chloro-2-quinoxalinyl)oxy]phenoxy]propanamide;
- N-(4-bromo-2-fluorophenyl)-2-[4-(6-chloro-2-quinoxalinyl)oxy]phenoxy]propanamide; and
- N-(4-chloro-2-fluorophenyl)-2-[4-(6-chloro-2-quinoxalinyl)oxy]phenoxy]acetamide.

Compositions of the invention for controlling the growth of undesired vegetation comprise an effective amount of the compound of the invention and at least one of the following: surfactant, solid or liquid diluent.

The method of using the compounds and compositions of the invention for controlling the growth of undesired vegetation comprise applying to the locus to be protected an effective amount of a compound or composition of the invention. However with respect to the method of using the compounds of the invention for controlling the growth of undesired vegetation by application to a corn or rice crop, the compound or composition is as defined above except that $R^2$ additionally includes $C_1$-$C_4$ alkyl and $NO_2$, $R^3$ additionally includes $C_1$-$C_4$ alkyl with only certain provisoes included, that is compounds of the following formula for use in corn or rice:

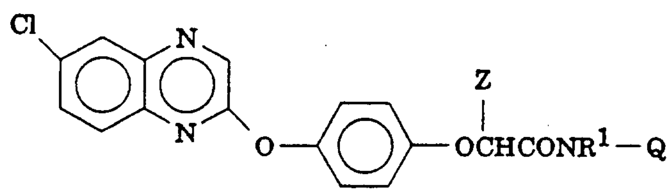

I

wherein:

Q is

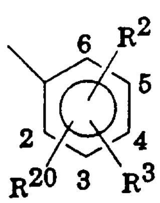
,
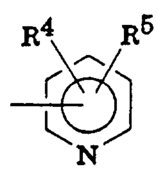
,
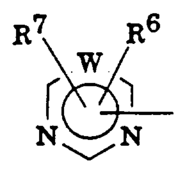
,

Q-1 Q-2 Q-3

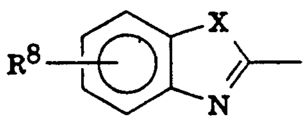 , 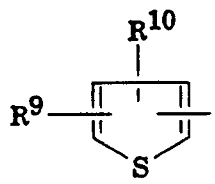 , 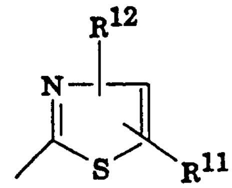 ,

Q-4 Q-5 Q-6

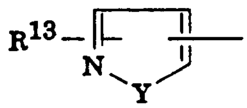 , 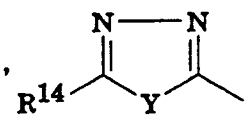 , 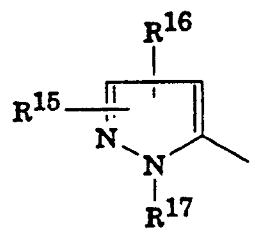 ,

Q-7 Q-8 Q-9

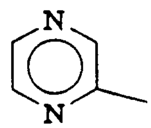 ,

Q-10

| | |
|---|---|
| $R^1$ | is H or $C_1$-$C_4$ alkyl; |
| $R^2$ | is H; halogen; $C_1$-$C_4$ alkyl optionally substituted with up to 3 halogens; $C_1$-$C_3$ alkoxy; $C_1$-$C_4$ haloalkoxy; CN; $NO_2$; $CO_2H$; $CO_2(C_1$-$C_4$ alkyl); $S(O)_n(C_1$-$C_3$ alkyl); O-phenyl; $NR^{18}R^{19}$; $SO_2N(CH_3)_2$; or $CH_2CO_2(C_1$-$C_4$ alkyl); |
| $R^3$ | is H, halogen, $C_1$-$C_4$ alkyl optionally substituted with up to 3 halogens, or $C_1$-$C_3$ alkoxy; |
| $R^4$ and $R^5$ | are independently H; halogen; $C_1$-$C_4$ alkyl optionally substituted with up to 3 halogens; $C_1$-$C_4$ alkoxy; $C_1$-$C_4$ haloalkoxy; $CO_2H$; $CO_2(C_1$-$C_4$ alkyl); CN; O-phenyl optionally substituted with up to 3 halogens or $CF_3$; or O-benzyl; |
| $R^6$ and $R^7$ | are independently H, $C_1$-$C_4$ alkyl, $C_1$-$C_2$ thioalkyl, $C_1$-$C_3$ alkoxy, $NR^{18}R^{19}$; Cl or Br; |
| $R^8$ | is H, halogen, $C_1$-$C_4$ alkyl optionally substituted with up to 3 halogens, $C_1$-$C_4$ alkoxy, nitro or O-phenyl; |
| $R^9$ and $R^{10}$ | are independently H, $C_1$-$C_4$ alkyl, $CO_2(C_1$-$C_3$ alkyl), phenyl or taken together as $-(CH_2)_4-$ to form a six-membered ring; |
| $R^{11}$ and $R^{12}$ | are independently H; halogen; $C_1$-$C_4$ alkyl optionally substituted with up to 3 halogens or $CO_2(C_1$-$C_2$ alkyl); or phenyl optionally substituted with up to 3 halogens, $CH_3$ or $OCH_3$; |
| $R^{13}$ | is H, $C_1$-$C_4$ alkyl, Cl or Br; |
| $R^{14}$ | is H, $C_1$-$C_4$ alkyl optionally substituted with up to 3 halogens, $S(O)_n(C_1$-$C_4$ alkyl), $S(O)_n$benzyl or phenyl optionally substituted with up to 3 halogens, $CH_3$ or $OCH_3$; |
| $R^{15}$ and $R^{16}$ | are independently H, $C_1$-$C_4$ alkyl, Cl, Br or $CO_2(C_1$-$C_4$ alkyl); |
| $R^{17}$ | is H; $C_1$-$C_4$ alkyl; or phenyl optionally substituted with up to 3 halogens, $CH_3$ or $OCH_3$; |
| $R^{18}$ and $R^{19}$ | are independently H, $C_1$-$C_3$ alkyl or $C_1$-$C_3$ alkenyl; |
| $R^{18}$ and $R^{19}$ | may be taken together as $-(CH_2CH_2OCH_2CH_2)-$ or $-(CH_2)_5-$; |
| $R^{20}$ | is H or halogen; |
| W | is CH or N; |
| X | is O, S or NH; |
| Y | is O or S; |
| Z | is H or $CH_3$; |
| n | is 0, 1 or 2; |

provided that:

(a) when $R^2$ is haloalkoxy or $C_1$-$C_2$ alkyl substituted with up to 3 halogens then $R^2$ is in the 2- or 3-position;

(b) when Q is Q-1 and $R^1$ is H then one of $R^2$, $R^3$ or $R^{20}$ must be other than H; and

(c) when $R^3$ is $C_1$-$C_2$ alkyl substituted with up to 3 halogens then $R^3$ is in the 2- or 3-position.

Preferred for reasons including greater herbicidal efficacy are:

1. A method for controlling the growth of undesired vegetation which comprises applying to the rice or corn crop a effective amount of a compound of Formula I wherein the substituents defined above except

| | |
|---|---|
| $R^1$ | is H or $C_1$-$C_3$ alkyl; |
| $R^2$ | is H; F; Cl; Br; $C_1$-$C_2$ alkyl optionally substituted with up to 3 halogens; $C_1$-$C_2$ alkoxy; $C_1$-$C_2$ haloalkoxy; CN; $NO_2$; $S(O)_n$($C_1$-$C_2$ alkyl); $CO_2$ ($C_1$-$C_2$ alkyl); O-phenyl; $N(CH_3)_2$; or $SO_2N(CH_3)_2$; |
| $R^3$ | is H, F, Cl, Br, $C_1$-$C_2$ alkyl, $CF_3$ or $C_1$-$C_2$ alkoxy; |
| $R^4$ and $R^5$ | are independently H, F, Cl, Br, $C_1$-$C_2$ alkyl, $CO_2$- ($C_1$-$C_2$ alkyl) or CN. |
| $R^6$ and $R^7$ | are independently H, $C_1$-$C_2$ alkyl, $C_1$-$C_2$ alkoxy, Cl or Br; |
| $R^8$ | is H, F, Cl, or nitro; |
| $R^9$ and $R^{10}$ | are independently H, $C_1$-$C_2$ alkyl, $CO_2CH_3$, phenyl or taken together as $-(CH_2)_4-$ to form a 6-membered ring; |
| $R^{11}$ and $R^{12}$ | are independently H, F, Cl, $CF_3$ or phenyl optionally substituted with F, Cl, $CH_3$ or $OCH_3$; |
| $R^{13}$ | is H, $C_1$-$C_2$ alkyl, Cl or Br; |
| $R^{14}$ | is $C_1$-$C_4$ alkyl, $CF_3$ or $SCH_3$; |
| $R^{15}$ and $R^{16}$ | are independently H, $C_1$-$C_2$ alkyl, Cl or Br; |
| $R^{17}$ | is $C_1$-$C_4$ alkyl, $CF_3$ or phenyl optionally substituted with F or Cl. |

2. A method of Preferred 1 wherein

Q is Q-1, Q-2, Q-4, Q-6, Q-8 or Q-9.

3. A method of Preferred 2 wherein

| | |
|---|---|
| $R^1$ | is H or $CH_3$; |
| $R^2$ | is H, F, Cl, Br, $CF_3$, $C_1$-$C_2$ alkoxy, $OCF_3$, $OCH_2CF_3$, $NO_2$, $S(O)_nCH_3$, $CO_2$($C_1$-$C_2$ alkyl), O-phenyl or $N(CH_3)_2$; |
| $R^3$ | is H, F, Cl $CH_3$ or $OCH_3$; |
| $R^4$ | is H, F, Cl, $CH_3$, $OCH_3$ or $CO_2$($C_1$-$C_2$ alkyl); |
| $R^{14}$ | is $C_1$-$C_4$ alkyl or $CF_3$. |

4. A method of Preferred 3 wherein

Q is Q-1.

Specifically Preferred for reasons of greatest herbicidal efficacy is the method of Preferred 4 which are:

- (R)-2-[4-[(6-chloro-2-quinoxalinyl)oxy]phenoxy]-N-(2,4-difluorophenyl)propanamide;
- (R)-2-[4-[(6-chloro-2-quinoxalinyl)oxy]phenoxy]-N-(3,5-dichlorophenyl)propanamide;
- (R)-N-(4-chloro-2-fluorophenyl)-2-[4-[(6-chloro-2-quinoxalinyl)oxy]phenoxy]propanamide;
- (R)-2-[4-[(6-chloro-2-quinoxalinyl)oxy]phenoxy]-N-(3,4-dichlorophenyl)propanamide;
- (R)-2-[4-[(6-chloro-2-quinoxalinyl)oxy]phenoxy]-N-methyl-N-phenylpropanamide;
- 2-[4-[(6-chloro-2-quinoxalinyl)oxy]phenoxy]-N-(2-fluorophenyl)acetamide;
- N-(4-chloro-2-fluorophenyl)-2-[4-[(6-chloro-2-quinoxalinyl)oxy]phenoxy]acetamide;
- (R)-N-(4-bromophenyl)-2-[4-[(6-chloro-2-quinoxalinyl)oxy]phenoxy]propanamide;
- (R)-N-(3-chloro-2-methylphenyl)-2-[4-(6-chloro-2-quinoxalinyl)oxy]phenoxy]propanamide;
- N-(4-bromo-2-fluorophenyl)-2-[4-(6-chloro-2-quinoxalinyl)oxy]phenoxy]propanamide; and
- N-(4-chloro-2-fluorophenyl)-2-[4-(6-chloro-2-quinoxalinyl)oxy]phenoxy]acetamide.

## DETAILED DESCRIPTION OF THE INVENTION

### Synthesis

The synthetic methods that are used to prepare the compounds of this invention are standard procedures that are known to those skilled in the art and details can be found in U.S. 4,968,343, U.S. 4,609,396, and references cited therein.

In general the compounds can be prepared by combining preferably in equimolar amounts the appropriate amine with 2-[4-(6-chloro-2-quinoxalinyloxy)phenoxy]propionyl chloride or 2-[4-(6-chloro-2-quinoxalinyloxy)phenoxy]acetyl chloride in a suitable solvent in the presence of an acid acceptor. (Scheme 1).

Scheme 1

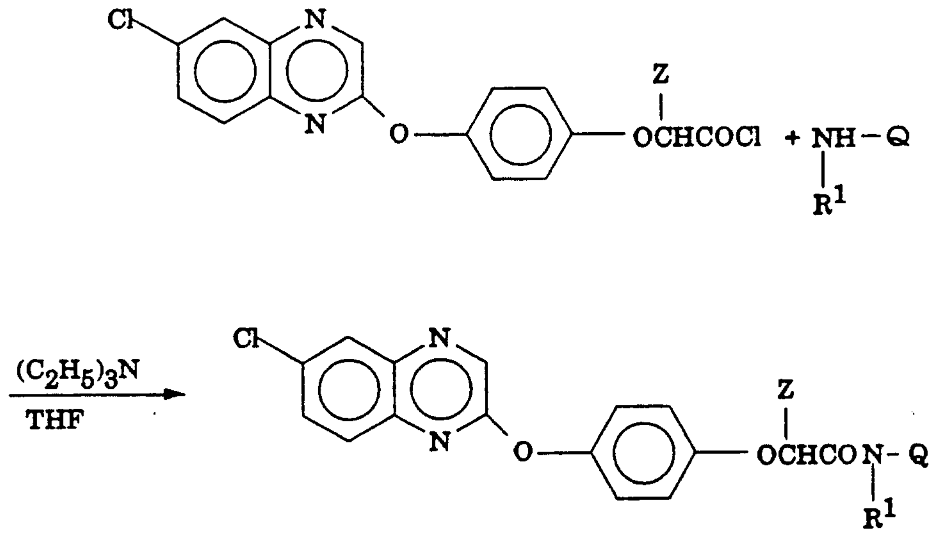


I

The reaction is preferably carried out at temperatures between 0 and 80 ° C. Suitable solvents for the reaction include tetrahydrofuran, acetonitrile and chloroform. In addition, the compounds can be synthesized by heating an equimolar mixture of 6-chloro-2-(4-hydroxyphenoxy)quinoxaline with the appropriate 2-haloamide in the presence of sodium hydride in dimethylformamide or preferably in the presence of potassium carbonate in 2-butanone (i.e., methyl ethyl ketone or MEK) or acetonitrile. For example:

Scheme 2

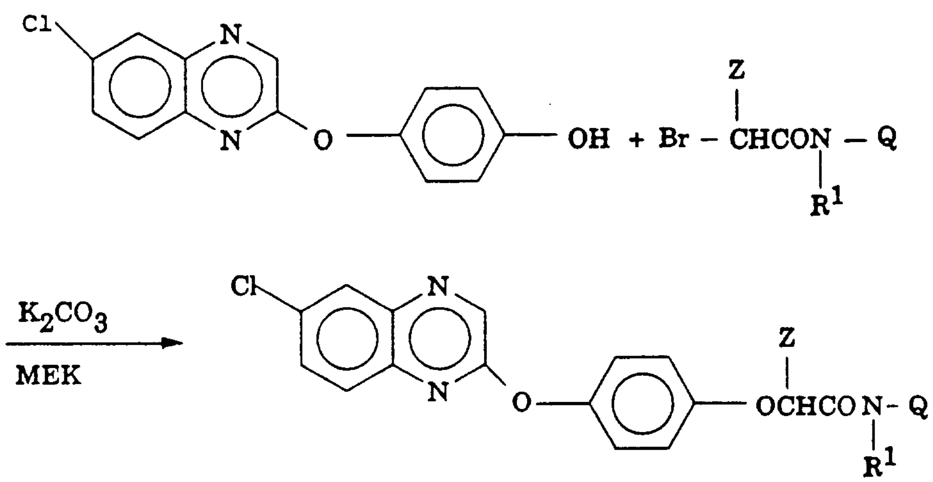


A third method (essentially a modification of the above method) can be utilized to prepare the desired compounds from 2,6-dichloroquinoxaline and the appropriate 2-(4-hydroxyphenoxy)propanamide or the acetamide in the presence of potassium carbonate in 2-butanone or dimethylformamide. For example:

Scheme 3

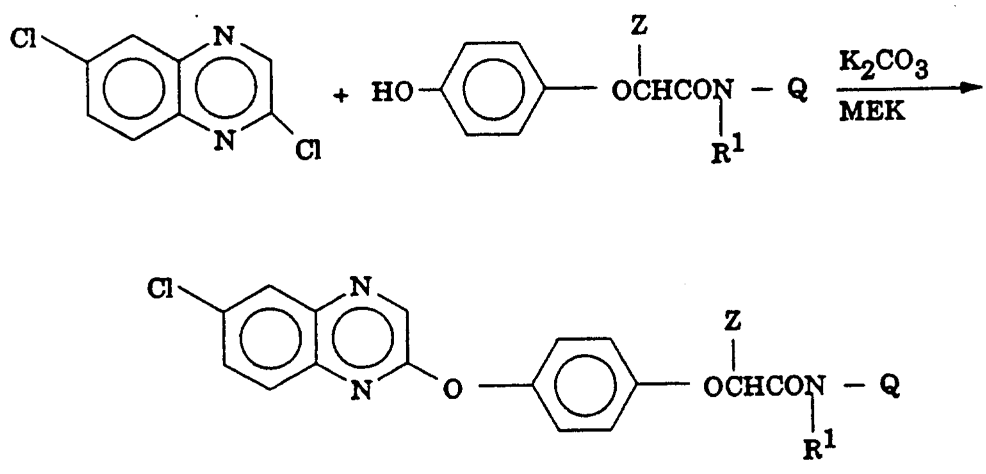


I

The following Examples illustrate the preparation of some of the compounds of the invention. They should not be regarded as limiting the invention in any way.

Example 1

2-[4-(6-chloro-2-quinoxalinyloxy)phenoxy]-N-(2,4-difluorophenyl)-propanamide

In a nitrogen atmosphere, a solution of 2.5 g (0.007 mol) 2-[4-(6-chloro-2-quinoxalinyloxy)phenoxy]-propionyl chloride in 8 cc tetrahydrofuran was rapidly added to a cold (0°C) solution of 1 g 2,4-difluoroaniline (0.0078 mol) and 1 g of triethylamine in 75 cc of tetrahydrofuran.

When the addition was complete, the reaction was stirred at ambient temperature for two hours.

The reaction mixture was cooled to 0°C and triethylamine hydrochloride was removed by filtration. Concentration of the filtrate gave the crude product which was purified by flash chromatography [silica gel 60, 230-400 mesh ASTM, E. Merck) with n-chlorobutane/ethylacetate (5:1) as elution solvent].

Crystallization from acetonitrile gave 1.72 g of the title compound m.p. 157-159°C.

NMR ($CDCl_3$) $\delta$ 1.71 (d, 3H), 4.85 (q, 1H), 6.8-7 (m, 2H), 7.07 (d. 2H), 7.25 (d, 2H), 7.60 (dd, 1H), 7.70 (d, 1H), 8.05 (d, 1H), 8.22-8.4 (m, 1H), 8.40 (s broad, 1H), 8.69 (s, 1H).

Example 2

2-[4-(6-chloro-2-quinoxalinyloxy)phenoxy]-N-(1,3-dimethyl-1H-pyrazol-5-yl)propanamide

The named compound was prepared from 2-[4-(6-chloro-2-quinoxalinyloxy)phenoxy]propionyl chloride and 5-amino-1,3-dimethylpyrazole by the method described in Example 1. The amide was crystallized from acetronitrile; and the resulting product had a m.p. 183-184°C and NMR ($CDCl_3$) $\delta$ 1.72 (d, 3H), 2.24 (s, 3H), 3.61 (s, 3H), 4.87 (q. 1H), 6.10 (s, 1H), 7.05 (d, 2H), 7.25 (d, 2H), 7.60 (dd, 1H), 7.70 (d, 1H), 8.00 (s broad, 1H), 8.05 (d, 1H), 8.70 (s, 1H).

Example 3

2-[4-(6-chloro-2-quinoxalinyl)oxy]phenoxyl-N-(2-fluorophenyl)acetamide

In a nitrogen atmosphere 1.5 g (0.011 mol) of finely powdered potassium carbonate was added to a solution of 1.9 g (0.007 mol) 6-chloro-2-(4-hydroxyphenoxy)quinoxaline and 1.3 g (0.007 mol) 2-chloro-N-(2-fluorophenyl)acetamide in 35 cc of 2-butanone. The mixture was stirred and heated under reflux overnight. The excess potassium carbonate and potassium chloride were removed by filtration and the filtrate was concentrated to give the crude product. Crystallization from n-chlorobutane gave 1.24 g of the title

compound; m.p. 158-161 °C and NMR ($CDCl_3$) $\delta$ 4.70 (s, 2H), 7.05-7.25 (m, 5H), 7.3 (d, 2H), 7.60 (dd, 1H), 7.70 (d, 1H), 8.05 (d, 1H), 8.38 (t, 1H), 8.65 (s broad, 1H), 8.70 (s, 1H).

Following the teachings of the above examples and schemes the compounds listed in the following tables can be made.

In the tables that follow the numbered compounds were not only prepared following the teachings of the above examples but were also tested for biological activity. The data of biological activity is present on subsequent pages.

**TABLE 1**

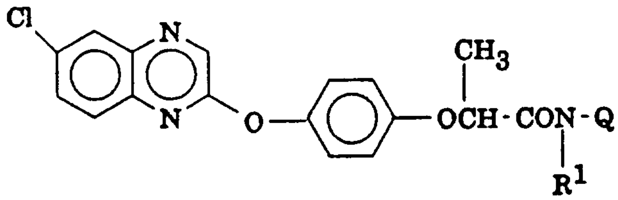

I

(R) isomer

| Compound Number | Q | $R^1$ | m.p. (°C) |
|---|---|---|---|
| | 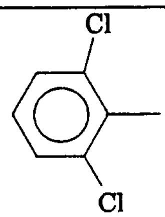 | H | |
| | 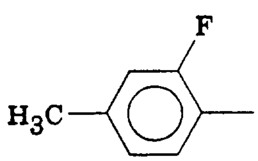 | $CH_3$- | |
| | 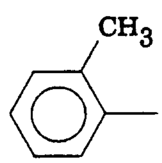 | $CH_3$- | |
| | 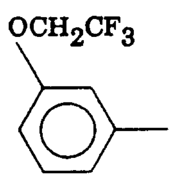 | H | |

| Compound Number | Q | $R^1$ | m.p. (°C) |
|---|---|---|---|
| | 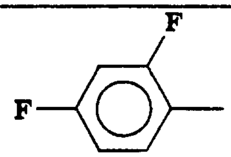 | $n-C_4H_9$ | |
| | 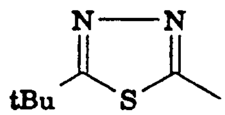 | $CH_3$- | |
| | 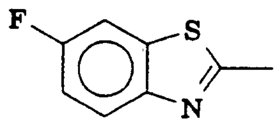 | $CH_3$- | |
| | 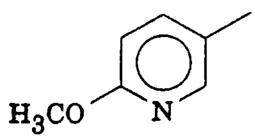 | $C_2H_5$- | |
| | 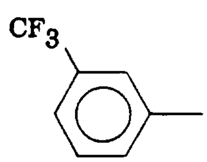 | $n-C_4H_9$ | |
| | 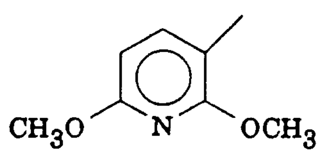 | H | |
| | 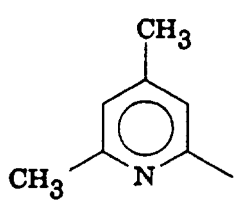 | H | |

| Compound Number | Q | $R^1$ | m.p. (°C) |
|---|---|---|---|
| | 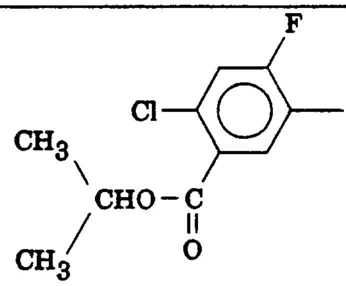 | H | 146-147 |
| | 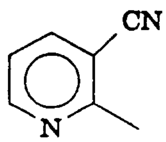 | n-$C_4H_9$ | |
| | 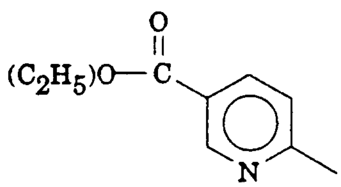 | H | |
| | 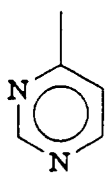 | H | |
| | 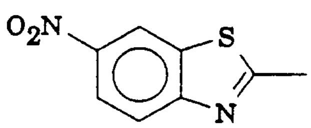 | H | |
| | 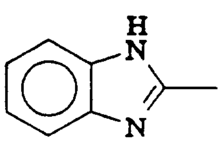 | H | |

| Compound Number | Q | $R^1$ | m.p. (°C) |
|---|---|---|---|
| | 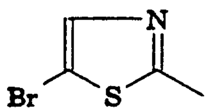 | H | |
| | 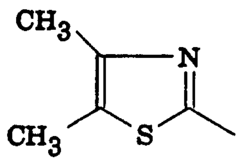 | H | |
| | 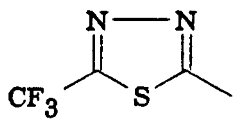 | $CH_3$ | |
| | 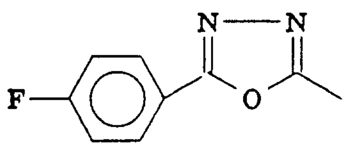 | H | |
| | 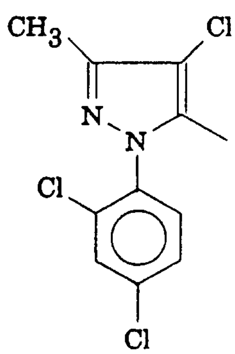 | H | |
| | 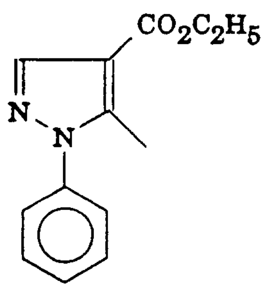 | H | |

| Compound Number | Q | $R^1$ | m.p. (°C) |
|---|---|---|---|
| 1 | 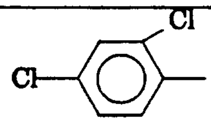 | H | 159-161 |
| 2 | 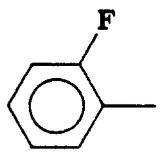 | H | 147-148 |
| 3 | 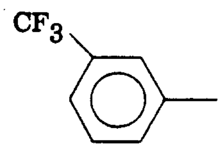 | H | 115-117 |
| 4 | 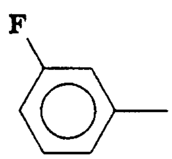 | H | 146-148 |
| 5 | 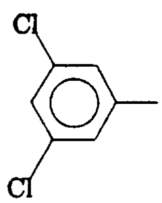 | H | 158-160 |
| 6 | 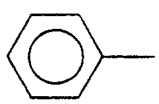 | $CH_3$- | Oil, NMR ($CDCl_3$) δ 1.5 (d, 3H), 3.32(s, 3H) 4.71 (q, 1H), 6.79 (d, 2H), 7.00-7.75 (m, 9H), 8.01 (d, 1H), 8.66 (s, 1H) |

| Compound Number | Q | $R^1$ | m.p. (°C) |
|---|---|---|---|
| 7 | 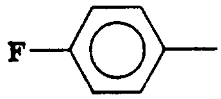 | H | 156-159 |
| 8 | 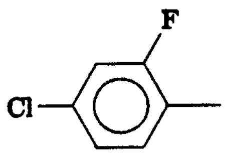 | H | 171-173 |
| 9 | 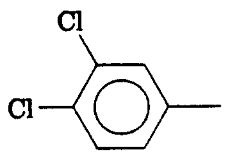 | H | 174-175 |
| 10 | 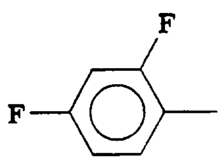 | H | 157-159 |
| 13 | 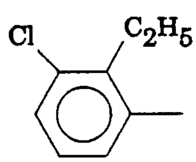 | H | 189-192 |
| 16 | 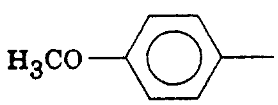 | $CH_3$- | Oil, NMR ($CDCl_3$) δ 1.49 (d, 3H), 3.28 (s, 3H) 3.82 (s, 3H), 4.7 (q, 1H) 6.82 (d, 2H), 6.91 (d, 2H) 7-7.2 (2d overlapping, 4H), 7.59 (dd, 1H), 7.7 (d, 1H), 8.05 (d, 1H), 8.67 (s, 1H) |

| Compound Number | Q | $R^1$ | m.p. (°C) |
|---|---|---|---|
| 18 | 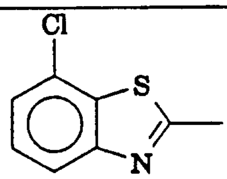 | H | 80-83 |
| 19 | 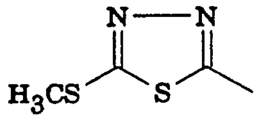 | H | 151-153 |
| 20 | 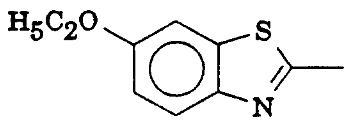 | H | 85-87 |
| 21 | 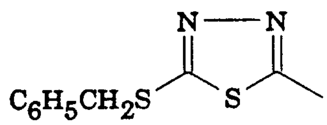 | H | 156-158 |
| 22 | 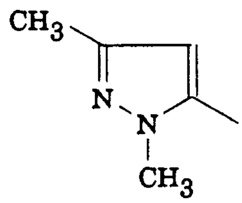 | H | 183-184 |
| 23 | 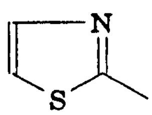 | H | 181-183 |
| 24 | 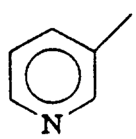 | H | 179-184 |

| Compound Number | Q | $R^1$ | m.p. (°C) |
|---|---|---|---|
| 25 | 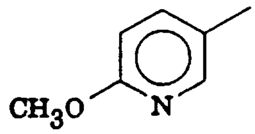 | H | 182-184 |
| 26 | 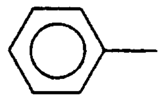 | $(CH_3)_2CH$- | Oil |
| 27 | 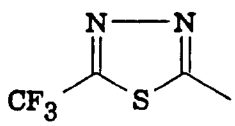 | H | 164-167 |
| 28 | 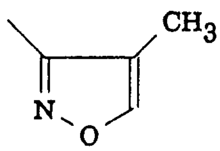 | H | 159-160 |
| 29 | 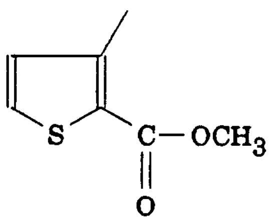 | H | 175-177 |
| 30 | 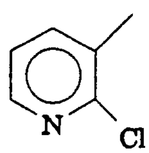 | H | 148-150 |
| 31 | 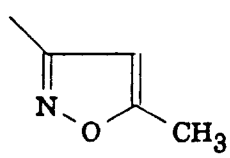 | H | 145-147 |

| Compound Number | Q | $R^1$ | m.p. (°C) |
|---|---|---|---|
| 32 | 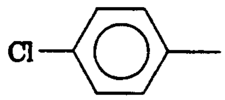 | $CH_3$ | Oil, NMR ($CDCl_3$) δ 1.50 (d, 3H), 3.29 (s, 3H), 4.7 (q, 1H), 6.78 (d, 2H), 7.05-7.2 (2d over-lapping, 4H), 7.28 (d, 2H), 7.6 (dd, 1H), 7.7 (d, 1H), 8.04 (d, 1H), 8.67 (s, 1H) |
| 33 | 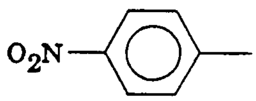 | $CH_3$ | Oil, NMR ($CDCl_3$) δ 1.59 (d, 3H), 3.85 (s, 3H), 4.81 (q, 1H), 6.75 (d, 2H), 7.14 (d, 2H), 7.33 (d, 2H), 7.60 (dd, 1H), 7.70 (d, 1H), 8.05 (d, 1H), 8.24 (d, 1H), 8.68 (s, 1H) |
| 34 | 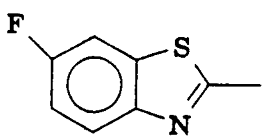 | H | 169-170 |
| 35 | 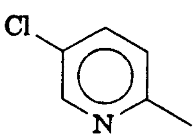 | H | 119-121 |

| Compound Number | Q | $R^1$ | m.p. (°C) |
|---|---|---|---|
| 36 | 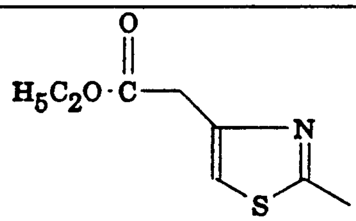 | H | Oil, NMR ($CDCl_3$) δ 1.28 (t, 3H), 1.70 (d, 3H), 3.71 (s, 2H), 4.20 (q, 2H), 4.9 (q, 1H), 6.86 (s, 1H), 7.01 (d, 2H), 7.23 (d, 2H), 7.6 (dd, 1H), 7.7 (d, 1H), 8.05 (d, 1H), 8.69 (s, 1H), 9.7 (s, 1H) |
| 37 | 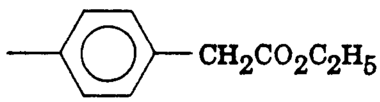 | H | 160-162 |
| 38 | 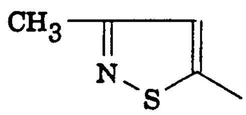 | H | 158-160 |
| 39 | 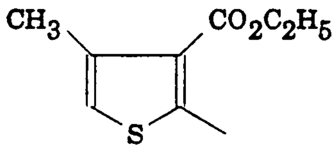 | H | 138-140 |
| 40 | 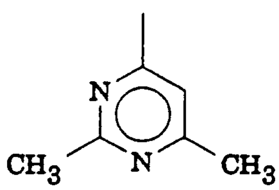 | H | 130-131 |
| 41 | 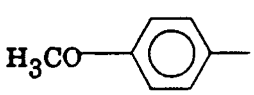 | H | 183-185 |

| Compound Number | Q | $R^1$ | m.p. (°C) |
|---|---|---|---|
| 42 | 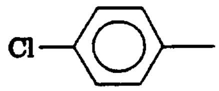 | H | 175-177 |
| 43 | 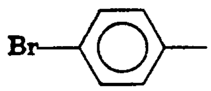 | H | 184-186 |
| 44 | 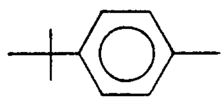 | H | 148-150 |
| 45 | 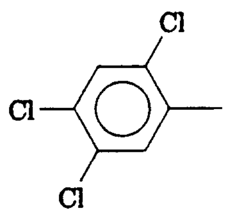 | H | 193-195 |
| 46 | 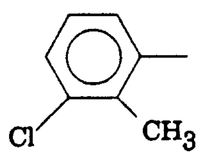 | H | 184-187 |
| 47 | 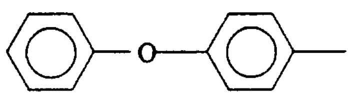 | H | 144-146 |
| 48 | 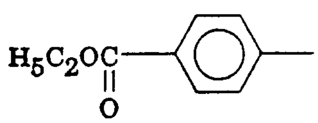 | H | 144-147 |

| Compound Number | Q | $R^1$ | m.p. (°C) |
|---|---|---|---|
| 49 | 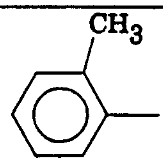 | H | 164-166 |
| 50 | 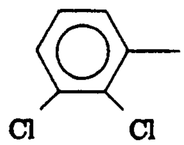 | H | 164-166 |
| 51 | 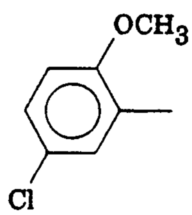 | H | 179-181 |
| 52 | 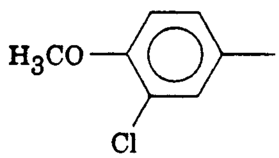 | H | 178-180 |
| 53 | 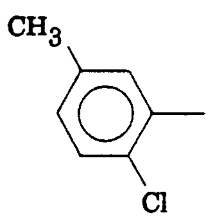 | H | 203-206 |
| 54 | 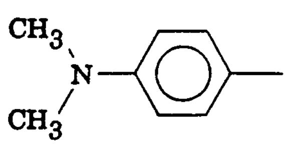 | H | 203-205 |

| Compound Number | Q | $R^1$ | m.p. (°C) |
|---|---|---|---|
| 55 | 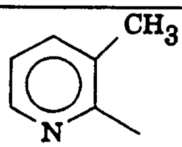 | H | 163-164 |
| 56 | 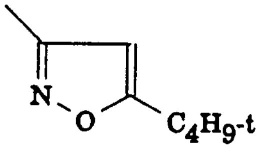 | H | 114-117 |
| 57 | 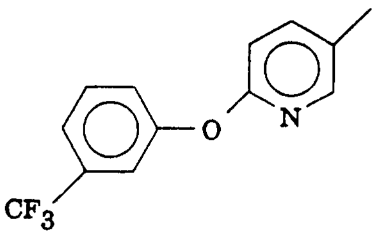 | H | 112-114 |
| 58 | 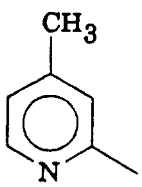 | H | 105-108 |
| 59 | 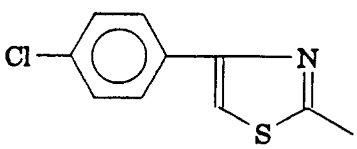 | H | 164-167 |
| 60 | 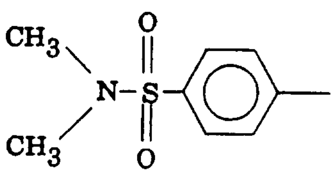 | H | 170-172 |

| Compound Number | Q | $R^1$ | m.p. (°C) |
|---|---|---|---|
| 61 | 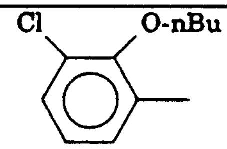 | H | 71-73 |
| 62 | 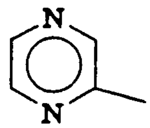 | H | 160-163 |
| 63 | 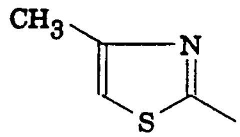 | H | Oil, NMR ($CDCl_3$) δ 1.7 (d, 3H), 2.36 (s, 1H), 4.9 (q, 1H), 6.59 (s, 1H), 7.01 (d, 2H), 7.24 (d, 2H), 7.6 (dd, 1H), 7.7 (d, 1H), 8.05 (d, 1H), 8.69 (s, 1H) |
| 64 | 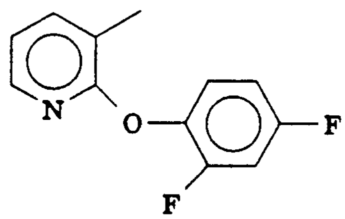 | H | 112-114 |
| 65 | 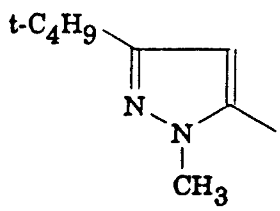 | H | 85-88 |
| 66 | 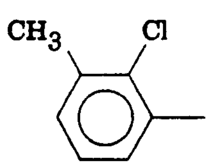 | H | 127-130 |

| Compound Number | Q | $R^1$ | m.p. (°C) |
|---|---|---|---|
| 67 | 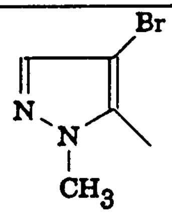 | H | 190-192 |
| 69 | 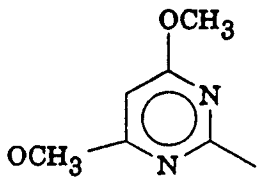 | H | 138-140 |
| 70 | 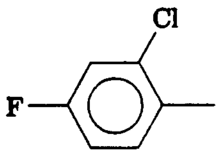 | H | 141-143 |
| 71 | 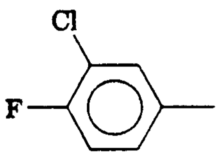 | H | 155-157 |
| 72 | 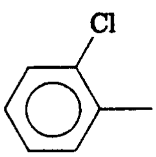 | H | 131-135 |
| 73 | 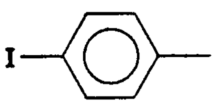 | H | 195-197 |

| Compound Number | Q | $R^1$ | m.p. (°C) |
|---|---|---|---|
| 74 | 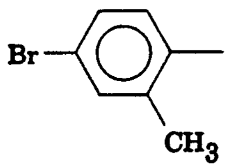 | H | 183-185 |
| 76 | 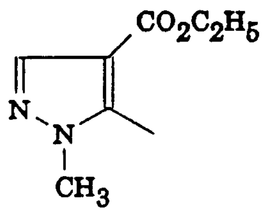 | H | 170-172 |
| 77 | 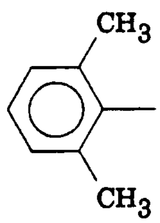 | H | 209-211 |
| 78 | 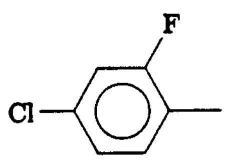 | $CH_3$ | 97-100 |
| 79 | 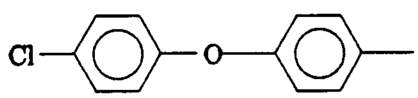 | H | |
| 80 | 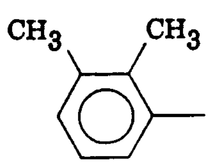 | H | 173-176 |

| Compound Number | Q | $R^1$ | m.p. (°C) |
|---|---|---|---|
| 81 | 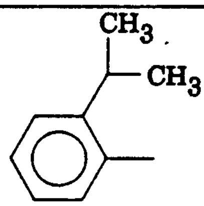 | H | 138-140 |
| 82 | 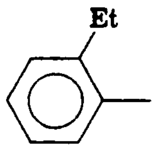 | H | 182-184 |
| 83 | 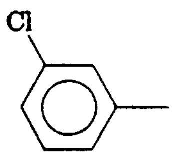 | H | 142-144 |
| 84 | 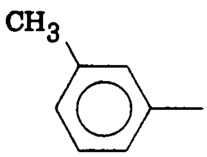 | H | 155-157 |
| 85 | 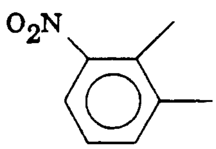 | H | 192-194 |
| 86 | 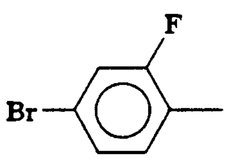 | H | 172-175 |

| Compound Number | Q | $R^1$ | m.p. (°C) |
|---|---|---|---|
| 87 | 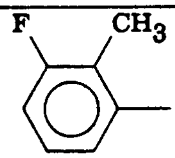 | H | 167-169 |
| 88 | 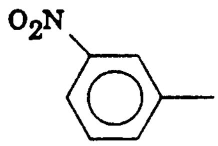 | H | 158-160 |
| 89 | 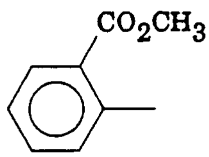 | H | 134-136 |
| 90 | 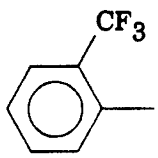 | H | 155-158 |
| 91 | 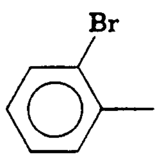 | H | 125-127 |
| 92 | 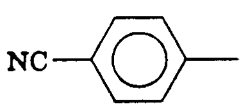 | H | 157-160 |
| 93 | 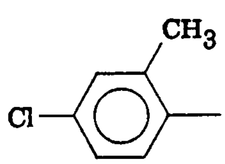 | H | 165-168 |

| Compound Number | Q | R1 | m.p. (°C) |
|---|---|---|---|
| 94 | 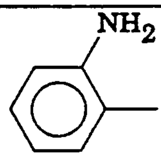 | H | 125-128 |
| 95 | 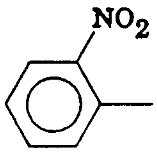 | H | 140-144 |
| 96 | 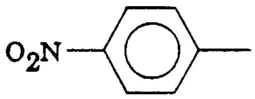 | H | 140-143 |
| 97 | 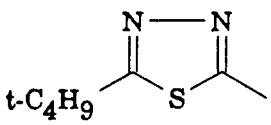 | H | 149-152 |
| 98 | 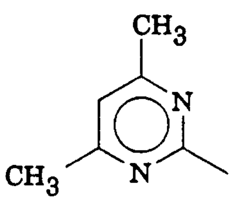 | H | 161-164 |
| 99 | 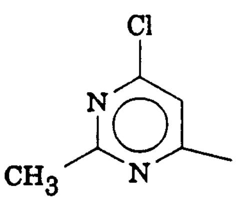 | H | 172-174 |

| Compound Number | Q | $R^1$ | m.p. (°C) |
|---|---|---|---|
| 100 | 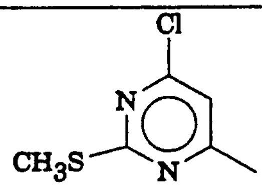 | H | Oil, NMR ($CDCl_3$) δ 1.68 (d, 2H), 2.55 (s, 3H), 4.80 (q, 1H), 7.13 (d, 2H), 7.25 (d, 2H), 7.60 (dd, 1H), 7.70 (d, 1H), 7.96 (s, 1H), 8.04 (d, 1H), 8.69 (s, 1H), 8.87 (s, broad, 1H) |
| 103 | 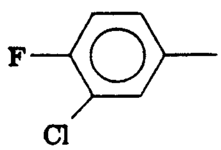 | $CH_3$ | 128-130 |
| 105 | 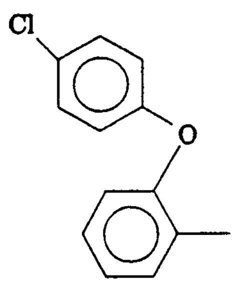 | H | 127-129 |
| 111 | 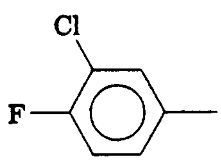 | 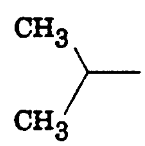 | Oil, NMR ($CDCl_3$) δ 1.06 (d, 6H), 1.48 (d, 3H), 4.4-4.6 (m, 1H), 4.9-5.1 (m, 1H), 6.76 (d, 2H), 6.9-7.3 (m, 5H), 7.6 (dd, 1H), 7.65 (d, 1H), 8,04 (d, 1H),8.68 (s, 1H) |

| Compound Number | Q | $R^1$ | m.p. (°C) |
|---|---|---|---|
| 112 | 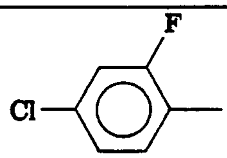 | 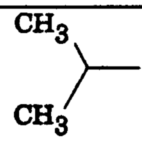 | 87-90 |
| 113 | 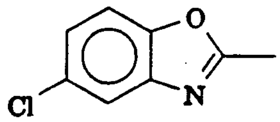 | H | 187-190 |
| 114 | 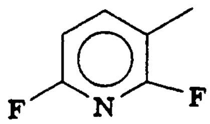 | H | 137-138 Dec. |
| 115 | 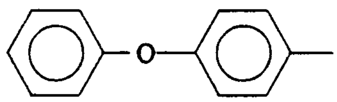 | $CH_3$ | Oil, NMR ($CDCl_3$) δ 1.5 (d, 2H), 3.31 (s, 3H), 4.75 (q, 1H), 6.80 (d, 2H), 6.90-7.40 (m, 1H), 7.50 (dd, 1H), 7.62 (d, 1H), 8.2 (d, 1H), 8.65 (s, 1H) |
| 116 | 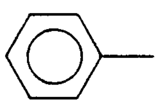 | n-$C_4H_9$- | Oil, NMR ($CDCl_3$) δ 0.89 (t, 3H), 1.42-1.7 (d+m overlapping, 5H), 3.6-3.82 (m, 2H), 4.62 (q, 1H), 6.8 (d, 2H), 7.05-7.5 (m, 7H), 7.60 (dd, 1H), 7.64 (d, 1H), 8.04 (d, 1H), 8.66 (s, 1H) |
| 117 | 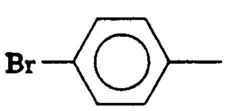 | $CH_3$ | 93-95 |

| Compound Number | Q | $R^1$ | m.p. (°C) |
|---|---|---|---|
| 118 | 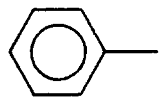 | $C_2H_5$- | Oil, NMR ($CDCl_3$) δ 1.14 (t, 3H), 1.48 (d, 3H), 3.62-3.95 (m, 2H), 4.6 (q, 1H), 6.80 (d, 2H), 7.1-7.5 (m, 7H), 7.59 (dd, 1H), 7.65 (d, 1H), 8.02 (d, 1H), 8.66 (1H) |
| 119 | 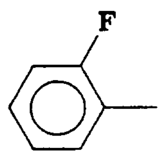 | 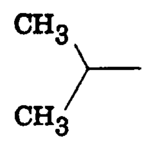 | Oil |
| 120 | 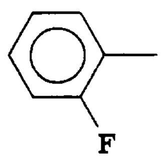 | $CH_3$ | Oil |
| 121 | 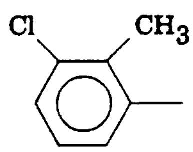 | $CH_3$ | Oil |
| 122 | 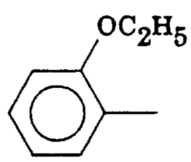 | H | 165-168 |
| 123 | 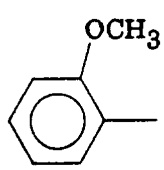 | H | 137-140 |

| Compound Number | Q | $R^1$ | m.p. (°C) |
|---|---|---|---|
| 124 | 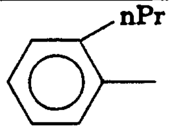 | H | 159-162 |
| 126 | 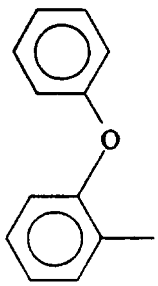 | H | 63-65 |
| 128 | 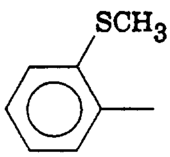 | H | 151-152 |
| 129 | 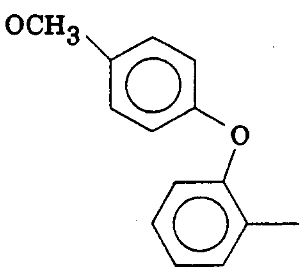 | H | 141-144 |

## TABLE 2

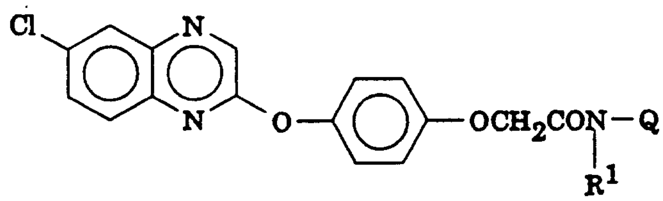

| Compound Number | Q | $R^1$ | m.p. (°C) |
|---|---|---|---|
| 11 | 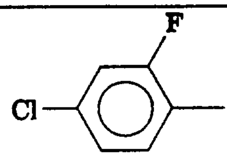 | H | 139.5-140 |
| 12 | 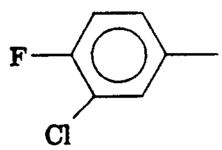 | H | 170-172 |
| 101 | 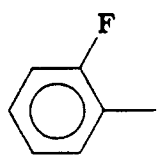 | H | 158-161 |
| 102 | 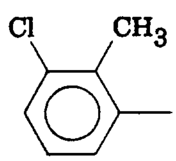 | H | 166-168 |
| 104 | 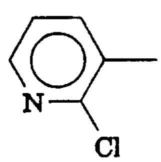 | H | 182-184 |

| Compound Number | Q | $R^1$ | m.p. (°C) |
|---|---|---|---|
| 106 | 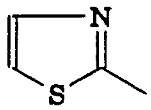 | H | 211-213 |
| 107 | 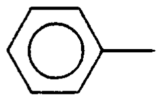 | $(CH_3)_2CH$- | 156-158 |
| 108 | 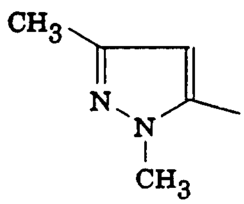 | H | 190-193 |
| 109 | 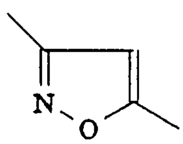 | H | 199-200 |
| 110 | 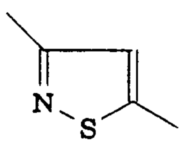 | H | 168-170 |
| | 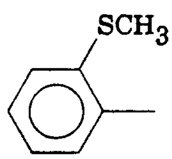 | H | 119-122 |
| | 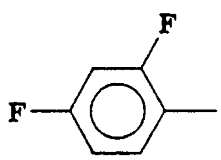 | H | 164-166 |

| Compound Number | Q | $R^1$ | m.p. (°C) |
|---|---|---|---|
| | 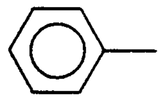 | H | 183-184 |
| | 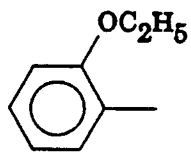 | H | 131-132 |
| | 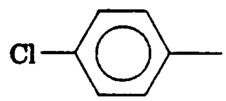 | H | 162-164 |
| | 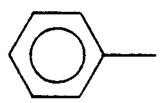 | $CH_3$ | 123-125 |
| | 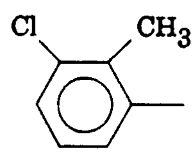 | H | 189-192 |
| | 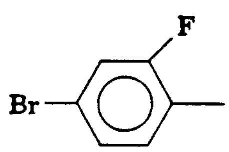 | H | 164-165 |

| Compound Number | Q | $R^1$ | m.p. (°C) |
|---|---|---|---|
| | 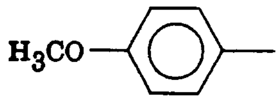 | H | 194-195 |
| | 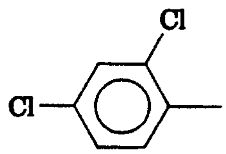 | H | 181-183 |
| | 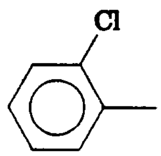 | H | 189-190 |
| | 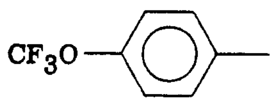 | H | 146-148 |

Formulations

The method of this invention can be conveniently carried out by formulating the compounds of Formula I in the conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The herbicidal formulations of the invention comprise 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

*Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0 °C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Hand- book", 5th Ed., Blackwell Scientific Publications, Oxford, 1968, pp. 101-103.

In the following Examples, all parts are by weight unless otherwise indicated.

Example A

Wettable Powder

| | |
|---|---|
| (R)-2-[4-[(6-chloro-2-quinoxalinyl)oxy]phenoxy]-N-(2,4-difluorophenyl)propanamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example B

Granule

| | |
|---|---|
| Wettable Powder of Example A | 5% |
| attapulgite granules (U.S.S. 20 to 40 mesh; 0.84-0.42 mm) | 95% |

A slurry of wettable powder containing 25% solids is sprayed on the surface of attapulgite granules in a double-cone blender. The granules are dried and packaged.

Example C

Extruded Pellet

| | |
|---|---|
| (R)-2-[4-[(6-chloro-2-quinoxalinyl)oxy]phenoxy]-N-(2,4-difluorophenyl)propanamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example D

Low Strength Granule

| | |
|---|---|
| (R)-2-[4-[(6-chloro-2-quinoxalinyl)oxy]phenoxy]-N-(2,4-difluorophenyl)propanamide | 0.1% |
| attapulgite granules (U.S.S. 20 to 40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example E

Low Strength Granule

| | |
|---|---|
| (R)-2-[4-[(6-chloro-2-quinoxalinyl)oxy]phenoxy]-N-(2,4-difluorophenyl)propanamide | 1% |
| N,N-dimethylformamide | 9% |
| attapulgite granules (U.S.S. 20 to 40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example F

Wettable Powder

| | |
|---|---|
| (R)-2-[4-[(6-chloro-2-quinoxalinyl)oxy]phenoxy]-N-(2,4-difluorophenyl)propanamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example G

Emulsifiable Concentrate

| | |
|---|---|
| (R)-2-[4-[(6-chloro-2-quinoxalinyl)oxy]phenoxy]-N-(2,4-difluorophenyl)propanamide | 35% |
| blend of polyalcohol carboxylic esters and oil soluble petroleum sulfonates | 6% |
| xylene | 59% |

The ingredients are combined and filtered to remove undissolved solids. The product can be used directly, extended with oils, or emulsified in water.

Example H

Dust

| | |
|---|---|
| (R)-2-[4-[(6-chloro-2-quinoxalinyl)oxy]phenoxy]-N-(2,4-difluorophenyl)propanamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example I

Wettable Powder

| | |
|---|---|
| (R)-2-[4-[(6-chloro-2-quinoxalinyl)oxy]phenoxy]-N-(2,4-difluorophenyl)propanamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example J

Ingredient

| | |
|---|---|
| (R)-2-[4-[(6-chloro-2-quinoxalinyl)oxy]phenoxy]-N-(2,4-difluorophenyl)propanamide | 48.8% |
| Altox* 3404F - anionic/nonionic emulsifier | 2.4% |
| Atlox* 3455F - anionic/nonionic emulsifier | 5.6% |
| Aromatic 100 (Exxon) - xylene range solvent | 43.2% |

The composition was prepared by mixing the ingredients together in a suitable vessel until complete dissolution of all the components occurred.

UTILITY

Test results indicate compounds of this invention are active postemergence and preemergence herbicides. Many compounds in this invention are useful for the control of selected grass weeds with tolerance to important agronomic crops such as corn (Zea mays), cotton (Gossypium hirsutum), rape (Brassica napus), rice (Oryza sativa), soybean (Glycine max), sugar beet (Beta vulgaris) and to vegetable crops. Grass weed species controlled include, but are not limited to, barnyardgrass (Echinochloa crus-galli), bermudagrass (Cynodon dactylon), blackgrass (Alopecurus myosuroides), crabgrass (Digitaria spp.), foxtail (Setaria spp.) and goosegrass (Potentilla ansurina). In Table D many compounds gave outstanding barnyardgrass control in paddy rice including (but not limited to) compounds 3, 6, 11, 30, 31, 43, 46 and 101. Several compounds of this invention are particularly useful to control grass weeds in corn. For example, compounds 46, 71 and 86 demonstrated excellent grass weed control in corn in Table F.

These compounds also have utility for weed control of selected vegetation in specified areas such as around storage tanks, parking lots, highways, and railways; in fallow crop areas; and in citrus and plantation crops such as banana, coffee, oil palm, and rubber. Alternatively, these compounds are useful to modify plant growth.

Effective rates of application for compounds of this invention are determined by a number of factors. These factors include: formulation selected, method of application, amount and type of vegetation present, growing conditions, etc. In general, the effective application rates for the compounds of the invention are from 0.02 to 20 kg/ha with a preferred rate range of 0.03 to 1 kg/ha. One skilled in the art can easily determine effective rates necessary for desired level of weed control.

Compounds of this invention may be used alone or in combination with other commercial herbicides, insecticides, or fungicides. The following list exemplifies some of the herbicides suitable for use in mixtures. A combination of a compound from this invention with one or more of the following herbicides may be particularly useful for weed control.

| Common Name | Chemical Name |
|---|---|
| acetochlor | 2-chloro-N-(ethoxymethyl)-N-(2-ethyl-6-methyl-phenyl)acetamide |
| acifluorfen | 5-[2-chloro-4-(trifluoromethyl)phenoxy]-2-nitro-benzoic acid |
| aclonifen | 2-chloro-6-nitro-3-phenoxybenzenamine |
| acrolein | 2-propenal |
| alachlor | 2-chloro-N-(2,6-diethylphenyl)-N-(methoxymethyl)-acetamide |
| alloxydim | methyl 2,2-dimethyl-4,6-dioxo-5-[1-[(2-propenyl-oxy)amino]butylidene] cyclohexanecarboxylate |
| ametryn | N-ethyl-N'-(1-methylethyl)-6-(methylthio)-1,3,5-triazine-2,4-diamine |
| amitrole | 1H-1,2,4-triazol-3-amine |
| AMS | ammonium sulfamate |
| anilofos | S-[2-[(4-chlorophenyl)(1-methylethyl)amino]-2-oxoethyl] O,O-dimethyl phosphorodithioate |
| asulam | methyl [(4-aminophenyl)sulfonyl]carbamate |
| atrazine | 6-chloro-N-ethyl-N'-(1-methylethyl)-1,3,5-triazine-2,4-diamine |
| aziprotryne | 4-azido-N-(1-methylethyl)-6-methylthio-1,3,5-triazin-2-amine |
| azoluron | N-(1-ethyl-1H-pyrazol-5-yl)-N'-phenylurea |
| barban | 4-chloro-2-butynyl 3-chlorocarbamate |

| Common Name | Chemical Name |
|---|---|
| benazolin | 4-chloro-2-oxo-3(2H)-benzothiazoleacetic acid |
| benfluralin | N-butyl-N-ethyl-2,6-dinitro-4-(trifluoromethyl)-benzenamine |
| bensulfuron | 2-[[[[[(4,6-dimethoxy-2-pyrimidinyl)amino]methyl-carbonyl]amino]sulfonyl]methyl]benzoic acid, methyl ester |
| bensulide | O,O-bis(1-methylethyl) S-[2-[(phenylsulfonyl)-amino]ethyl]phosphorodithioate |
| bentazon | 3-(1-methylethyl)-(1H)-2,1,3-benzothiadiazin-4(3H)-one,2,2-dioxide |
| benzofluor | N-[4-(ethylthio)-2-(trifluoromethyl)phenyl]-methanesulfonamide |
| benzoylprop | N-benzoyl-N-(3,4-dichlorophenyl)-DL-alanine |
| benzthiazuron | N-2-benzothiazolyl-N'-methylurea |
| bialaphos | 4-(hydroxymethylphosphinyl)-L-2-aminobutanoyl-L-alanyl-L-alanine |
| bifenox | methyl 5-(2,4-dichlorophenoxy)-2-nitrobenzoate |
| bromacil | 5-bromo-6-methyl-3-(1-methylpropyl)-2,4(1H,3H)-pyrimidinedione |
| bromobutide | (+)2-bromo-3,3-dimethyl-N-(1-methyl-1-phenyl-ethyl)butanamide |
| bromofenoxim | 3,5-dibromo-4-hydroxybenzaldhyde O-(2,4-dinitro-phenyl)oxime |
| bromoxynil | 3,5-dibromo-4-hydroxybenzonitrile |

| Common Name | Chemical Name |
|---|---|
| bromuron | N'-(4-bromophenyl)-N,N-dimethylurea |
| buminafos | dibutyl [1-(butylamino)cyclohexyl]phosphonate |
| butachlor | N-(butoxymethyl)-2-chloro-N-(2,6-diethylphenyl)-acetamide |
| butamifos | O-ethyl O-(5-methyl-2-nitrophenyl)-(1methyl-propyl)phosphoramidothioate |
| buthidazole | 3-[5-(1,1-dimethylethyl)-1,3,4-thiadiazol-2-yl]-4-hydroxy-1-methyl-2-imidazolidinone |
| butralin | 4-(1,1-dimethylethyl)-N-(1-methylpropyl)-2,6-dinitrobenzenamine |
| butylate | S-ethyl bis(2-methylpropyl)carbamothioate |
| cacodylic | dimethyl arsinic oxide acid |
| carbetamide | (R)-N-ethyl-2-[[(phenylamino)carbonyl]oxy]-propanamide |
| CDAA | 2-chloro-N,N-di-2-propenylacetamide |
| CDEC | 2-chloroallyl diethyldithiocarbamate |
| chlomethoxyfen | 4-(2,4-dichlorophenoxy)-2-methoxy-1-nitrobenzene |
| chloramben | 3-amino-2,5-dichlorobenzoic acid |
| chlorbromuron | 3-(4-bromo-3-chlorophenyl)-1-methoxy-1-methyl-urea |
| chlorbufam | 1-methyl-2-propynl(3-chlorophenyl)carbamate |
| chlorfenac | 2,3,6-trichlorobenzeneacetic acid |

| Common Name | Chemical Name |
|---|---|
| chlorflurecolmethyl | methyl 2-chloro-9-hydroxy-9H-fluorene-9-carboxylate |
| chloridazon | 5-amino-4-chloro-2-phenyl-3(2H)-pyridazinone |
| chlorimuron | 2-[[[[(4-chloro-6-methoxy-2-pyrimidinyl)ethyl-amino]carbonyl]amino]sulfonyl]benzoic acid, ethyl ester |
| chlornitrofen | 1,3,5-trichloro-2-(4-nitrophenoxy)benzene |
| chloropicrin | trichloronitromethane |
| chloroxuron | N'-[4-(4-chlorophenoxy)phenyl]-N,N-dimethylurea |
| chlorpropham | 1-methylethyl 3-chlorophenylcarbamate |
| chlorsulfuron | 2-chloro-N-[[(4-methoxy-6-methyl-1,3,5-triazin-2-yl)amino]carbonyl]benzenesulfonamide |
| chlorthaldimethyl | dimethyl 2,3,5,6-tetrachloro-1,4-benzene-dicarboxylate |
| chlorthiamid | 2,6-dichlorobenzene carbothioamide |
| chlortoluron | N'-(3-chloro-4-methylphenyl)-N,N-dimethylurea |
| cinmethylin | exo-1-methyl-4-(1-methylethyl)-2-[(2-methyl-phenyl)methoxy]-7-oxabicyclo-[2.2.1]heptane |
| clethodim | (E,E)-(±)-2-[1-[[(3-chloro-2-propenyl)oxy]imino]-propyl]-5-[2-(ethylthio)propyl]-3-hydroxy-2-cyclohexen-1-one |
| clomazone | 2-[(2-chlorophenyl)methyl]-4,4-dimethyl-3-isoxazolidinone |

| Common Name | Chemical Name |
|---|---|
| cloproxydim | (E,E)-2-[1-[[(3-chloro-2-propenyl)oxy)imino]butyl]-5-[2-(ethylthio)propyl]-3-hydroxy-2-cyclohexen-1-one |
| clopyralid | 3,6-dichloro-2-pyridinecarboxylic acid |
| CMA | calcium salt of MAA |
| cyanazine | 2-[[4-chloro-6-(ethylamino)-1,3,5-triazin-2-yl]-amino]-2-methylpropanenitrile |
| cycloate | S-ethyl cyclohexylethylcarbamothioate |
| cycloxydim | 2-[1-ethoxyimino)butyl]-3-hydroxy-5-(tetrahydro-2H-thiopyran-3-yl)-2-cyclohexene-1-one |
| cycluron | 3-cyclooctyl-1,1-dimethylurea |
| cyperquat | 1-methyl-4-phenylpyridinium |
| cyprazine | 2-chloro-4-(cyclopropylamino)-6-(isopropylamino)-s-triazine |
| cyprazole | N-[5-(2-chloro-1,1-dimethylethyl)-1,3,4-thiadiazol-2-yl]cyclopropanecarboxamide |
| cypromid | 3',4'-dichlorocyclopropanecarboxanilide |
| dalapon | 2,2-dichloropropanoic acid |
| dazomet | tetrahydro-3,5-dimethyl-2H-1,3,5-thiadiazine-2-thione |
| DCPA | dimethyl 2,3,5,6-tetrachloro-1,4-benzene-dicarboxylate |
| desmedipham | ethyl [3-[[(phenylamino)carbonyl]oxy]phenyl]-carbamate |

| Common Name | Chemical Name |
|---|---|
| desmetryn | 2-(isopropylamino)-4-(methylamino)-6-(methylthio)-s-triazine |
| diallate | S-(2,3-dichloro-2-propenyl)bis(1-methylethyl)-carbamothioate |
| dicamba | 3,6-dichloro-2-methoxybenzoic acid |
| dichlobenil | 2,6-dichlorobenzonitrile |
| dichlorprop | (±)-2-(2,4-dichlorophenoxy)propanoic acid |
| diclofopmethyl | (±)-2-[4-(2,4-dichlorophenoxy)phenoxy]propanoic acid, methyl ester |
| diethatyl | N-(chloroacetyl)-N-(2,6-diethylphenyl)glycine |
| difenoxuron | N'-[4-(4-methoxyphenoxy)phenyl]-N,N-dimethylurea |
| difenzoquat | 1,2-dimethyl-3,5-diphenyl-1H-pyrazolium ion |
| diflufenican | N-(2,4-difluorophenyl)-2-(3-trifluoromethylphenoxy)pyridine-3-carboxamide |
| dimefuron | N'-[3-chloro-4-[5-(1,1-dimethylethyl)-2-oxo-1,3,4-oxadiazol-3(2H)-yl]phenyl]-N,N-dimethylurea |
| dimethachlor | 2-chloro-N-(2,6-dimethylphenyl)-N-(2-methoxyethyl)acetamide |
| dimethametryn | N-(1,2-dimethylpropyl)-N'-ethyl-6-(methylthio)-1,3,5-triazine-2,4-diamine |
| dimethipin | 2,3-dihydro-5,6-dimethyl-1,4-dithiin 1,1,4,4-tetraoxide |
| dimethylarsinic | dimethylarsinic acid |

| Common Name | Chemical Name |
|---|---|
| dinitramine | $N^3$,$N^3$-diethyl-2,4-dinitro-6-(trifluoromethyl)-1,3-benzenediamine |
| dinoseb | 2-(1-methylpropyl)-4,6-dinitrophenol |
| dinoterb | 2-(1,1-dimethylethyl)-4,6-dinitrophenol |
| diphenamid | N,N-dimethyl-α-phenylbenzeneacetamide |
| dipropetryn | 6-(ethylthio)-N,N'-bis(1-methylethyl)-1,3,5-triazine-2,4-diamine |
| diquat | 6,7-dihydrodipyrido[1,2-a:2',1'-c]-pyrazinediium ion |
| diuron | N'-(3,4-dichlorophenyl)-N,N-dimethylurea |
| DNOC | 2-methyl-4,6-dinitrophenol |
| DSMA | disodium salt of MAA |
| dymron | N-(4-methylphenyl)-N'-(1-methyl-1-phenylethyl)-urea |
| eglinazine-ethyl | N-[4-chloro-6-(ethylamino)-1,3,5-triazin-2-yl]glycine ethyl ester |
| endothall | 7-oxabicyclo[2.2.1]heptane-2,3-dicarboxylic acid |
| EPTC | S-ethyl dipropylcarbamothioate |
| ethalfluralin | N-ethyl-N-(2-methyl-2-propenyl)-2,6-dinitro-4-(trifluoromethyl)benzenamine |
| ethidimuron | N-[5-(ethylsulfonyl)-1,3,4-thiadiazol-2-yl]-N,N'-dimethylurea |
| ethofumesate | (±)-2-ethoxy-2,3-dihydro-3,3-dimethyl-5-benzofuranyl methanesulfonate |

| Common Name | Chemical Name |
|---|---|
| fenac | 2,3,6-trichlorobenzeneacetic acid |
| fenoprop | (±)-2-(2,4,5-trichlorophenoxy)propanoic acid |
| fenoxaprop | (±)-2-[4-[(6-chloro-2-benzoxazolyl)oxy]phenoxy]-propanoic acid |
| fenuron | N,N-dimethyl-N'-phenylurea |
| fenuron TCA | Salt of fenuron and TCA |
| flamprop-M-ispropyl | 1-methylethyl N-benzoyl-N-(3-chloro-4-fluoro-phenyl)-D-alanine |
| flamprop-methyl | methyl N-benzoyl-N-(3-chloro-4-fluorophenyl)-DL-alaninate |
| fluazifop | (±)-2-[4-[[5-(trifluoromethyl)-2-pyridinyl]oxy]-phenoxy]propanoic acid |
| fluazifop-P | (R)-2-[4-[[5-(trifluoromethyl)-2-pyridinyl]oxy]-phenoxy]propanoic acid |
| fluchloralin | N-(2-chloroethyl)-2,6-dinitro-N-propyl-4-(trifluoro-methyl)benzenamine |
| fluometuron | N,N-dimethyl-N'-[3-(trifluoromethyl)phenyl]urea |
| fluralin | N-butyl-N-ethyl-2,6-dinitro-4-(trifluoromethyl)-benzenamine |
| fluorodifen | p-nitrophenyl α,α,α-trifluoro-2-nitro-p-tolyl ether |
| fluoroglycofen | carboxymethyl 5-[2-chloro-4-(trifluoromethyl)-phenoxy]-2-nitrobenzoate |
| flurecol-butyl | butyl 9-hydroxy-9H-fluorene-9-carboxylate |

| Common Name | Chemical Name |
|---|---|
| fluridone | 1-methyl-3-phenyl-5-[3-(trifluoromethyl)phenyl]-4(1H)-pyridinone |
| flurochloridone | 3-chloro-4-(chloromethyl)-1-[3-(trifluoromethyl)-phenyl]-2-pyrrolidinone |
| fluroxypyr | [(4-amino-3,5-dichloro-6-fluoro-2-pyridinyl)oxy]-acetic acid |
| fomesafen | 5-[2-chloro-4-(trifluoromethyl)phenoxy]-N-(methyl-sulfonyl)-2-nitrobenzamide |
| fosamine-ammonium | ethyl hydrogen (aminocarbonyl)phosphonate ammonium ethyl |
| glufosinate-ammonium | ammonium 2-amino-4-(hydroxymethylphos-phinyl)butanoate |
| glyphosate | N-(phosphonomethyl)glycine |
| haloxyfop | 2-[4-[[3-chloro-5-(trifluoromethyl)-2-pyridinyl]oxy]-phenoxy]propanoic acid |
| hexaflurate | potassium hexafluoroarsenate |
| hexazinone | 3-cyclohexyl-6-(dimethylamino)-1-methyl-1,3,5-triazine-2,4(1H,3H)-dione |
| imazametha-benz | 6-(4-isopropyl-4-methyl-5-oxo-2-imidazolin-2-yl)-m-toluic acid, methyl ester and 6-(4-isopropyl-4-methyl-5-oxo-2-imidazolin-2-yl)-p-toluic acid, methyl ester |
| imazapyr | (±)-2-[4,5-dihydro-4-methyl-4-(1-methyl-ethyl)-5-oxo-1H-imidazol-2-yl]-3-pyridinecarboxylic acid |
| imazaquin | 2-[4,5-dihydro-4-methyl-4-(1-methylethyl)-5-oxo-1H-imidazol-2-yl]-3-quinolinecarboxylic acid |

| Common Name | Chemical Name |
|---|---|
| imazethapy | (±)-2-[4,5-dihydro-4-methyl-4-(1-methylethyl)-5-oxo-1H-imidazol-2-yl]-5-ethyl-3-pyridine-carboxylic acid |
| ioxynil | 4-hydroxy-3,5-diiodobenzonitrile |
| isocarbamid | N-(2-methylpropyl)-2-oxo-1-imidazolidine-carboxamide |
| isopropalin | 4-(1-methylethyl)-2,6-dinitro-N,N-dipropyl-benzenamine |
| isoproturon | N-(4-isopropylphenyl)-N',N'-dimethylurea |
| isouron | N'-[5-(1,1-dimethylethyl)-3-isoxazolyl]-N,N-dimethylurea |
| isoxaben | N-[3-(1-ethyl-1-methylpropyl)-5-isoxazolyl]-2,6-dimethoxybenzamide |
| karbutilate | 3-[[(dimethylamino)carbonyl]amino]phenyl-(1,1-dimethylethyl)carbamate |
| lactofen | (±)-2-ethoxy-1-methyl-2-oxoethyl 5-[2-chloro-4-(trifluoromethyl)phenoxy]-2-nitrobenzoate |
| lenacil | 3-cyclohexyl-6,7-dihydro-1H-cyclopenta-pyrimidine-2,4(3H,5H)-dione |
| linuron | N'-(3,4-dichlorophenyl)-N-methoxy-N-methylurea |
| MAA | methylarsonic acid |
| MAMA | monoammonium salt of MAA |
| MCPA | (4-chloro-2-methylphenoxy)acetic acid |
| MCPA-thioethyl | S-ethyl (4-chloro-2-methylphenoxy)ethanethioate |

| Common Name | Chemical Name |
|---|---|
| MCPB | 4-(4-chloro-2-methylphenoxy)butanoic acid |
| mecoprop | (±)-2-(4-chloro-2-methylphenoxy)propanoic acid |
| mefenacet | 2-(2-benzothiazolyloxy)-N-methyl-N-phenyl acetamide |
| mefluidide | N-[2,4-dimethyl-5-[[(trifluoromethyl)sulfonyl]-amino]phenyl]acetamide |
| metamitron | 4-amino-3-methyl-6-phenyl-1,2,4-triazin-5(4H)-one |
| metazachlor | 2-chloro-N-(2,6-dimethylphenyl)-N-(1(H)-pyrazol-1-ylmethyl)acetamide |
| methabenz- | 1,3-dimethyl-3-(2-benzothiazolyl)urea thiazuron |
| methal-propalin | N-(2-methyl-2-propenyl)-2,6-dinitro-N-propyl-4-(trifluoromethyl)benzenamide |
| metham | methylcarbamodithioic acid |
| methazole | 2-(3,4-dichlorophenyl)-4-methyl-1,2,4-oxadiazolidine-3,5-dione |
| methoxuron | N'-(3-chloro-4-methoxyphenyl)-N,N-dimethylurea |
| methoxyphenone | (4-methoxy-3-methylphenyl)(3-methylphenyl)-methanone |
| methyldymron | N-methyl-N'-(1-methyl-1-phenylethyl)-N-phenyl-urea |
| metobromuron | N'-(4-bromophenyl)-N-methoxy-N-methylurea |
| metolachlor | 2-chloro-N-(2-ethyl-6-methylphenyl)-N-(2-methoxy-1-methylethyl)acetamide |

| Common Name | Chemical Name |
|---|---|
| metoxuron | N'-(3-chloro-4-methoxyphenyl)-N,N-dimethylurea |
| metribuzin | 4-amino-6-(1,1-dimethylethyl)-3-(methylthio)-1,2,4-triazin-5(4H)-one |
| metsulfuron methyl | 2-[[[[(4-methoxy-6-methyl-1,3,5-triazin-2-yl)-amino]carbonyl]amino]sulfonyl]benzoic acid, methyl ester |
| MH | 1,2-dihydro-3,6-pyridazinedione |
| molinate | S-ethyl hexahydro-1H-azepine-1-carbothioate |
| monalide | N-(4-chlorophenyl)-2,2-dimethylpentanamide |
| monolinuron | 3-(p-chlorophenyl)-1-methoxy-1-methylurea |
| monuron | N'-(4-chlorophenyl)-N,N-dimethylurea |
| MSMA | monosodium salt of MAA |
| naproanilide | 2-(2-naphthalenyloxy)-N-phenylpropanamide |
| napropamide | N,N-diethyl-2-(1-naphthalenyloxy)propanamide |
| naptalam | 2-[(1-naphthalenylamino)carbonyl]benzoic acid |
| neburon | 1-butyl-3-(3,4-dichlorophenyl)-1-methylurea |
| nicosulfuron | 2-[[(4,6-dimethoxypyrimidin-2-yl)aminocarbonyl]-aminosulfonyl]-N,N-dimethyl 3-pyridine-carboxamide |
| nitralin | 4-methylsulfonyl-2,6-dinitro-N,N-dipropylaniline |
| nitrofen | 2,4-dichloro-1-(4-nitrophenoxy)benzene |

| Common Name | Chemical Name |
|---|---|
| nitrofluorfen | 2-chloro-1-(4-nitrophenoxy)-4-(trifluoromethyl)-benzene |
| norea | N,N-dimethyl-N'-(octahydro-4,7-methano-1H-inden-5-yl)urea 3aα,4α,5α,7α,7aα-isomer |
| norflurazon | 4-chloro-5-(methylamino)-2-[3-(trifluoromethyl)-phenyl]-3(2H)-pyridazinone |
| orbencarb | S-[2-(chlorophenyl)methyl]diethylcarbamothioate |
| oryzalin | 4-(dipropylamino)-3,5-dinitrobenzenesulfonamide |
| oxadiazon | 3-[2,4-dichloro-5-(1-methylethoxy)phenyl]-5-(1,1-dimethylethyl)-1,3,4-oxadiazol-2(3H)-one |
| oxyfluorfen | 2-chloro-1-(3-ethoxy-4-nitrophenoxy)-4-(trifluoro-methyl)benzene |
| paraquat | 1,1'-dimethyl-4,4'-dipyridinium ion |
| pebulate | S-propyl butylethylcarbamothioate |
| pendimethalin | N-(1-ethylpropyl)-3,4-dimethyl-2,6-dinitro-benzenamine |
| perfluidone | 1,1,1-trifluoro-N-[2-methyl-4-(phenylsulfonyl)-phenyl]methanesulfonamide |
| phenisopham | 3-[[(1-methylethoxy)carbonyl]amino]phenyl ethylphenylcarbamate |
| phenmedipham | 3-[(methoxycarbonyl)amino]phenyl (3-methyl-phenyl)carbamate |
| picloram | 4-amino-3,5,6-trichloro-2-pyridinecarboxylic acid |

| Common Name | Chemical Name |
|---|---|
| piperophos | S-[2-(2-methyl-1-piperidinyl)-2-oxoethyl]O,O-dipropyl phosphorodithioate |
| pretilachlor | 2-chloro-N-(2,6-diethylphenyl)-N-(2-propoxyethyl)-acetamide |
| procyazine | 2-[[4-chloro-6-(cyclopropylamino)-1,3,5-triazine-2-yl]amino]-2-methylpropanenitrile |
| prodiamine | 2,4-dinitro-N3,N3-dipropyl-6-(trifluoromethyl)-1,3-benzenediamine |
| profluralin | N-(cyclopropylmethyl)-2,6-dinitro-N-propyl-4-(trifluoromethyl)benzenamine |
| proglinazine-ethyl | N-[4-chloro-6-[(1-methylethyl)amino]-1,3,5-triazin-2-yl]glycine ethyl ester |
| prometon | 6-methoxy-N,N'-bis(1-methylethyl)-1,3,5-triazine-2,4-diamine |
| prometryn | N,N'-bis(1-methylethyl)-6-(methylthio)-1,3,5-triazine-2,4-diamine |
| pronamide | 3,5-dichloro-N-(1,1-dimethyl-2-propynyl)-benzamide |
| propachlor | 2-chloro-N-(1-methylethyl)-N-phenylacetamide |
| propanil | N-(3,4-dichlorophenyl)propanamide |
| propaquizafop | 2-[[(1-methylethylidene)amino]oxy]ethyl 2-[4-[(6-chloro-2-quinoxalinyl)oxy]phenoxy]propanoate |
| propazine | 6-chloro-N,N'-bis(1-methylethyl)-1,3,5-triazine-2,4-diamine |
| propham | 1-methylethyl phenylcarbamate |

| Common Name | Chemical Name |
|---|---|
| propyzamide | 3,5-dichloro-N-(1,1-dimethyl-2-propynl)benzamide |
| prosulfalin | N-[[4-(dipropylamino)-3,5-dinitrophenyl]sulfonyl]-S,S-dimethylsulfilimine |
| prosulfocarb | S-benzyldipropylthiocarbamate |
| prynachlor | 2-chloro-N-(1-methyl-2-propynyl)acetanilide |
| pyrazon | 5-amino-4-chloro-2-phenyl-3(2H)-pyridazinone |
| pyrazosulfuron-ethyl | ethyl 5-[[[[(4,6-dimethoxy-2-pyrimidinyl)amino]-carbonyl]amino]sulfonyl]-1-methyl-1H-pyrazole-4-carboxylate |
| pyrazoxyfen | 2-[[4-(2,4-dichlorobenzoyl)-1,3-dimethyl-1H-pyrazol-5-yl]oxy]-1-phenylethanone |
| pyridate | O-(6-chloro-3-phenyl-4-pyridazinyl) S-octyl carbonothioate |
| quizalofop ethyl | (±)-2-[4-[(6-chloro-2-quinoxalinyl)oxy]phenoxy]-propanoic acid, ethyl ester |
| secbumeton | N-ethyl-6-methoxy-N'-(1-methylpropyl)-1,3,5-triazine-2,4-diamine |
| sethoxydim | 2-[1-(ethoxyimino)butyl]-5-[2-(ethylthio)propyl]-3-hydroxy-2-cyclohexen-1-one |
| siduron | N-(2-methylcyclohexyl)-N'-phenylurea |
| simazine | 6-chloro-N,N'-diethyl-1,3,5-triazine-2,4-diamine |
| simetryn | N,N'-diethyl-6-(methylthio)-1,3,5-triazine-2,4-diamine |
| sodium chlorate | sodium chlorate |

| Common Name | Chemical Name |
|---|---|
| sodium mono-chloroacetate | chloroacetic acid, sodium salt |
| sulfometuron methyl | 2-[[[[(4,6-dimethyl-2-pyrimidinyl)amino]carbonyl]-amino]sulfonyl]benzoic acid, methyl ester |
| 2,4,5-T | (2,4,5-trichlorophenoxy)acetic acid |
| 2,3,6-TBA | 2,3,6-trichlorobenzoic acid |
| TCA | trichloroacetic acid |
| tebutam | 2,2-dimethyl-N-(1-methylethyl)-N-(phenylmethyl)-propanamide |
| tebuthiuron | N-[5-(1,1-dimethylethyl)-1,3,4-thiadiazol-2-yl]-N,N'-dimethylurea |
| terbacil | 5-chloro-3-(1,1-dimethylethyl)-6-methyl-2,4-(1H,3H)-pyrimidinedione |
| terbuchlor | N-(butoxymethyl)-2-chloro-N-[2-(1,1-dimethyl-ethyl)-6-methylphenyl]acetamide |
| terbumeton | N-(1,1-dimethylethyl)-N'-ethyl-6-methoxy-1,3,5-triazine-2,4-diamine |
| terbuthylazine | 2-(tert-butylamino)-4-chloro-6-(ethylamino)-s-triazine |
| terbutol | 2,6-di-tert-butyl-p-tolyl methylcarbamate |
| terbutryn | N-(1,1-dimethylethyl)-N'-ethyl-6-(methylthio)-1,3,5-triazine-2,4-diamine |
| thifensulfuron | 3-[[[[(4-methoxy-6-methyl-1,3,5,triazin-2-yl)-amino]carbonyl]amino]sulfonyl]-2-thiophene-carboxylic acid, methyl ester |

| Common Name | Chemical Name |
|---|---|
| thiameturon-methyl | methyl 3-[[[[(4-methoxy-6-methyl-1,3,5-triazin-2-yl)amino]carbonyl]amino]sulfonyl]2-thiophene-carboxylate |
| thiazafluron | N,N'-dimethyl-N-[5-(trifluoromethyl)-1,3,4-thiadiazol-2-yl]urea |
| thiobencarb | S-[(4-chlorophenyl)methyl] diethylcarbamothioate |
| tiocarbazil | S-(phenylmethyl) bis(1-methylpropyl)-carbamothioate |
| tralkoxydim | 2-[1-(ethoxyimino)propyl]-3-hydroxy-5-(2,4,6-trimethylphenyl)-2-cyclohexen-1-one |
| triallate | S-(2,3,3-trichloro-2-propenyl) bis(1-methylethyl)-carbamothioate |
| triasulfuron | 2-(2-chloroethoxy)-N-[[(4-methoxy-6-methyl-1,3,5-triazin-2-yl)amino]carbonyl]benzenesulfonamide |
| tribenuron methyl | 2-[[[[N-(4-methoxy-6-methyl-1,3,5-triazine-2-yl)-N-methylamino]carbonyl]amino]sulfonyl]benzoic acid, methyl ester |
| triclopyr | [(3,5,6-trichloro-2-pyridinyl)oxy]acetic acid |
| tridiphane | (±)2-(3,5-dichlorophenyl)-2-(2,2,2-trichloroethyl)-oxirane |
| trietazine | 6-chloro-N,N,N'-triethyl-1,3,5-triazine-2,4-diamine |
| trifluralin | 2,6-dinitro-N,N-dipropyl-4-(trifluoromethyl)-benzenamine |
| trimeturon | 1-(p-chlorophenyl)-2,3,3-trimethylpseudourea |
| 2,4-D | (2,4-dichlorophenoxy)acetic acid |

| Common Name | Chemical Name |
|---|---|
| 2,4-DB | 4-(2,4-dichlorophenoxy)butanoic acid |
| vernolate | S-propyl dipropylcarbamothioate |
| xylachlor | 2-chloro-N-(2,3-dimethylphenyl)-N-(1-methylethyl)acetamide |

The biological activity of representative compounds of the invention identified in a preceeding table as Compounds No. 1-129 were determined in greenhouse tests as described below.

TEST A

Seeds of barley (Hordeum vulgare), barnyardgrass (Echinocloa crus-galli), bedstraw (Galium aparine), blackgrass (Alopecurus myosuroides), cheatgrass (Bromus secalinus), chickweed (Stellaria media), cocklebur (Xanthium pensylyanicum), corn (Zea mays), cotton (Gossypium hirsutum), crabgrass (Digitaria spp.), giant foxtail (Setaria faberii), lambsquarters (Chenopodium album), morningglory (Ipomoea hederacea), rape (Brassica napus), rice (Oryza satiya), sorghum (Sorghum bicolor), soybean (Glycine max), sugar beet (Beta vulgaris), velvetleaf (Abutilon theophrasti), wheat (Triticum aestivum), wild buckwheat (Polygonum convolvulus), wild oat (Avena fatua) and purple nutsedge (Cyperus rotundus) tubers were planted and treated preemergence with test chemicals dissolved in a non-phytotoxic solvent. At the same time, these crop and weed species were also treated with postemergence applications of test chemicals. Plants ranged in height from two to eighteen cm (one to four leaf stage) for postemergence treatments. Treated plants and controls were maintained in a greenhouse for twelve to sixteen days, after which all species were compared to controls and visually evaluated. Plant response ratings, summarized in Table A, are based on a scale of 0 to 10 where 0 is no effect and 10 is complete control. A dash (-) response means no test result.

| Table A | COMPOUND |
|---|---|
| Rate (400 g/ha) | 10 |
| POSTEMERGENCE | |
| Barley | 10 |
| Barnyardgrass | 10 |
| Bedstraw | 0 |
| Blackgrass | 10 |
| Cheatgrass | 10 |
| Chickweed | 0 |
| Corn | 10 |
| Cotton | 0 |
| Crabgrass | 10 |
| Giant foxtail | 10 |
| Lambsquarters | 0 |
| Morningglory | 0 |
| Nutsedge | 0 |
| Rape | 0 |
| Rice | 10 |
| Sorghum | 10 |
| Soybean | 1 |
| Sugar beet | 0 |
| Velvetleaf | 0 |
| Wheat | 10 |
| Wild buckwheat | 0 |
| Wild oat | 10 |

| Table A | COMPOUND |
|---|---|
| Rate (400 g/ha) | 10 |
| PREEMERGENCE | |
| Barley | 3 |
| Barnyardgrass | 10 |
| Bedstraw | 0 |
| Blackgrass | 10 |
| Cheatgrass | 10 |
| Chickweed | 0 |
| Corn | 9 |
| Cotton | 0 |
| Crabgrass | 10 |
| Giant foxtail | 9 |
| Lambsquarters | 0 |
| Morningglory | 0 |
| Nutsedge | 0 |
| Rape | 0 |
| Rice | 9 |
| Sorghum | 9 |
| Soybean | 0 |
| Sugar beet | 0 |
| Velvetleaf | 0 |
| Wheat | 8 |
| Wild buckwheat | 0 |
| Wild oat | 9 |

Table A

COMPOUND

| Rate (200 g/ha) | 1 | 2 | 3 | 4 | 5 | 6 | 7 | 8 | 9 | 11 | 12 | 14 | 15 | 16 | 17 | 18 | 19 | 20 | 21 | 22 | 23 | 24 | 25 | 26 | 27 | 28 | 29 | 30 | 31 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley | 3 | 10 | 10 | 10 | 10 | 10 | 10 | 9 | 9 | 9 | 3 | 10 | 10 | 3 | 2 | 2 | 10 | 3 | 9 | 10 | 10 | 10 | 10 | 9 | 5 | 10 | 2 | 10 | 10 |
| Barnyardgrass | 2 | 10 | 10 | 10 | 9 | 10 | 10 | 10 | 9 | 10 | 10 | 10 | 9 | 9 | 3 | 2 | 10 | 6 | 7 | 10 | 10 | 10 | 10 | 2 | 1 | 10 | 1 | 10 | 10 |
| Bedstraw | 0 | 0 | 0 | 0 | 0 | 0 | 0 | 0 | 0 | 1 | 0 | 2 | 0 | 0 | 0 | 0 | 0 | 0 | 0 | 0 | 0 | 0 | 0 | 0 | 0 | 0 | 0 | 0 | 2 |
| Blackgrass | 0 | 9 | 9 | 10 | 9 | 9 | 9 | 9 | 6 | 10 | 0 | 9 | 9 | 1 | 7 | 1 | 9 | 1 | 9 | 10 | 10 | 10 | 10 | 3 | 9 | 10 | 1 | 10 | 9 |
| Cheatgrass | 2 | 9 | 9 | 9 | 8 | 9 | 9 | 9 | 3 | 9 | 0 | 10 | 9 | 8 | 1 | 1 | 9 | 1 | 9 | 10 | 10 | 10 | 10 | 2 | 3 | 10 | 0 | 10 | 10 |
| Chickweed | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 4 |
| Cocklebur | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 1 | 10 | 9 | 10 | 0 | 10 | 10 | 0 | 0 | 10 | 4 | 10 | 1 | 2 | 2 | 1 | 9 | 2 | 1 | 10 | 10 | 10 | 9 | 1 | 1 | 10 | 0 | 10 | 10 |
| Cotton | 1 | 0 | 0 | 2 | 0 | 0 | 0 | 0 | 0 | 0 | 0 | 0 | 0 | 0 | 0 | 0 | 0 | 0 | 0 | 0 | 2 | 2 | 0 | 0 | 0 | 0 | 0 | 0 | 0 |
| Crabgrass | 9 | 10 | 10 | 10 | 9 | 10 | 10 | 9 | 9 | 9 | 5 | 10 | 10 | 4 | 8 | 1 | 9 | 6 | 7 | 9 | 10 | 9 | 9 | 1 | 2 | 9 | 1 | 10 | 10 |
| Giant foxtail | 8 | 10 | 10 | 10 | 8 | 10 | 10 | 8 | 7 | 9 | 4 | 10 | 9 | 2 | 4 | 1 | 10 | 3 | 9 | 10 | 10 | 9 | 10 | 1 | 3 | 10 | 2 | 10 | 10 |
| Lambsquarters | - | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 | 0 | 0 | 0 | 0 | 4 |
| Morningglory | 0 | 0 | 0 | 2 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 |
| Nutsedge | 0 | 0 | 0 | 0 | 0 | 0 | 0 | 0 | 5 | 0 | 0 | 0 | 0 | 9 | 1 | 0 | 4 | 0 | - | 0 | 0 | 8 | 0 | 0 | 0 | 0 | 0 | 0 | 0 |
| Rape | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 |
| Rice | 10 | 10 | 10 | 10 | 9 | 9 | 10 | 9 | 9 | 9 | 6 | 10 | 10 | 2 | 9 | 9 | 9 | 9 | 9 | 10 | 10 | 9 | 10 | 0 | 10 | 10 | 0 | 10 | 10 |
| Sorghum | 1 | 10 | 10 | 10 | 9 | 10 | 10 | 8 | 8 | 9 | 9 | 10 | 10 | 9 | 4 | 2 | 10 | 9 | 9 | 10 | 10 | 10 | 10 | 0 | 0 | 10 | 0 | 10 | 10 |
| Soybean | 0 | 0 | 0 | 0 | 0 | 0 | 0 | 0 | 0 | 0 | 0 | 0 | 0 | 1 | 1 | 1 | 3 | 2 | 1 | 2 | 2 | 2 | 1 | 0 | 0 | 1 | 0 | 2 | 3 |
| Sugar beet | 0 | 0 | 0 | 0 | 0 | 0 | 0 | 0 | 0 | 0 | 0 | 2 | 2 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 3 | 0 | 1 | 0 | 0 | 0 | 0 | 0 | 2 | 0 | 0 | 0 | 0 | 0 | 0 | 0 | 0 | 0 |
| Wheat | 1 | 9 | 9 | 9 | 9 | 9 | 9 | 9 | 6 | 8 | 0 | 9 | 9 | 1 | 1 | 0 | 10 | 2 | 9 | 9 | 10 | 9 | 9 | 2 | 4 | 9 | 0 | 10 | 10 |
| Wild buckwheat | 0 | - | 0 | 0 | 0 | 0 | 0 | 0 | 0 | 0 | 0 | 5 | 0 | - | - | - | - | - | - | - | - | - | - | - | - | - | - | - | - |
| Wild oat | 0 | 9 | 9 | 9 | 4 | 9 | 9 | 5 | 0 | 6 | 0 | 9 | 2 | 1 | 0 | 1 | 9 | 0 | 9 | 9 | 9 | 9 | 9 | 8 | 1 | 9 | 0 | 9 | 9 |

Table A

COMPOUND

| Rate (200 g/ha) | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley | 9 | 9 | 0 | 10 | 10 | 10 | 10 | 9 | 9 | 10 | 10 | 10 | 7 | 0 | 10 | 9 | 10 | 10 | 9 | 2 | 9 | 8 | 10 | 10 | 9 | 9 | 10 | 10 | 9 |
| Barnyardgrass | 9 | 8 | 2 | 10 | 9 | 9 | 10 | 10 | 10 | 10 | 10 | 10 | 9 | 0 | 10 | 9 | 10 | 10 | 1 | 1 | 9 | 9 | 10 | 10 | 9 | 9 | 10 | 10 | 10 |
| Bedstraw | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 |
| Blackgrass | 7 | 2 | 0 | 10 | 9 | 10 | 10 | 9 | 9 | 9 | 9 | 9 | 7 | 0 | 9 | 3 | 9 | 9 | 4 | 2 | 8 | 3 | 9 | 10 | 9 | 8 | 10 | 9 | 9 |
| Cheatgrass | 7 | 2 | 0 | 9 | 9 | 9 | 10 | 9 | 7 | 9 | 10 | 9 | 3 | 0 | 9 | 3 | 9 | 9 | 3 | 0 | 5 | 4 | 9 | 9 | 6 | 5 | 10 | 9 | 3 |
| Chickweed | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 |
| Cocklebur | 0 | 0 | 0 | 0 | 0 | 2 | 0 | 0 | 0 | 0 | 0 | 0 | 1 | 0 | 0 | 0 | 0 | 0 | 0 | 0 | 0 | 0 | 0 | 0 | - | 0 | 0 | 0 | 0 |
| Corn | 0 | 0 | 1 | 10 | 9 | 9 | 10 | 9 | 7 | 8 | 10 | 2 | 6 | 0 | 4 | 1 | 2 | 10 | 1 | 0 | 1 | 1 | 9 | 9 | 2 | 1 | 10 | 10 | 9 |
| Cotton | 0 | 0 | 0 | 0 | 0 | 0 | 0 | 0 | 0 | 0 | 2 | 0 | 0 | 0 | 0 | 3 | 0 | 0 | 0 | 0 | 0 | 0 | 0 | 0 | 0 | 0 | 3 | 0 | 0 |
| Crabgrass | 7 | 4 | 5 | 10 | 10 | 10 | 10 | 9 | 10 | 9 | 10 | 9 | 7 | 0 | 5 | 9 | 9 | 8 | 2 | 3 | 5 | 9 | 9 | 7 | 8 | 7 | 10 | 10 | - |
| Giant foxtail | 9 | 4 | 2 | 10 | 10 | 8 | 9 | 9 | 9 | 10 | 9 | 9 | 7 | 0 | 9 | 4 | 9 | 9 | 7 | 1 | 7 | 7 | 9 | 8 | 7 | 9 | 10 | 9 | 9 |
| Lambsquarters | 0 | 0 | 0 | 0 | 0 | 0 | - | 0 | - | 0 | 0 | 0 | 0 | 0 | 0 | 0 | 0 | 0 | 0 | 0 | 0 | 0 | 0 | 0 | 0 | - | 0 | 0 | 0 |
| Morningglory | 0 | 0 | 0 | 0 | 0 | 2 | 2 | 0 | 0 | 0 | 0 | 0 | 2 | 0 | 2 | 0 | 0 | 0 | 0 | 0 | 0 | 0 | 0 | 2 | 0 | 0 | 0 | 0 | 0 |
| Nutsedge | 0 | - | 0 | 0 | 0 | 0 | 0 | 0 | 0 | 0 | 0 | 0 | 2 | 0 | 2 | 0 | 0 | 0 | 0 | 0 | 0 | 0 | 0 | 0 | 0 | 0 | 0 | 3 | 0 |
| Rape | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 0 | 9 | 10 | 10 | 10 | 10 | 9 | 10 | 10 | 10 | 10 | 9 | 0 | 10 | 9 | 10 | 10 | 9 | 10 | 10 | 10 | 10 | 10 | 10 | 10 | 10 | 10 | 10 |
| Sorghum | 9 | 3 | 2 | 10 | 9 | 9 | 10 | 10 | 10 | 10 | 10 | 9 | 9 | 0 | 10 | 10 | 10 | 10 | 9 | 0 | 5 | 5 | 10 | 9 | 10 | 6 | 10 | 10 | 10 |
| Soybean | 1 | 0 | 0 | 2 | 3 | 1 | 3 | 3 | 3 | 0 | 0 | 0 | 0 | 0 | 0 | 1 | 0 | 1 | 0 | 0 | 0 | 2 | 0 | 0 | 0 | 0 | 1 | 1 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 5 | 3 | 0 | 9 | 9 | 9 | 9 | 9 | 9 | 9 | 9 | 9 | 8 | 0 | 9 | 9 | 9 | 9 | 9 | 1 | 7 | 9 | 9 | 9 | 9 | 9 | 10 | 9 | 7 |
| Wild buckwheat | - | - | - | - | - | - | 0 | 0 | 0 | - | - | - | - | - | - | - | - | - | - | - | - | - | - | - | - | - | - | - | - |
| Wild oat | 9 | 1 | 0 | 9 | 9 | 9 | 9 | 9 | 5 | 9 | 9 | 7 | 7 | 0 | 9 | 3 | 9 | 9 | 1 | 0 | 2 | 0 | 8 | 8 | 3 | 1 | 9 | 8 | 1 |

Table A

COMPOUND

| Rate (200 g/ha) | 61 | 62 | 63 | 64 | 65 | 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley | 0 | 10 | 10 | 10 | 9 | 10 | 10 | 10 | 10 | 10 | 10 | 9 | 1 | 0 | 10 | 1 | 9 | 9 | 10 | 9 | 10 | 10 | 10 | 10 | 10 | 10 | 10 | 1 | 0 |
| Barnyardgrass | 1 | 10 | 10 | 9 | 6 | 10 | 10 | 10 | 10 | 10 | 10 | 10 | 2 | 0 | 10 | 1 | 9 | 9 | 10 | 0 | 10 | 10 | 10 | 10 | 10 | 10 | 10 | 0 | 0 |
| Bedstraw | 0 | 2 | 0 | 0 | 0 | 0 | 0 | 0 | 0 | 0 | 0 | 0 | - | 0 | 2 | 0 | - | 0 | 0 | 0 | 0 | 0 | 0 | 0 | 0 | 0 | - | 0 | 0 |
| Blackgrass | 0 | 10 | 10 | 10 | 2 | 10 | 8 | 10 | 10 | 10 | 10 | 9 | 0 | 4 | 9 | 1 | 4 | 2 | 9 | 5 | 9 | 9 | 9 | 9 | 9 | 9 | 10 | 2 | - |
| Cheatgrass | 0 | 10 | 10 | 10 | 4 | 10 | 8 | 10 | 10 | 9 | 10 | 7 | 3 | 2 | 9 | 2 | 6 | 1 | 9 | 4 | 9 | 10 | 10 | 7 | 7 | 9 | 10 | 0 | 1 |
| Chickweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 |
| Corn | 0 | 10 | 10 | 6 | 3 | 10 | 7 | 10 | 6 | 3 | 10 | 1 | 0 | 0 | 10 | 0 | 7 | 4 | 9 | 0 | 10 | 10 | 10 | 7 | 1 | 9 | 10 | 0 | 2 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 0 | 9 | 9 | 9 | 8 | 9 | 5 | 10 | 10 | 10 | 10 | 7 | 8 | 0 | 9 | 3 | 9 | 10 | 9 | 5 | 6 | 7 | 10 | 9 | 9 | 9 | 10 | 0 | 1 |
| Giant foxtail | 0 | 10 | 10 | 9 | 9 | 10 | 9 | 10 | 10 | 10 | 10 | 7 | 5 | 0 | 9 | 1 | 9 | 5 | 9 | 5 | 9 | 9 | 10 | 9 | 9 | 9 | 8 | 0 | 2 |
| Lambsquarters | 0 | 0 | 4 | 4 | 0 | 0 | - | 0 | 0 | - | 0 | - | 0 | - | 0 | 0 | 0 | 0 | 0 | 0 | 0 | 0 | 0 | 0 | 0 | 0 | 0 | 0 | 0 |
| Morningglory | 0 | 0 | 0 | 0 | 1 | 0 | 0 | 0 | 0 | 0 | 0 | 0 | 0 | 0 | 2 | 0 | 0 | 0 | 2 | 0 | 0 | 2 | 0 | 0 | 0 | 0 | 0 | 1 | 0 |
| Nutsedge | 0 | - | 0 | 0 | 0 | 0 | 0 | 0 | 0 | 0 | 0 | 0 | 0 | 0 | 0 | - | 0 | 0 | - | - | - | - | 0 | 0 | 0 | 0 | 0 | 0 | 0 |
| Rape | 0 | 0 | 0 | 0 | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | 0 |
| Rice | 0 | 10 | 10 | 10 | 9 | 10 | 9 | 10 | 10 | 10 | 10 | 10 | 9 | 0 | 10 | 0 | 0 | 8 | 10 | 9 | 10 | 10 | 10 | 10 | 10 | 10 | 10 | 0 | 9 |
| Sorghum | - | 10 | 10 | 10 | 5 | 10 | 10 | 10 | 10 | 10 | 10 | 10 | 0 | 0 | 10 | 0 | 10 | 10 | 10 | 10 | 10 | 10 | 10 | 10 | 10 | 10 | 10 | 0 | 3 |
| Soybean | 0 | 1 | 2 | 0 | 0 | 1 | 0 | 2 | 0 | 0 | 0 | 0 | 0 | 0 | 0 | 0 | 0 | 0 | 0 | 0 | 0 | 0 | 0 | 0 | 0 | 0 | 1 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 2 | 4 | 2 | 0 | 0 | 0 | 0 | 2 | - | 0 | 0 | - | 0 | - | 0 | 0 | 0 |
| Wheat | 0 | 10 | 10 | 9 | 6 | 10 | 9 | 10 | 9 | 9 | 10 | 9 | 2 | 0 | 10 | 1 | 8 | 9 | 10 | 9 | 10 | 10 | 10 | 10 | 9 | 10 | 10 | 0 | 0 |
| Wild buckwheat | 0 | - | - | 0 | 0 | 0 | - | 0 | 0 | 0 | 0 | 0 | 0 | 0 | - | - | 0 | 0 | 0 | 0 | 0 | 0 | 0 | 0 | 0 | 0 | 0 | 0 | 0 |
| Wild oat | 0 | 10 | 10 | 9 | 3 | 9 | 2 | 9 | 9 | 10 | 10 | 3 | 2 | 2 | 9 | 1 | 7 | 0 | 7 | 4 | 9 | 10 | 9 | 6 | 2 | 9 | 9 | 0 | 0 |

Table A

| COMPOUND | | | | | | | | | | | | | | | | | | | | | | | | |
|---|---|---|---|---|---|---|---|---|---|---|---|---|---|---|---|---|---|---|---|---|---|---|---|---|
| Rate (200 g/ha) | 91 | 92 | 93 | 94 | 95 | 96 | 97 | 98 | 99 | 100 | 101 | 102 | 103 | 104 | 105 | 106 | 107 | 108 | 109 | 110 | 111 | 112 | 113 | 114 |
| POSTEMERGENCE | | | | | | | | | | | | | | | | | | | | | | | | |
| Barley | 10 | 10 | 3 | 10 | 10 | 10 | 4 | 10 | 10 | 10 | 9 | 3 | 7 | 6 | 9 | 3 | 0 | 4 | 0 | 8 | 0 | 0 | 9 | 10 |
| Barnyardgrass | 10 | 10 | 0 | 10 | 10 | 10 | 3 | 10 | 10 | 10 | 10 | 7 | 9 | 10 | 8 | 5 | 0 | 9 | 9 | 10 | 0 | 0 | 10 | 10 |
| Bedstraw | 0 | 0 | 0 | 0 | 0 | 0 | - | 0 | 1 | 3 | 0 | 0 | 0 | 0 | 0 | 0 | 0 | 0 | 0 | 0 | 0 | 0 | 0 | - |
| Blackgrass | 10 | 10 | 5 | 9 | 9 | 10 | 0 | 10 | 10 | 10 | 2 | 0 | 2 | 0 | 1 | 0 | 0 | 0 | 0 | 2 | 0 | 0 | 3 | - |
| Cheatgrass | 10 | 10 | 2 | 9 | 9 | 10 | 0 | 9 | 10 | 10 | 2 | 0 | 8 | 0 | 0 | 0 | 0 | 0 | 0 | 4 | 0 | 0 | 3 | - |
| Chickweed | 0 | - | 0 | 0 | 0 | 0 | 0 | 0 | 0 | 2 | 0 | 0 | 0 | 0 | 0 | 0 | 0 | 0 | 0 | 0 | 0 | 0 | 0 | - |
| Cocklebur | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 |
| Corn | 10 | 10 | 0 | 10 | 10 | 8 | 1 | 10 | 10 | 9 | 8 | 3 | 2 | 5 | 1 | 1 | 0 | 9 | 5 | 10 | 0 | 0 | 10 | 10 |
| Cotton | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 |
| Crabgrass | 10 | 10 | 4 | 10 | 9 | 6 | 8 | 10 | 10 | 10 | 6 | 2 | 9 | 9 | 2 | 7 | 0 | 7 | 9 | 9 | 0 | 0 | 9 | 10 |
| Giant foxtail | 10 | 10 | 7 | 9 | 9 | 10 | 5 | 9 | 10 | 10 | 5 | 1 | 9 | 3 | 2 | 1 | 0 | 6 | 2 | 8 | 0 | 0 | 5 | 10 |
| Lambsquarters | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | - |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 0 | 0 | 0 | 0 | 0 | 0 | 0 | - | - | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 10 | 10 | 10 | 10 | 10 | 10 | 9 | 10 | 10 | 10 | 2 | 0 | 0 | 1 | 9 | 0 | 0 | 5 | 1 | 8 | 0 | 0 | 9 | 10 |
| Sorghum | 10 | 10 | 1 | 10 | 10 | 10 | 4 | 10 | 10 | 10 | 7 | 1 | 10 | 7 | 9 | 0 | 0 | 9 | 7 | 10 | 0 | 0 | 10 | 10 |
| Soybean | 1 | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 10 | 10 | 4 | 10 | 9 | 10 | 0 | 10 | 10 | 10 | 0 | 0 | 9 | 0 | 8 | 0 | 0 | 0 | 0 | 2 | 0 | 0 | 9 | 10 |
| Wild buckwheat | 0 | 0 | - | 0 | 0 | 0 | 0 | 0 | 0 | 0 | 0 | 0 | 0 | 0 | 0 | 0 | 0 | 0 | 0 | 0 | 0 | 0 | 0 | - |
| Wild oat | 9 | 9 | 1 | 5 | 7 | 3 | 0 | 7 | 9 | 8 | 0 | 0 | 9 | 0 | 0 | 0 | 0 | 0 | 0 | 4 | 0 | 0 | 3 | 9 |

Table A

| Rate (200 g/ha) | COMPOUND 115 | 116 | 117 | 118 | 119 | 120 | 121 | 122 | 123 | 124 | 125 | 126 | 127 | 128 | 129 |
|---|---|---|---|---|---|---|---|---|---|---|---|---|---|---|---|
| POSTEMERGENCE | | | | | | | | | | | | | | | |
| Barley | 0 | 10 | 6 | 9 | 8 | 10 | 9 | 10 | 10 | 10 | 1 | 10 | 0 | 10 | 8 |
| Barnyardgrass | 0 | 10 | 9 | 10 | 10 | 10 | 10 | 10 | 10 | 10 | 2 | 10 | 1 | 10 | 9 |
| Bedstraw | 0 | 0 | 0 | 0 | 0 | 0 | 0 | 0 | 0 | 0 | 0 | 0 | 0 | 0 | 0 |
| Blackgrass | 0 | 3 | 1 | 8 | 2 | 7 | 9 | 10 | 10 | 4 | 1 | 9 | 0 | 9 | 2 |
| Cheatgrass | 0 | 4 | 2 | 9 | 2 | 10 | 10 | 9 | 10 | 5 | 2 | 6 | 0 | 5 | 1 |
| Chickweed | 0 | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 | 0 |
| Corn | 0 | 10 | 0 | 10 | 10 | 10 | 10 | 10 | 10 | 7 | 1 | 10 | 0 | 9 | 2 |
| Cotton | 0 | 0 | 0 | 3 | 0 | 0 | 0 | 0 | 0 | 0 | 0 | 0 | 0 | 0 | 0 |
| Crabgrass | 0 | 6 | 8 | 9 | 6 | 10 | 10 | 10 | 10 | 9 | 7 | 8 | 0 | 9 | 7 |
| Giant foxtail | 0 | 9 | 8 | 10 | 7 | 9 | 10 | 9 | 10 | 8 | 7 | 9 | 1 | 9 | 4 |
| Lambsquarters | 0 | 0 | 0 | 0 | 0 | 0 | 0 | 0 | 0 | 0 | 0 | 0 | 0 | 0 | 0 |
| Morningglory | 0 | 0 | 4 | 0 | 0 | 0 | 0 | 0 | 0 | 0 | 0 | 0 | 0 | 0 | 5 |
| Nutsedge | 0 | 0 | 0 | 0 | 0 | 0 | 0 | 0 | 0 | 0 | - | 0 | - | 0 | 3 |
| Rape | 0 | 2 | 0 | 0 | 0 | 0 | 0 | 0 | 0 | 0 | 0 | 0 | 0 | 0 | 0 |
| Rice | 0 | 1 | 1 | 2 | 0 | 9 | 5 | 10 | 10 | 10 | 9 | 9 | 2 | 9 | 9 |
| Sorghum | 0 | 10 | 9 | 10 | 9 | 10 | 10 | 10 | 10 | 10 | 9 | 10 | 0 | 10 | 9 |
| Soybean | 0 | 0 | 0 | 0 | 0 | 2 | 0 | 2 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 |
| Velvetleaf | 0 | 4 | 0 | 0 | 0 | 0 | 0 | 4 | 0 | 0 | 0 | 0 | 3 | 0 | 2 |
| Wheat | 0 | 5 | 5 | 9 | 3 | 9 | 9 | 10 | 10 | 10 | 2 | 10 | 0 | 10 | 7 |
| Wild buckwheat | 0 | 0 | 0 | 0 | 0 | 0 | 0 | 0 | 0 | 0 | 0 | 0 | 0 | 0 | 0 |
| Wild oat | 0 | 9 | 4 | 10 | 10 | 10 | 9 | 9 | 10 | 2 | 0 | 6 | 0 | 6 | 0 |

Table A

COMPOUND

| Rate (200 g/ha) | 1 | 2 | 3 | 4 | 5 | 6 | 7 | 8 | 9 | 11 | 12 | 14 | 15 | 16 | 17 | 18 | 19 | 20 | 21 | 22 | 23 | 24 | 25 | 26 | 27 | 28 | 29 | 30 | 31 |
|---|---|---|---|---|---|---|---|---|---|---|---|---|---|---|---|---|---|---|---|---|---|---|---|---|---|---|---|---|---|
| PREEMERGENCE | | | | | | | | | | | | | | | | | | | | | | | | | | | | | |
| Barley | 0 | 3 | 1 | 2 | 0 | 0 | 3 | 0 | 0 | 0 | 0 | 1 | 0 | 0 | 0 | 0 | 0 | 0 | 0 | 0 | 0 | 0 | 0 | 0 | 0 | 0 | 0 | 0 | 3 |
| Barnyardgrass | 2 | 10 | 9 | 9 | 2 | 9 | 9 | 7 | 3 | 0 | 0 | 9 | 8 | 0 | 1 | 0 | 0 | 0 | 0 | 9 | 7 | 8 | 2 | 0 | 0 | 7 | 0 | 9 | 9 |
| Bedstraw | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 9 | 0 |
| Blackgrass | 2 | 8 | 8 | 7 | 4 | 3 | 8 | 9 | 4 | 3 | 0 | 10 | 9 | 0 | 8 | 0 | 0 | 2 | 0 | 5 | 3 | 5 | 10 | 0 | 8 | 9 | 0 | 9 | 9 |
| Cheatgrass | 0 | 9 | 2 | 2 | 2 | 2 | 5 | 6 | 2 | 0 | 0 | 5 | 3 | 0 | 0 | 0 | 0 | 0 | 0 | 6 | 8 | 8 | 9 | 0 | 6 | 9 | 0 | 9 | 5 |
| Chickweed | 0 | 0 | 0 | 0 | 0 | 0 | 0 | 0 | 0 | 0 | 0 | 0 | 0 | 0 | 0 | - | 0 | 0 | 0 | 0 | 0 | 0 | 0 | 0 | 0 | - | 0 | 0 | 0 |
| Cocklebur | 0 | - | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 0 | 5 | 0 | 0 | 0 | 0 | 0 | 0 | 0 | 0 | 0 | 2 | 0 | 0 | 2 | 0 | 0 | 0 | 0 | 0 | 2 | 0 | 5 | 0 | 0 | 9 | 0 | 3 | 8 |
| Cotton | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 6 | 9 | 9 | 9 | 2 | 9 | 9 | 9 | 9 | 0 | 5 | 10 | 9 | 2 | 8 | 0 | 9 | 0 | 7 | 9 | 9 | 9 | 9 | 1 | 6 | 10 | 2 | 10 | 9 |
| Giant foxtail | 6 | 9 | 9 | 9 | 5 | 9 | 9 | 9 | 5 | 0 | 3 | 9 | 9 | 0 | 7 | 0 | 6 | 0 | 6 | 9 | 9 | 9 | 9 | 0 | 3 | 10 | 2 | 10 | 9 |
| Lambsquarters | 0 | 0 | 0 | 0 | 0 | 0 | 0 | 0 | 0 | 3 | 0 | 0 | 0 | 0 | 0 | 0 | 0 | 0 | 0 | 0 | - | 0 | 0 | 0 | 0 | 0 | 0 | 0 | 0 |
| Morningglory | 0 | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 10 | 0 | - | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 8 | 0 | 2 | 0 | 0 | 8 | 0 | 0 | 0 | 0 | 0 | 0 | 0 | 0 | 4 | 0 | 0 | 4 | 5 | 0 | 4 | 0 | 0 | 0 | 2 | 0 | 0 | 0 |
| Sorghum | 0 | 9 | 0 | 2 | 0 | 0 | 0 | 0 | 0 | 0 | 0 | 3 | 3 | 0 | 0 | 0 | 0 | 0 | 0 | 0 | 0 | 0 | 0 | 0 | 0 | 2 | 0 | 0 | 5 |
| Soybean | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 |
| Wheat | 0 | 3 | 0 | 6 | 0 | 0 | 2 | 0 | 0 | 0 | 0 | 3 | 2 | 0 | 0 | 0 | 0 | 0 | 0 | 5 | 5 | 7 | 5 | 0 | 0 | 4 | 0 | 3 | 5 |
| Wild buckwheat | - | - | - | - | - | - | - | - | - | 0 | 0 | - | - | - | - | - | - | - | - | - | - | - | - | - | - | - | - | - | - |
| Wild oat | 0 | 7 | 2 | 6 | 2 | 4 | 2 | 2 | 2 | 0 | 0 | 5 | 3 | 0 | 0 | 0 | 0 | 0 | 0 | 2 | 2 | 0 | 2 | 0 | 0 | 7 | 0 | 5 | 5 |

Table A

| Rate (200 g/ha) | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 |
|---|---|---|---|---|---|---|---|---|---|---|---|---|---|---|---|---|---|---|---|---|---|---|---|---|---|---|---|---|---|
| PREEMERGENCE | | | | | | | | | | | | | | | | | | | | | | | | | | | | | |
| Barley | 0 | 0 | 0 | 2 | 0 | 0 | 0 | 0 | 0 | 1 | 1 | 1 | 0 | 0 | 0 | 0 | 1 | 1 | 0 | 0 | 0 | 0 | 0 | 0 | 0 | 0 | 2 | 0 | 0 |
| Barnyardgrass | 0 | 0 | 0 | 2 | 2 | 9 | 4 | 3 | 1 | 6 | 9 | 8 | 0 | 0 | 8 | 2 | 5 | 10 | 2 | 0 | 7 | 2 | 3 | 9 | 8 | 0 | 10 | 0 | 2 |
| Bedstraw | 0 | 0 | 0 | 0 | 0 | 0 | 0 | 0 | 0 | 0 | 0 | 0 | 0 | 0 | 0 | 0 | 0 | - | - | 0 | - | - | - | 6 | 3 | 0 | 0 | 0 | 0 |
| Blackgrass | 0 | 2 | 2 | 8 | 2 | 7 | 7 | 7 | 8 | 4 | 8 | 8 | 0 | 0 | 5 | 3 | 3 | 9 | 9 | 0 | 7 | 0 | 5 | 6 | 9 | 2 | 6 | 2 | 3 |
| Cheatgrass | 0 | 0 | 0 | 0 | 0 | 2 | 2 | 0 | 0 | 9 | 5 | 5 | 0 | 0 | 5 | 0 | 2 | 9 | 0 | 0 | 2 | 0 | 0 | 0 | 6 | 9 | 5 | 0 | 4 |
| Chickweed | 0 | 0 | 0 | 0 | 0 | 0 | 0 | 0 | 0 | - | 0 | 0 | - | 0 | 0 | 0 | 0 | 0 | 0 | 0 | 0 | 0 | 0 | 0 | 0 | - | 5 | 0 | 5 |
| Cocklebur | 0 | 0 | 0 | - | 9 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 |
| Corn | 0 | 0 | 0 | 1 | 0 | 1 | 3 | 0 | 2 | 5 | 0 | 0 | 0 | 0 | 0 | 0 | 0 | 3 | 0 | 0 | 2 | 1 | 0 | 3 | 0 | 2 | 3 | 0 | 0 |
| Cotton | 0 | 0 | 0 | 0 | 0 | 0 | 0 | 0 | 2 | 0 | 0 | 0 | 0 | 0 | 0 | 0 | 0 | 0 | 0 | 0 | 6 | 0 | 7 | 2 | 0 | 0 | 0 | 0 | 0 |
| Crabgrass | 9 | 3 | 2 | 10 | 7 | 9 | 5 | 7 | 8 | 8 | 9 | 9 | 9 | 2 | 9 | 5 | 9 | 10 | 9 | 0 | 9 | 6 | 4 | 2 | 8 | 3 | 3 | 2 | 1 |
| Giant foxtail | 0 | 4 | 2 | 9 | 9 | 9 | 8 | 9 | 9 | 9 | 9 | 9 | 1 | 0 | 9 | 4 | 9 | 9 | 8 | 0 | 9 | 8 | 0 | 8 | 9 | 6 | 10 | 0 | 2 |
| Lambsquarters | 0 | 0 | - | 0 | 2 | 0 | 0 | - | 0 | 4 | 0 | 0 | 0 | 0 | 0 | 0 | 0 | 0 | 0 | 0 | 0 | 3 | - | 0 | - | - | 0 | 0 | - |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 4 | 0 | 0 | 0 | 9 | - | 0 | 0 | 0 | 0 | 0 | 4 | 0 | 0 | 0 | 0 | 0 | 0 | 0 | 0 | 0 | 0 | 10 | 0 | - | 0 | 8 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 0 | 0 | 0 | 0 | 8 | 0 | 0 | 0 | 3 | 0 | 0 | 0 | 0 | 0 | 0 | 3 | 3 | 0 | 0 | 0 | 0 | 3 | 7 | 0 | 0 | 2 | 0 | 0 |
| Sorghum | 0 | 0 | 0 | 5 | 0 | 1 | 0 | 0 | 0 | 0 | 0 | 0 | 0 | 0 | 0 | 0 | 5 | 3 | 2 | 0 | 0 | 0 | 0 | 2 | 2 | 0 | 2 | 0 | 2 |
| Soybean | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 |
| Wheat | 0 | 0 | 0 | 2 | 0 | 0 | 2 | 0 | 0 | 0 | 2 | 2 | 0 | 0 | 0 | 0 | 6 | 6 | 0 | 0 | 2 | 0 | 0 | 0 | 0 | 2 | 6 | 0 | 0 |
| Wild buckwheat | - | - | - | - | - | - | 0 | 0 | 3 | - | - | - | - | - | - | - | - | - | - | - | - | - | - | 0 | - | 0 | 0 | 0 | 0 |
| Wild oat | 0 | 0 | 0 | 4 | 3 | 2 | 3 | 0 | 2 | 2 | 0 | 2 | 0 | 0 | 2 | 2 | 0 | 6 | 0 | 0 | 2 | 0 | 0 | 2 | 0 | 5 | 3 | 2 | 2 |

Table A

| Rate (200 g/ha) | 61 | 62 | 63 | 64 | 65 | 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 |
|---|---|---|---|---|---|---|---|---|---|---|---|---|---|---|---|---|---|---|---|---|---|---|---|---|---|---|---|---|---|
| PREEMERGENCE | | | | | | | | | | | | | | | | | | | | | | | | | | | | | |
| Barley | 0 | 5 | 0 | 0 | 0 | 1 | 0 | 0 | 0 | 0 | 4 | 0 | 0 | 0 | 0 | 0 | 0 | 0 | 1 | 0 | 3 | 0 | 0 | 0 | 0 | 1 | 0 | 0 | 0 |
| Barnyardgrass | 0 | 9 | 8 | 2 | 1 | 10 | 0 | 8 | 10 | 10 | 9 | 3 | 2 | 0 | 9 | 0 | 2 | 2 | 10 | 2 | 10 | 10 | 10 | 9 | 10 | 10 | 10 | 0 | 0 |
| Bedstraw | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 | - | 0 |
| Blackgrass | 0 | 9 | 8 | 4 | 2 | 9 | 0 | 9 | - | 8 | 9 | 3 | 5 | 0 | 8 | 0 | 0 | 0 | 9 | 2 | 9 | 8 | 5 | 4 | 4 | 9 | 10 | 4 | 0 |
| Cheatgrass | 0 | 8 | 2 | 2 | 3 | 9 | 0 | 0 | 9 | 5 | 10 | 6 | 2 | 0 | 6 | 0 | 0 | 6 | 7 | 0 | 7 | 8 | 7 | 4 | 2 | 6 | 8 | 0 | 0 |
| Chickweed | 0 | 0 | 0 | 0 | 0 | 0 | 0 | 0 | 0 | 0 | 0 | 0 | 0 | 0 | 0 | 0 | 0 | 0 | 3 | 0 | 0 | 0 | 2 | 0 | 2 | 0 | 0 | 0 | 0 |
| Cocklebur | - | 0 | - | 0 | 0 | 0 | 0 | 0 | 0 | 0 | 0 | 0 | - | 0 | - | 0 | 0 | 0 | - | 0 | 0 | 0 | 0 | 0 | 0 | 3 | 4 | 3 | 0 |
| Corn | 0 | 3 | 3 | 0 | 1 | 2 | 0 | 1 | 1 | 0 | 2 | 0 | 0 | 0 | 2 | 0 | 2 | 0 | 0 | 1 | 1 | 1 | 3 | 1 | 2 | 1 | 2 | 0 | 0 |
| Cotton | 0 | 0 | 0 | 0 | 0 | 0 | 0 | 0 | 0 | 0 | 0 | 8 | 0 | 0 | 0 | 0 | 0 | 0 | 0 | 0 | 0 | 0 | 0 | 0 | 0 | 0 | 0 | 0 | 0 |
| Crabgrass | 0 | 9 | 8 | 9 | 1 | 9 | 0 | 10 | 10 | 9 | 9 | 9 | 9 | 0 | 8 | 0 | 8 | 7 | 10 | 10 | 10 | 10 | 9 | 9 | 9 | 10 | 10 | 2 | 0 |
| Giant foxtail | 0 | 9 | 9 | 8 | 5 | 9 | 3 | 9 | 10 | 9 | 8 | 9 | 8 | 0 | 3 | 0 | 1 | 8 | 10 | 7 | 10 | 7 | 9 | 9 | 9 | 10 | 10 | 0 | 0 |
| Lambsquarters | 0 | 4 | 0 | 0 | 0 | 0 | 0 | 0 | - | - | - | 0 | 0 | 0 | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 |
| Nutsedge | 0 | - | 0 | 0 | 0 | 0 | 0 | 0 | 0 | 0 | 0 | 0 | 0 | 0 | 10 | 0 | 0 | 0 | 0 | 0 | - | - | 0 | 0 | 0 | 0 | 5 | 0 | 0 |
| Rape | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 3 | 0 | 0 | 0 | 2 | 0 | 0 | 1 | 0 | 2 | 0 | 0 | 0 | 0 | 0 | 0 | 0 | - | 0 | 2 | 7 | 0 | 0 | 0 | 2 | 0 | 2 | 0 |
| Sorghum | 0 | 5 | 3 | 0 | 0 | 2 | 0 | 3 | 3 | 3 | 4 | 0 | 0 | 0 | 0 | 0 | 0 | 0 | 2 | 1 | 5 | 5 | 5 | 1 | 2 | 3 | 3 | 0 | 0 |
| Soybean | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 |
| Wheat | 0 | 6 | 2 | 0 | 0 | 9 | 2 | 0 | 5 | 0 | 5 | 0 | 0 | 0 | 0 | 0 | 0 | 0 | 0 | 0 | 2 | 0 | 3 | 0 | 0 | 2 | 0 | 0 | 0 |
| Wild buckwheat | 0 | 0 | - | 0 | 0 | 2 | 0 | 0 | 0 | 0 | 0 | - | 0 | 0 | - | - | 0 | 0 | 3 | 0 | 0 | 0 | 2 | 0 | 2 | 0 | 0 | 0 | - |
| Wild oat | 0 | 4 | 3 | 3 | 2 | 7 | 3 | 2 | 7 | 4 | 8 | 0 | 0 | 0 | 3 | 0 | 0 | 0 | 0 | 0 | 6 | 2 | 3 | 0 | 0 | 3 | 2 | 0 | 0 |

Table A

| | COMPOUND | | | | | | | | | | | | | | | | | | | | | | | |
|---|---|---|---|---|---|---|---|---|---|---|---|---|---|---|---|---|---|---|---|---|---|---|---|
| Rate (200 g/ha) | 91 | 92 | 93 | 94 | 95 | 96 | 97 | 98 | 99 | 100 | 101 | 102 | 103 | 104 | 105 | 106 | 107 | 108 | 109 | 110 | 111 | 112 | 113 | 114 |
| PREEMERGENCE | | | | | | | | | | | | | | | | | | | | | | | | |
| Barley | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Barnyardgrass | 10 | 10 | 5 | 0 | 3 | 8 | 2 | 10 | 10 | 2 | 1 | 1 | 0 | 0 | 0 | 0 | 1 | 1 | 3 | 0 | 0 | 0 | 10 | 10 |
| Bedstraw | 0 | 0 | 0 | 0 | - | 0 | 0 | 0 | 0 | 0 | - | - | 0 | 0 | 0 | 9 | 0 | 0 | 0 | 0 | - | 0 | 0 | 0 |
| Blackgrass | 9 | 10 | 0 | 0 | 5 | 9 | 3 | 8 | 9 | 2 | 0 | 0 | 0 | 0 | 0 | 0 | 0 | 0 | 2 | 0 | 0 | 0 | 5 | 8 |
| Cheatgrass | 7 | 8 | 0 | 0 | 0 | 6 | 0 | 4 | 7 | 0 | 0 | 0 | 0 | 0 | 0 | 0 | 0 | 0 | 0 | 0 | 0 | 0 | 5 | 7 |
| Chickweed | 2 | 0 | 0 | 0 | 0 | 0 | 0 | 0 | 0 | 0 | 0 | 0 | - | 0 | 0 | 2 | 0 | 0 | 0 | 0 | 0 | 0 | 0 | 0 |
| Cocklebur | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | - |
| Corn | 0 | 2 | 0 | 0 | 1 | 5 | 1 | 2 | 2 | 0 | 1 | 0 | 1 | 0 | 0 | 0 | 0 | 0 | 2 | 0 | 0 | 0 | 2 | 8 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 9 | 10 | 9 | 2 | 10 | 9 | 3 | 9 | 9 | 3 | 5 | 3 | 6 | 0 | 2 | 2 | 0 | 5 | 5 | 5 | 0 | 0 | 9 | 10 |
| Giant foxtail | 10 | 10 | 8 | 0 | 9 | 9 | 6 | 9 | 9 | 7 | 1 | 0 | 2 | 0 | 2 | 0 | 0 | 1 | 3 | 0 | 0 | 0 | 9 | 9 |
| Lambsquarters | 0 | 0 | 0 | 0 | 0 | 0 | 0 | 0 | 0 | 0 | 0 | 0 | 0 | 0 | 0 | 10 | 0 | 0 | 0 | 0 | 0 | 0 | 0 | 0 |
| Morningglory | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 8 | 7 | 0 | 4 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 7 | 2 | 0 | 0 | 3 | 2 | 0 | 3 | 2 | 0 | 0 | 0 | 0 | 0 | 0 | 0 | 0 | 0 | 1 | 0 | 0 | 5 | 9 |
| Sorghum | 0 | 2 | 2 | 0 | 2 | 2 | 2 | 3 | 6 | 0 | 2 | 0 | 1 | 0 | 0 | 0 | 0 | 4 | 0 | 2 | 0 | 0 | 2 | 8 |
| Soybean | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 |
| Wheat | 0 | 1 | 0 | 0 | 0 | 6 | 0 | 2 | 0 | 0 | 0 | 0 | 0 | 0 | 0 | 0 | 0 | 0 | 0 | 2 | 0 | 0 | 2 | 2 |
| Wild buckwheat | 0 | 0 | 0 | 0 | 0 | 0 | 0 | 0 | 0 | 0 | 0 | 0 | 0 | 0 | 0 | 0 | 0 | 3 | 0 | 0 | 0 | 0 | 0 | 3 |
| Wild oat | 5 | 4 | 0 | 0 | 0 | 2 | 0 | 2 | 0 | 0 | 0 | 0 | 0 | 0 | 0 | 0 | 0 | 0 | 0 | 2 | 0 | 0 | 0 | 2 |

| Table A | COMPOUND | | | | | | | | | | | | | | |
|---|---|---|---|---|---|---|---|---|---|---|---|---|---|---|---|
| Rate (200 g/ha) | 115 | 116 | 117 | 118 | 119 | 120 | 121 | 122 | 123 | 124 | 125 | 126 | 127 | 128 | 129 |
| PREEMERGENCE | | | | | | | | | | | | | | | |
| Barley | 0 | 0 | 0 | 0 | 0 | 0 | 0 | 0 | 0 | 0 | 0 | 0 | 0 | 0 | 0 |
| Barnyardgrass | 0 | 0 | 0 | 0 | 0 | 0 | 0 | 6 | 7 | 9 | 2 | 0 | 0 | 4 | 1 |
| Bedstraw | 0 | 0 | 0 | 0 | 0 | 0 | 0 | 0 | 8 | 0 | 0 | 0 | 0 | 0 | 0 |
| Blackgrass | 0 | 0 | 0 | 2 | 0 | 0 | 0 | 9 | 9 | 8 | 9 | 2 | 0 | 9 | 0 |
| Cheatgrass | 0 | 0 | 0 | 2 | 0 | 0 | 0 | 5 | 8 | 2 | 5 | 0 | 0 | 2 | 0 |
| Chickweed | 0 | 0 | 0 | 0 | 0 | 0 | 0 | 0 | 0 | 0 | 0 | 0 | 0 | 0 | 0 |
| Cocklebur | 0 | - | 0 | 0 | 0 | 0 | - | 0 | - | 0 | 0 | 0 | 0 | 0 | 0 |
| Corn | 0 | 0 | 0 | 0 | 0 | 0 | 0 | 0 | 0 | 0 | 0 | 0 | 0 | 0 | 0 |
| Cotton | 0 | 0 | 0 | 0 | 0 | 0 | 0 | 0 | 2 | 0 | 0 | 0 | 0 | 0 | 0 |
| Crabgrass | 0 | 0 | 2 | 3 | 0 | 3 | 0 | 9 | 9 | 9 | 9 | 8 | 2 | 9 | 2 |
| Giant foxtail | 0 | 0 | 0 | 3 | 0 | 0 | 0 | 9 | 9 | 9 | 4 | 4 | 0 | 7 | 6 |
| Lambsquarters | 0 | 0 | 0 | 0 | 0 | 0 | 0 | 0 | 0 | 0 | - | 0 | 0 | 0 | 0 |
| Morningglory | 0 | 0 | 0 | 0 | 0 | 0 | 0 | 1 | 0 | 0 | - | 0 | 0 | 0 | 0 |
| Nutsedge | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 |
| Rice | 0 | 0 | 0 | 0 | 0 | 0 | 0 | 0 | 1 | 0 | 0 | 0 | 0 | 0 | 0 |
| Sorghum | 0 | 0 | 0 | 0 | 0 | 0 | 0 | 0 | 0 | 0 | 0 | 0 | 0 | 0 | 0 |
| Soybean | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 2 | 0 | 3 | 0 | 0 | 0 | 0 | 0 |
| Velvetleaf | 0 | 0 | 0 | 0 | 0 | 0 | 0 | 1 | 0 | 0 | 0 | 0 | 0 | 1 | 0 |
| Wheat | 0 | 0 | 0 | 0 | 0 | 0 | 0 | 2 | 0 | 0 | 0 | 0 | 0 | 0 | 0 |
| Wild buckwheat | 0 | 0 | 0 | 0 | 0 | 0 | 0 | 0 | 0 | 0 | 0 | 0 | 0 | 0 | 0 |
| Wild oat | 0 | 0 | 0 | 0 | 0 | 0 | 0 | 2 | 3 | 3 | 2 | 2 | 0 | 3 | 0 |

| Table A | COMPOUND | Table A | COMPOUND |
|---|---|---|---|
| Rate (100 g/ha) | 10 | Rate (100 g/ha) | 10 |
| POSTEMERGENCE | | PREEMERGENCE | |
| Barley | 10 | Barley | 0 |
| Barnyardgrass | 10 | Barnyardgrass | 10 |
| Bedstraw | 0 | Bedstraw | 0 |
| Blackgrass | 10 | Blackgrass | 9 |
| Cheatgrass | 10 | Cheatgrass | 9 |
| Chickweed | 0 | Chickweed | 0 |
| Corn | 10 | Corn | 3 |
| Cotton | 0 | Cotton | 0 |
| Crabgrass | 10 | Crabgrass | 8 |
| Giant foxtail | 10 | Giant foxtail | 9 |
| Lambsquarters | - | Lambsquarters | 0 |
| Morningglory | 0 | Morningglory | 0 |
| Nutsedge | 0 | Nutsedge | 0 |
| Rape | 0 | Rape | 0 |
| Rice | 10 | Rice | 0 |
| Sorghum | 10 | Sorghum | 3 |
| Soybean | 0 | Soybean | 0 |
| Sugar beet | 0 | Sugar beet | 0 |
| Velvetleaf | 0 | Velvetleaf | 0 |
| Wheat | 10 | Wheat | 5 |
| Wild buckwheat | 0 | Wild buckwheat | 0 |
| Wild oat | 10 | Wild oat | 7 |

Table A

| Rate (50 g/ha) | COMPOUND 1 | 2 | 3 | 4 | 5 | 6 | 7 | 8 | 9 | 11 | 12 | 14 | 15 | 16 | 17 | 18 | 19 | 20 | 21 | 22 | 23 | 24 | 25 | 26 | 27 | 28 | 29 | 30 | 31 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley | 2 | 10 | 10 | 10 | 9 | 9 | 10 | 9 | 9 | 6 | 3 | 10 | 10 | 2 | 2 | 0 | 10 | 1 | 9 | 10 | 9 | 10 | 9 | 4 | 5 | 10 | 0 | 10 | 10 |
| Barnyardgrass | 0 | 10 | 10 | 10 | 9 | 10 | 10 | 9 | 9 | 10 | 9 | 10 | 9 | 3 | 0 | 0 | 10 | 0 | 2 | 10 | 10 | 10 | 9 | 0 | 0 | 10 | 0 | 10 | 10 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 0 | 9 | 9 | 9 | 8 | 9 | 9 | 4 | 6 | 8 | 0 | 9 | 9 | 0 | 8 | 0 | 9 | 0 | 8 | 9 | 10 | 9 | 9 | 0 | 7 | 10 | 0 | 10 | 9 |
| Cheatgrass | 0 | 9 | 9 | 9 | 5 | 9 | 9 | 7 | 3 | 7 | 0 | 9 | 8 | 0 | 0 | 0 | 9 | 1 | 5 | 10 | 10 | 10 | 9 | 2 | 3 | 9 | 0 | 9 | 9 |
| Chickweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 0 | 10 | 8 | 10 | 0 | 9 | 10 | 0 | 0 | 9 | 6 | 10 | 1 | 1 | 0 | 0 | 7 | 0 | 0 | 9 | 10 | 10 | 5 | 0 | 0 | 10 | 0 | 10 | 10 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 9 | 10 | 10 | 10 | 9 | 9 | 10 | 9 | 7 | 9 | 5 | 10 | 9 | 1 | 7 | 1 | 9 | 8 | 4 | 7 | 9 | 10 | 7 | 0 | 1 | 9 | 1 | 10 | 8 |
| Giant foxtail | 3 | 10 | 10 | 10 | 5 | 9 | 10 | 8 | 6 | 9 | 6 | 10 | 9 | 1 | 5 | 1 | 9 | 2 | 5 | 10 | 9 | 9 | 9 | 0 | 2 | 10 | 1 | 10 | 10 |
| Lambsquarters | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 |
| Rice | 9 | 10 | 10 | 9 | 9 | 3 | 9 | 9 | 9 | 7 | 7 | 10 | 10 | 2 | 9 | 6 | 9 | 9 | 9 | 9 | 9 | 10 | 10 | 0 | 10 | 10 | 0 | 10 | 9 |
| Sorghum | 0 | 10 | 10 | 10 | 5 | 10 | 10 | 9 | 7 | 9 | 8 | 10 | 9 | 1 | 2 | 0 | 10 | 4 | 9 | 10 | 10 | 10 | 5 | 0 | 0 | 10 | 0 | 10 | 9 |
| Soybean | 0 | 0 | 0 | 0 | 0 | 0 | 0 | 0 | 0 | 0 | 0 | 0 | 0 | 0 | 0 | 1 | 1 | 0 | 1 | 1 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 0 | 9 | 9 | 9 | 9 | 9 | 9 | 8 | 6 | 7 | 0 | 9 | 9 | 0 | 0 | 0 | 9 | 0 | 9 | 9 | 9 | 9 | 9 | 0 | 3 | 9 | 0 | 9 | 9 |
| Wild buckwheat | 0 | - | 0 | 0 | 0 | 0 | 0 | 0 | 0 | 0 | 0 | 0 | 0 | - | - | - | - | - | - | - | - | - | - | - | - | - | - | - | - |
| Wild oat | 0 | 9 | 9 | 9 | 0 | 9 | 9 | 5 | 0 | 4 | 0 | 9 | 0 | 0 | 0 | 0 | 9 | 0 | 1 | 9 | 9 | 9 | 9 | 8 | 0 | 8 | 0 | 9 | 9 |

Table A

| Rate (50 g/ha) | COMPOUND | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 |
| POSTEMERGENCE | | | | | | | | | | | | | | | | | | | | | | | | | | | | | |
| Barley | 3 | 1 | 0 | 10 | 10 | 10 | 10 | 9 | 7 | 9 | 10 | 9 | 2 | 0 | 9 | 9 | 9 | 10 | 3 | 2 | 9 | 3 | 9 | 10 | 9 | 8 | 10 | 9 | 3 |
| Barnyardgrass | 6 | 2 | 1 | 10 | 9 | 9 | 10 | 9 | 7 | 10 | 9 | 9 | 2 | 0 | 9 | 8 | 10 | 10 | 1 | 0 | 9 | 2 | 9 | 9 | 2 | 5 | 10 | 9 | 9 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 |
| Blackgrass | 0 | 0 | 0 | 10 | 9 | 9 | 9 | 9 | 9 | 9 | 9 | 9 | 0 | 0 | 8 | 5 | 7 | 6 | 5 | 0 | 5 | 3 | 9 | 9 | - | 6 | 9 | 9 | 8 |
| Cheatgrass | 1 | 1 | 0 | 10 | 9 | 9 | 9 | 9 | 7 | 5 | 9 | 7 | 1 | 0 | 7 | 2 | 9 | 9 | 2 | 0 | 2 | 1 | 9 | 9 | - | 2 | 10 | 9 | 2 |
| Chickweed | 0 | 0 | 0 | - | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 |
| Cocklebur | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 0 | 0 | 1 | 9 | 9 | 1 | 2 | 9 | 5 | 9 | 4 | 1 | 0 | 0 | 1 | 1 | 1 | 10 | 0 | 0 | 0 | 1 | 4 | 5 | 2 | 1 | 10 | 10 | 5 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 9 | 0 | 0 | 0 | 0 | 0 | 0 |
| Crabgrass | 2 | 9 | 1 | 10 | 7 | 7 | 9 | 7 | 9 | 8 | 4 | 3 | 0 | 0 | 5 | 7 | 2 | 6 | 5 | 3 | 3 | 8 | 3 | 7 | 9 | 3 | 10 | 6 | 6 |
| Giant foxtail | 6 | 1 | 1 | 10 | 9 | 8 | 7 | 6 | 7 | 5 | 9 | 9 | 2 | 0 | 5 | 3 | 9 | 9 | 6 | 1 | 2 | 3 | 8 | 8 | 7 | 4 | 10 | 3 | 7 |
| Lambsquarters | 0 | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | - | 0 | 0 | 0 | - |
| Morningglory | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 0 | 9 | 10 | 9 | 9 | 9 | 9 | 10 | 9 | 9 | 9 | 9 | 0 | 9 | 9 | 10 | 10 | 9 | 10 | 8 | 9 | 0 | 10 | 10 | 9 | 10 | 10 | 9 |
| Sorghum | 9 | 0 | 0 | 10 | 9 | 9 | 10 | 9 | 9 | 9 | 9 | 9 | 3 | 0 | 10 | 9 | 10 | 10 | 3 | 0 | 3 | 0 | 9 | 8 | 6 | 6 | 10 | 10 | 10 |
| Soybean | 0 | 0 | 0 | 0 | 3 | 0 | 0 | 2 | 1 | 0 | 0 | 0 | 0 | 0 | 0 | 0 | 0 | 0 | 0 | 0 | 0 | 0 | 6 | 0 | 0 | 0 | 1 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 0 | 1 | 0 | 9 | 9 | 9 | 9 | 8 | 8 | 8 | 9 | 8 | 2 | 0 | 9 | 7 | 9 | 9 | 8 | 0 | 7 | 1 | 9 | 9 | 8 | 5 | 10 | 9 | 2 |
| Wild buckwheat | - | - | - | - | - | - | 0 | - | 0 | - | - | - | - | - | - | - | - | - | - | - | - | - | - | - | - | - | - | - | - |
| Wild oat | 6 | 0 | 0 | 9 | 8 | 5 | 8 | 8 | 4 | 9 | 9 | 4 | 1 | 0 | 8 | 1 | 3 | 9 | 0 | 0 | 0 | 0 | 6 | 9 | 1 | 0 | 9 | 6 | 0 |

Table A

| Rate (50 g/ha) | COMPOUND 61 | 62 | 63 | 64 | 65 | 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley | 0 | 10 | 10 | 10 | 2 | 9 | 5 | 10 | 10 | 10 | 10 | 9 | 0 | 0 | 10 | 0 | 2 | 9 | 10 | 9 | 10 | 10 | 10 | 10 | 9 | 10 | 10 | 0 | 0 |
| Barnyardgrass | 0 | 10 | 10 | 8 | 1 | 10 | 9 | 10 | 10 | 9 | 10 | 9 | 0 | 0 | 10 | 0 | 5 | 9 | 10 | 0 | 10 | 10 | 10 | 10 | 10 | 10 | 10 | 0 | 0 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 0 | 10 | 10 | 8 | 0 | 9 | 7 | 10 | 9 | 9 | 9 | 9 | 0 | 0 | 9 | 0 | 2 | 1 | 8 | 0 | 8 | 8 | 8 | 7 | 7 | 9 | 10 | 0 | 2 |
| Cheatgrass | 0 | 10 | 10 | 8 | 2 | 9 | 7 | 10 | 9 | 9 | 9 | 7 | 0 | 0 | 10 | 0 | 2 | 1 | 9 | 2 | 9 | 10 | 10 | 8 | 6 | 9 | 10 | 0 | 0 |
| Chickweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 0 | 10 | 10 | 4 | 2 | 10 | 1 | 10 | 6 | 3 | 10 | 1 | 0 | 0 | 10 | 0 | 0 | 4 | 9 | 0 | 10 | 10 | 10 | 1 | 0 | 9 | 10 | 0 | 0 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 0 | 9 | 6 | 9 | 2 | 8 | 5 | 10 | 10 | 3 | 10 | 8 | 3 | 0 | 9 | 1 | 4 | 9 | 5 | 3 | 9 | 9 | 8 | 8 | 3 | 8 | 8 | 0 | 0 |
| Giant foxtail | 0 | 9 | 9 | 9 | 3 | 8 | 8 | 9 | 9 | 8 | 10 | 8 | 3 | 0 | 9 | 1 | 5 | 3 | 8 | 3 | 8 | 9 | 9 | 8 | 7 | 9 | 8 | 0 | 1 |
| Lambsquarters | 0 | 0 | 0 | 0 | 0 | 0 | - | 0 | 0 | 0 | 0 | - | - | - | 0 | - | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | - | 0 | 0 | 0 | - | - | 0 | 0 | - | 0 | 0 | 0 | 0 | 0 | 0 | 0 | - | - | - | - | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 10 | 10 | 10 | 9 | 9 | 9 | 10 | 10 | 10 | 10 | 10 | 9 | 0 | 10 | 0 | 0 | 1 | 10 | 9 | 10 | 10 | 0 | 10 | 10 | 10 | 10 | 0 | 9 |
| Sorghum | 0 | 10 | 10 | 9 | 0 | 10 | 10 | 10 | 10 | 10 | 10 | 9 | 0 | 0 | 9 | 0 | 9 | 6 | 10 | 9 | 10 | 10 | 10 | 9 | 9 | 10 | 10 | 0 | 0 |
| Soybean | 0 | 0 | 0 | 0 | 0 | 1 | 0 | 0 | 0 | 0 | 0 | 0 | 0 | 0 | 0 | 0 | 0 | 0 | 8 | 0 | 0 | 0 | 10 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 2 | 0 | 0 | 0 | 0 | 0 | 0 | - | 0 | 0 | - | 0 | 0 | 0 | 0 |
| Wheat | 0 | 10 | 10 | 10 | 2 | 9 | 9 | 10 | 9 | 10 | 10 | 9 | 0 | 0 | 10 | 0 | 2 | 9 | 9 | 8 | 10 | 10 | 10 | 9 | 9 | 10 | 10 | 0 | 0 |
| Wild buckwheat | 0 | - | - | 0 | 0 | 0 | - | 0 | 0 | 0 | 0 | 0 | - | 0 | - | - | 0 | 0 | 0 | 0 | 0 | 0 | 0 | 0 | 0 | 0 | 0 | 0 | 0 |
| Wild oat | 0 | 10 | 8 | 5 | 2 | 9 | 1 | 4 | 9 | 9 | 9 | 2 | 0 | 0 | 9 | 0 | 2 | 0 | 3 | 0 | 7 | 9 | 9 | 2 | 1 | 7 | 9 | 0 | 0 |

Table A

COMPOUND

| Rate (50 g/ha) | 91 | 92 | 93 | 94 | 96 | 97 | 98 | 99 | 100 | 101 | 102 | 103 | 104 | 105 | 106 | 107 | 108 | 109 | 110 | 111 | 112 | 113 | 114 | 115 |
|---|---|---|---|---|---|---|---|---|---|---|---|---|---|---|---|---|---|---|---|---|---|---|---|---|
| POSTEMERGENCE | | | | | | | | | | | | | | | | | | | | | | | | |
| Barley | 10 | 10 | 1 | 10 | 10 | 0 | 10 | 10 | 10 | 6 | 0 | 2 | 2 | 9 | 0 | 0 | 0 | 0 | 3 | 0 | 0 | 9 | 10 | 0 |
| Barnyardgrass | 10 | 10 | 0 | 10 | 10 | 1 | 10 | 10 | 6 | 10 | 7 | 2 | 10 | 7 | 3 | 0 | 9 | 9 | 9 | 0 | 0 | 10 | 10 | 0 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 10 | 10 | - | 9 | 10 | 0 | 10 | 10 | 9 | 0 | 0 | 2 | 0 | 0 | 0 | 0 | 0 | 0 | 0 | 0 | 0 | 0 | 10 | 0 |
| Cheatgrass | 10 | 10 | 1 | 9 | 9 | 0 | 10 | 10 | 3 | 0 | 0 | 0 | 0 | 0 | 0 | 0 | 0 | 0 | 0 | 0 | 0 | 1 | 10 | 0 |
| Chickweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 9 | 10 | 0 | 10 | 7 | 2 | 10 | 10 | 5 | 6 | 1 | 0 | 0 | 2 | 0 | 0 | 10 | 6 | 8 | 0 | 0 | 10 | 10 | 0 |
| Cotton | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 |
| Crabgrass | 10 | 9 | - | 8 | 6 | 8 | 10 | 10 | 9 | 3 | 0 | 6 | 7 | 4 | 1 | 0 | 8 | 6 | 5 | 0 | 0 | 9 | 8 | 0 |
| Giant foxtail | 10 | 10 | 3 | 9 | 8 | 5 | 9 | 9 | 6 | 3 | 0 | 7 | 2 | 2 | 2 | 0 | 6 | 2 | 6 | 0 | 0 | 7 | 9 | 0 |
| Lambsquarters | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 10 | 10 | 10 | 10 | 10 | 9 | 10 | 10 | 9 | 2 | 0 | 0 | 0 | 9 | 0 | 0 | 4 | 0 | 1 | 0 | 0 | 9 | 10 | 0 |
| Sorghum | 10 | 10 | 0 | 10 | 9 | 1 | 10 | 10 | 9 | 8 | 0 | 10 | 4 | 9 | 0 | 0 | 9 | 5 | 9 | - | - | - | 10 | 0 |
| Soybean | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 10 | 10 | 2 | 10 | 9 | 0 | 9 | 8 | 10 | 0 | 0 | 0 | 0 | 7 | 0 | 0 | 0 | 0 | 0 | 0 | 0 | 9 | 10 | 0 |
| Wild buckwheat | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 |
| Wild oat | 9 | 8 | 0 | 3 | 0 | 0 | 9 | 5 | 0 | 0 | 0 | 8 | 0 | 0 | 0 | 0 | 0 | 0 | 2 | 0 | 0 | 2 | 9 | 0 |

| Table A | COMPOUND | | | | | | | | | | | | | |
|---|---|---|---|---|---|---|---|---|---|---|---|---|---|---|
| Rate (50 g/ha) | 116 | 117 | 118 | 119 | 120 | 121 | 122 | 123 | 124 | 125 | 126 | 127 | 128 | 129 |
| POSTEMERGENCE | | | | | | | | | | | | | | |
| Barley | 5 | 0 | 9 | 4 | 10 | 9 | 9 | 10 | 10 | 1 | 10 | 0 | 10 | 7 |
| Barnyardgrass | 10 | 2 | 10 | 8 | 10 | 10 | 10 | 10 | 7 | 2 | 9 | 0 | 10 | 3 |
| Bedstraw | 0 | 0 | 0 | 0 | 0 | 0 | 0 | 0 | 0 | 0 | 0 | 0 | 0 | 0 |
| Blackgrass | 0 | 0 | 5 | 1 | 5 | 5 | 9 | 9 | 4 | 2 | 3 | 0 | 9 | 0 |
| Cheatgrass | 0 | 1 | 8 | 2 | 9 | 9 | 7 | 10 | 3 | 0 | 3 | 0 | 4 | 0 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 5 | 0 | 10 | 5 | 10 | 10 | 10 | 10 | 0 | 0 | 7 | 0 | 10 | 1 |
| Cotton | 0 | 0 | 0 | 0 | 0 | 0 | 0 | 0 | 0 | 0 | 0 | 0 | 0 | 0 |
| Crabgrass | 2 | 1 | 8 | 5 | 9 | 9 | 6 | 10 | 9 | 7 | 8 | 0 | 9 | 4 |
| Giant foxtail | 9 | 3 | 9 | 3 | 9 | 9 | 9 | 10 | 7 | 6 | 9 | 0 | 10 | 2 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 0 | 0 | 0 | 0 | 0 | 0 | 0 |
| Rape | 1 | 0 | 0 | 0 | 0 | 0 | 0 | 0 | 0 | 0 | 0 | 0 | 0 | 0 |
| Rice | 0 | 0 | 1 | 0 | 5 | 2 | 9 | 9 | 10 | 9 | 9 | 0 | 10 | 9 |
| Sorghum | 9 | 9 | 10 | 9 | 10 | 10 | 10 | 10 | 10 | 9 | 10 | 0 | 10 | 9 |
| Soybean | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 3 | 0 | 0 | 0 | 0 | 0 | 0 | 0 |
| Wheat | 2 | 0 | 5 | 1 | 9 | 9 | 9 | 10 | 10 | 1 | 9 | 0 | 10 | 6 |
| Wild buckwheat | 0 | 0 | 0 | 0 | 0 | 0 | 0 | 0 | 0 | 0 | 0 | 0 | 0 | 0 |
| Wild oat | 9 | 2 | 9 | 7 | 9 | 9 | 2 | 10 | 0 | 0 | 0 | 0 | 6 | 0 |

Table A

| | COMPOUND | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| Rate (50 g/ha) | 1 | 2 | 3 | 4 | 5 | 6 | 7 | 8 | 9 | 11 | 12 | 14 | 15 | 16 | 17 | 18 | 19 | 20 | 21 | 22 | 23 | 24 | 25 | 26 | 27 | 28 | 29 | 30 | 31 |
| PREEMERGENCE | | | | | | | | | | | | | | | | | | | | | | | | | | | | | |
| Barley | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Barnyardgrass | 1 | 2 | 1 | 1 | 0 | 0 | 2 | 0 | 0 | 0 | 0 | 0 | 4 | 0 | 0 | 0 | 0 | 0 | 0 | 0 | 0 | 3 | 0 | 0 | 0 | 1 | 0 | 0 | 0 |
| Bedstraw | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 | 0 |
| Blackgrass | 0 | 4 | 0 | 5 | 0 | 0 | 2 | 4 | 0 | 0 | 0 | 0 | 0 | 0 | 0 | 0 | 0 | 0 | 0 | 0 | 0 | 0 | 3 | 0 | 3 | 7 | 0 | 0 | 0 |
| Cheatgrass | 0 | 0 | 0 | 0 | 0 | 0 | 0 | 0 | 0 | 0 | 0 | 1 | 3 | 0 | 0 | 0 | 0 | 0 | 0 | 0 | 0 | 0 | 0 | 0 | 0 | 2 | 0 | 0 | 0 |
| Chickweed | 0 | 0 | 0 | 0 | 0 | 0 | 0 | 0 | 0 | 0 | 0 | 0 | 0 | - | 0 | 0 | - | 0 | - | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 |
| Corn | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 2 | 0 |
| Cotton | 0 | 0 | 0 | 0 | 0 | 0 | 0 | 2 | 0 | 0 | 0 | 0 | 0 | 0 | - | 0 | 0 | 0 | 0 | 0 | 0 | - | 0 | 0 | 0 | 0 | 0 | 0 | 0 |
| Crabgrass | 3 | 5 | 5 | 7 | 1 | 1 | 6 | 7 | 1 | 0 | 0 | 9 | 8 | 0 | 5 | 0 | 3 | 0 | 0 | 9 | 4 | 7 | 4 | 0 | 0 | 4 | 0 | 9 | 9 |
| Giant foxtail | 1 | 6 | 5 | 4 | 1 | 0 | 7 | 7 | 1 | 0 | 0 | 3 | 4 | 0 | 4 | 0 | 1 | 0 | 0 | 2 | 4 | 2 | 3 | 0 | 0 | 6 | 0 | 6 | 3 |
| Lambsquarters | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 9 | 0 | 0 | 0 | 0 | 0 | - | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sorghum | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Soybean | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wild buckwheat | - | - | - | - | 0 | - | - | - | - | 0 | 0 | - | - | - | - | - | - | - | - | - | - | - | - | - | - | - | - | - | - |
| Wild oat | 0 | 0 | 0 | 0 | 0 | 0 | 0 | 0 | 0 | 0 | 0 | 1 | 0 | 0 | 0 | 0 | 0 | 0 | 0 | 0 | 2 | 0 | 2 | 0 | 0 | 0 | 0 | 0 | 0 |

Table A

| COMPOUND | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| Rate (50 g/ha) | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 |
| PREEMERGENCE | | | | | | | | | | | | | | | | | | | | | | | | | | | | | |
| Barley | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Barnyardgrass | 0 | 0 | 0 | 0 | 1 | 0 | 0 | 0 | 0 | 0 | 0 | 1 | 0 | 0 | 0 | 0 | 0 | 3 | 0 | 0 | 0 | 0 | 0 | 0 | 0 | 0 | 0 | 0 | 0 |
| Bedstraw | 0 | 0 | 0 | 0 | 0 | 0 | 0 | 0 | 0 | 0 | 0 | 0 | 0 | 0 | 0 | 0 | 0 | 0 | - | - | - | - | - | 0 | 0 | 0 | 0 | 0 | 0 |
| Blackgrass | 0 | 0 | 0 | 2 | 0 | 0 | 0 | 0 | 0 | 0 | 0 | 2 | 0 | 0 | 0 | 0 | 0 | 0 | 0 | 0 | 0 | 0 | 0 | 0 | 2 | 0 | 3 | 0 | 0 |
| Cheatgrass | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Chickweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 |
| Cotton | 0 | 0 | - | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 |
| Crabgrass | 2 | 2 | 1 | 3 | 3 | 3 | 0 | 0 | 3 | 1 | 2 | 2 | 2 | 0 | 0 | 0 | 0 | 7 | 6 | 0 | 0 | 0 | 0 | 2 | 3 | 0 | 0 | 0 | 0 |
| Giant foxtail | 0 | 0 | 0 | 3 | 1 | 2 | 0 | 0 | 3 | 0 | 2 | 2 | 0 | 0 | 0 | 0 | 0 | 4 | 3 | 0 | 0 | 0 | 0 | 2 | 4 | 0 | 2 | 0 | 1 |
| Lambsquarters | 0 | 0 | 0 | - | 0 | 0 | 0 | - | 0 | 0 | 0 | 0 | - | 0 | 0 | 0 | 0 | 0 | 0 | 0 | 0 | 0 | 0 | 0 | - | - | 0 | - | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | - |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 |
| Sorghum | 0 | 0 | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 |
| Soybean | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wild buckwheat | - | - | - | 0 | - | - | 0 | 0 | 0 | - | - | - | - | - | - | - | - | - | - | - | - | - | - | - | 0 | 0 | 0 | 0 | 0 |
| Wild oat | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

Table A

| COMPOUND | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| Rate (50 g/ha) | 61 | 62 | 63 | 64 | 65 | 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 |
| PREEMERGENCE | | | | | | | | | | | | | | | | | | | | | | | | | | | | | |
| Barley | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Barnyardgrass | 0 | 3 | 0 | 0 | 0 | 0 | 0 | 2 | 0 | 0 | 3 | 0 | 0 | 0 | 0 | 0 | 0 | 1 | 0 | 0 | 3 | 2 | 7 | 3 | 5 | 5 | 3 | 0 | 0 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 0 | 2 | 3 | 0 | 2 | 4 | 0 | 2 | 0 | 0 | 9 | 0 | 0 | 0 | 3 | 0 | 0 | 2 | 2 | 0 | 0 | 2 | 2 | 2 | 0 | 3 | 0 | 0 | 0 |
| Cheatgrass | 0 | 1 | 0 | 0 | 2 | 3 | 0 | 0 | 0 | 0 | 5 | 0 | 0 | 0 | 2 | 0 | 0 | 0 | 0 | 0 | 0 | 0 | 0 | 0 | 0 | 0 | 0 | 0 | 0 |
| Chickweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Cocklebur | - | 0 | 0 | 0 | - | 0 | 0 | 0 | 0 | - | 0 | 0 | 0 | 0 | - | 0 | 0 | 0 | - | 0 | 0 | 0 | 0 | - | - | 0 | 0 | 0 | 0 |
| Corn | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 1 | 0 | 0 | 0 | 0 | 0 | 0 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 0 | 5 | 0 | 0 | 0 | 0 | 0 | 7 | 4 | 2 | 4 | 0 | 2 | 0 | 0 | 0 | 0 | 3 | 1 | 0 | 9 | 9 | 7 | 2 | 5 | 9 | 6 | 0 | 0 |
| Giant foxtail | 0 | 9 | 6 | 0 | 0 | 0 | 0 | 2 | 4 | 4 | 3 | 3 | 4 | 0 | 0 | 0 | 0 | 2 | 5 | 0 | 3 | 5 | 3 | 3 | 5 | 9 | 3 | 0 | 0 |
| Lambsquarters | 0 | 0 | 0 | 0 | 0 | 0 | 0 | 0 | - | - | - | 0 | 0 | 0 | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | - | 0 | 0 | 0 | 0 | 0 | 0 | - | 0 | 0 | 0 | 0 | 0 | 0 | 8 | 0 | 0 | - | 0 | 0 | 0 | - | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sorghum | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 |
| Soybean | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wild buckwheat | 0 | 0 | - | 0 | 0 | 0 | 0 | 0 | 0 | - | 0 | 0 | 0 | 0 | 0 | 0 | 0 | 0 | 0 | 0 | 0 | 0 | 0 | - | 0 | 0 | 0 | 0 | 0 |
| Wild oat | 0 | 0 | 0 | 0 | 0 | 0 | 0 | 0 | 0 | 0 | 3 | 0 | 0 | 0 | 2 | 0 | 0 | 0 | 0 | 0 | 2 | 0 | 0 | 0 | 0 | 0 | 0 | 0 | 0 |

Table A

| | COMPOUND | | | | | | | | | | | | | | | | | | | | | | | |
|---|---|---|---|---|---|---|---|---|---|---|---|---|---|---|---|---|---|---|---|---|---|---|---|---|
| Rate (50 g/ha) | 91 | 92 | 93 | 94 | 96 | 97 | 98 | 99 | 100 | 101 | 102 | 103 | 104 | 105 | 106 | 107 | 108 | 109 | 110 | 111 | 112 | 113 | 114 | 115 |
| PREEMERGENCE | | | | | | | | | | | | | | | | | | | | | | | | |
| Barley | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Barnyardgrass | 0 | 1 | 0 | 0 | 3 | 0 | 1 | 2 | 0 | 0 | 0 | 0 | 0 | 0 | 0 | 0 | 0 | 0 | 0 | 0 | 0 | 2 | 8 | 0 |
| Bedstraw | 0 | 0 | - | 0 | 0 | 0 | 0 | 0 | 0 | - | 0 | 0 | 0 | 0 | 2 | 0 | 0 | 0 | 0 | 0 | 0 | 0 | 0 | 0 |
| Blackgrass | 0 | 2 | 0 | 0 | - | 0 | 3 | 0 | 0 | 0 | 0 | 0 | 0 | 0 | 0 | 0 | 0 | 0 | 0 | 0 | 0 | 0 | 6 | 0 |
| Cheatgrass | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 |
| Chickweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Cocklebur | 0 | 0 | 0 | 0 | - | 0 | 0 | 0 | 0 | 0 | 0 | 0 | 0 | - | 0 | 0 | 0 | 0 | 0 | 0 | 0 | 0 | - | - |
| Corn | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 6 | 8 | 3 | 0 | 3 | 0 | 5 | 6 | 0 | 0 | 3 | 2 | 0 | - | 0 | 0 | 2 | 2 | 3 | 0 | 0 | 6 | 9 | 0 |
| Giant foxtail | 2 | 0 | 0 | 0 | 7 | 0 | 1 | 1 | 0 | 0 | 0 | 0 | 0 | 0 | 0 | 0 | 0 | 0 | 0 | 0 | 0 | 6 | 6 | 0 |
| Lambsquarters | 0 | 0 | 0 | 0 | - | 0 | 0 | 5 | 0 | 0 | 0 | 0 | 0 | 0 | 9 | 0 | 0 | 0 | 0 | 0 | - | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | - | 0 | 0 | 0 | 0 | 0 | 0 | 0 | 0 | 0 | 0 | 0 | 0 | 0 | 0 | 0 | 0 | 0 | 0 | 0 | 0 | - |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rice | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sorghum | 0 | 0 | 0 | 0 | 0 | 0 | 0 | 0 | 0 | 0 | 0 | - | 0 | 0 | 0 | 0 | 0 | 0 | 0 | 0 | 0 | 0 | 1 | 0 |
| Soybean | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wild buckwheat | 0 | 0 | 0 | - | - | 0 | 0 | 0 | 0 | 0 | 0 | 0 | 0 | 0 | 0 | 0 | 0 | 0 | 0 | 0 | 0 | 0 | 0 | 0 |
| Wild oat | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

| Table A | COMPOUND | | | | | | | | | | | | | |
|---|---|---|---|---|---|---|---|---|---|---|---|---|---|---|
| Rate (50 g/ha) | 116 | 117 | 118 | 119 | 120 | 121 | 122 | 123 | 124 | 125 | 126 | 127 | 128 | 129 |
| **PREEMERGENCE** | | | | | | | | | | | | | | |
| Barley | 0 | 0 | 0 | 0 | 0 | 0 | 0 | 0 | 0 | 0 | 0 | 0 | 0 | 0 |
| Barnyardgrass | 0 | 0 | 0 | 0 | 0 | 0 | 0 | 0 | 0 | 0 | 0 | 0 | 0 | 0 |
| Bedstraw | 0 | 0 | 0 | 0 | 0 | 0 | 0 | 0 | 0 | 0 | 0 | 0 | 0 | 0 |
| Blackgrass | 0 | 0 | 0 | 0 | 0 | 0 | 3 | 3 | 0 | 5 | 0 | 0 | 0 | 0 |
| Cheatgrass | 0 | 0 | 0 | 0 | 0 | 0 | 3 | 3 | 0 | 0 | 0 | 0 | 0 | 0 |
| Chickweed | 0 | 0 | 0 | 0 | 0 | 0 | 0 | 0 | 0 | 0 | 0 | 0 | 0 | 0 |
| Cocklebur | 0 | 0 | 1 | 0 | 0 | 0 | 0 | 0 | 0 | 0 | 0 | 0 | 0 | 0 |
| Corn | 0 | 0 | 0 | 0 | 0 | 0 | 0 | 0 | 0 | 0 | 0 | 0 | 0 | 0 |
| Cotton | 0 | 0 | 0 | 0 | 0 | 0 | 0 | 0 | 0 | 1 | 0 | 0 | 0 | 0 |
| Crabgrass | 0 | 0 | 2 | 0 | 0 | 0 | 0 | 3 | 0 | 0 | 0 | 0 | 0 | 0 |
| Giant foxtail | 0 | 0 | 2 | 0 | 0 | 0 | 0 | 3 | 0 | 0 | 0 | 0 | 0 | 0 |
| Lambsquarters | 0 | 0 | 0 | 0 | 0 | 0 | 0 | 0 | 0 | 0 | 1 | 0 | 0 | 0 |
| Morningglory | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 0 | 0 | 0 | 0 | 0 | 1 | 0 |
| Rape | 0 | 0 | 0 | 0 | 0 | 0 | 0 | 0 | 0 | 0 | 0 | 0 | 0 | 0 |
| Rice | 0 | 0 | 0 | 0 | 0 | 0 | 0 | 0 | 0 | 0 | 0 | 0 | 0 | 0 |
| Sorghum | 0 | 0 | 0 | 0 | 0 | 0 | 0 | 0 | 0 | 0 | 0 | 0 | 0 | 0 |
| Soybean | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 |
| Wheat | 0 | 0 | 0 | 0 | 0 | 0 | 0 | 0 | 0 | 0 | 0 | 0 | 0 | 0 |
| Wild buckwheat | 0 | 0 | 0 | 0 | 0 | 0 | 0 | 0 | 0 | 0 | 0 | 0 | 0 | 0 |
| Wild oat | 0 | 0 | 0 | 0 | 0 | 0 | 2 | 2 | 0 | 0 | 0 | 0 | 2 | 0 |

## TEST B

The compounds evaluated in this test were formulated in a non-phytoxic solvent and applied to the soil surface before plant seedlings emerged (preemergence application), to water that covered the soil surface (paddy application), and to plants that were in the one-to-four leaf stage (postemergence application). A sandy loam soil was used for the preemergence and postemergence tests, while a silt loam soil was used in the paddy test. Water depth was approximately 2.5 cm for the paddy test and was maintained at this level for the duration of the test.

Plant species in the preemergence and postemergence tests consisted of barley (Hordeum vulgare), bedstraw (Galium aparine), blackgrass (Alopecurus myosuroides), chickweed (Stellaria media), corn (Zea mays), cotton (Gossypium hirsutum), crabgrass (Digitaria sanguinalis), downy brome (Bromus tectorum), giant foxtail (Setaria faberii), lambsquarters (Chenopodium album), morningglory (Ipomoea hederacea), pigweed (Amaranthusretroflexus), rape (Brassica napus), ryegrass (Lolium multiflorum), sorghum (Sorghum bicolor), soybean (Glycine max), speedwell (Veronica persica), sugar beet (Beta vulgaris), velvetleaf (Abutilon theophrasti), wheat (Triticum aestivum), wild buckwheat (Polygonum convolvulus), and wild oat (Avena fatua). All plant species were planted one day before application of the compound for the preemergence portion of this test. Plantings of these species were adjusted to produce plants of appropriate size for the postemergence portion of the test. Plant species in the paddy test consisted of barnyardgrass (Echinochloa crus-galli), rice (Oryza sativa), umbrella sedge (Cyperus difformis) and duck salad (Heteranthera limosa).

All plant species were grown using normal greenhouse practices. Visual evaluations of injury expressed on treated plants, when compared to untreated controls, were recorded approximately fourteen to twenty one days after application of the test compound. Plant response ratings, summarized in Table B, were recorded on a 0 to 10 scale where 0 is no effect and 10 is complete control. A dash (-) response means no test result.

| Table B | COMPOUND | | | | | | |
|---|---|---|---|---|---|---|---|
| Rate (500 g/ha) | 5 | 8 | 15 | 48 | 52 | 53 | 103 |
| POSTEMERGENCE | | | | | | | |
| Barley Igri | 9 | 9 | 9 | 10 | 10 | 0 | 6 |
| Bedstraw | 0 | 0 | 0 | 0 | 0 | 0 | 0 |
| Blackgrass | 9 | 9 | - | 10 | 9 | 5 | 0 |
| Chickweed | 0 | 0 | 0 | 0 | 0 | 0 | 0 |
| Corn | 2 | 2 | 2 | 5 | 2 | 0 | 1 |
| Cotton | 0 | 0 | 0 | 0 | 0 | 0 | 0 |
| Crabgrass | 9 | 8 | 10 | 10 | 3 | 2 | 10 |
| Downy brome | 0 | 0 | 0 | 3 | 0 | 0 | 4 |
| Duck salad | 0 | 0 | 0 | 4 | 0 | 0 | 0 |
| Giant foxtail | 6 | 8 | 9 | 10 | 5 | 8 | 10 |
| Lambsquarters | 0 | 0 | 0 | 0 | 0 | 0 | 0 |
| Morningglory | 0 | 0 | 0 | 0 | 0 | 0 | 0 |
| Pigweed | 0 | 0 | 0 | 0 | 0 | 0 | 0 |
| Rape | 0 | 0 | 0 | 0 | 0 | 0 | 0 |
| Ryegrass | 0 | 0 | 0 | 2 | 0 | 0 | 4 |
| Sorghum | 10 | 9 | 10 | 10 | 10 | 3 | 10 |
| Soybean | 0 | 0 | 0 | 0 | 0 | 0 | 0 |
| Speedwell | 0 | 0 | 0 | 0 | 0 | 0 | 0 |
| Sugar beet | 0 | 0 | 0 | 0 | 0 | 0 | 0 |
| Velvetleaf | 0 | 0 | 0 | 0 | 0 | 0 | 0 |
| Wheat | 9 | 8 | 9 | 10 | 5 | 0 | 2 |
| Wild buckwheat | 0 | 0 | 0 | 0 | 0 | 0 | 0 |
| Wild oat | 0 | 0 | 0 | 2 | 0 | 2 | 7 |
| Barnyardgrass | 10 | 10 | 10 | 10 | 9 | 0 | 10 |
| Rice Japonica | 3 | 6 | 10 | 10 | 4 | 2 | 0 |
| Umbrella sedge | 3 | 0 | 0 | 0 | 0 | 0 | 0 |

| Table B | COMPOUND | | | | | |
|---|---|---|---|---|---|---|
| Rate (500 g/ha) | 5 | 8 | 15 | 48 | 52 | 53 |
| PREEMERGENCE | | | | | | |
| Barley Igri | 0 | 0 | 0 | 0 | - | 0 |
| Bedstraw | 0 | 0 | 0 | 0 | 0 | 0 |
| Blackgrass | 9 | 10 | 10 | 10 | 10 | 0 |
| Chickweed | 0 | 0 | 0 | 0 | 0 | 0 |
| Corn | 2 | 0 | 0 | 9 | 4 | 2 |
| Cotton | 0 | 0 | 0 | 2 | 0 | 0 |
| Crabgrass | 10 | 10 | 10 | 10 | 10 | 8 |
| Downy brome | 0 | 0 | 2 | 0 | 0 | 0 |
| Duck salad | - | - | - | - | - | - |
| Giant foxtail | 8 | 10 | 10 | 10 | 10 | 5 |
| Lambsquarters | 4 | 0 | 0 | 0 | 0 | 0 |
| Morningglory | 0 | 0 | 0 | 0 | 2 | 0 |
| Pigweed | 0 | 0 | 0 | 0 | 0 | 3 |
| Rape | 0 | 0 | 0 | 0 | 0 | 0 |
| Ryegrass | 4 | 10 | 10 | 0 | 0 | 0 |
| Sorghum | 3 | 3 | 3 | 10 | 7 | 2 |
| Soybean | 0 | 0 | 0 | 0 | 1 | 1 |
| Speedwell | 4 | 0 | 0 | 0 | 0 | 0 |
| Sugar beet | 0 | 0 | 0 | 0 | 0 | 0 |
| Velvetleaf | 0 | 0 | 0 | 0 | 0 | 0 |
| Wheat | 0 | 0 | 0 | 0 | 0 | 0 |
| Wild buckwheat | 3 | 6 | 0 | 0 | 0 | 0 |
| Wild oat | 0 | 4 | 0 | 0 | 0 | 0 |
| Barnyardgrass | - | - | - | - | - | - |
| Rice Japonica | - | - | - | - | - | - |
| Umbrella sedge | - | - | - | - | - | - |

| Table B | COMPOUND | | | | | | | | | | | | | |
|---|---|---|---|---|---|---|---|---|---|---|---|---|---|---|
| Rate(250 g/ha) | 5 | 6 | 8 | 10 | 15 | 26 | 32 | 36 | 37 | 48 | 52 | 53 | 78 | 113 |
| POSTEMERGENCE | | | | | | | | | | | | | | |
| Barley Igri | 9 | 10 | 9 | 10 | 9 | 10 | 3 | - | 10 | 10 | 9 | 0 | 3 | 6 |
| Bedstraw | 0 | 0 | 0 | 0 | 0 | 0 | 0 | - | 0 | 0 | 0 | 0 | 2 | 0 |
| Blackgrass | 9 | 10 | 9 | 10 | 10 | 3 | 9 | - | 10 | 10 | 9 | 5 | 8 | 7 |
| Chickweed | 0 | 0 | 0 | 0 | 0 | 2 | 0 | - | 0 | 0 | 0 | 0 | 5 | 0 |
| Corn | 2 | 10 | 2 | 10 | 2 | 3 | 1 | - | - | 3 | 0 | 0 | 10 | 10 |
| Cotton | 0 | 0 | 0 | 0 | 0 | 0 | 0 | - | 0 | 0 | 0 | 0 | 0 | 0 |
| Crabgrass | 9 | 10 | 8 | 10 | 10 | 2 | 10 | - | 10 | 10 | 2 | 0 | 10 | 10 |
| Downy brome | 0 | 10 | 0 | 10 | 0 | 6 | 0 | - | 0 | 3 | 0 | 0 | 0 | 2 |
| Duck salad | 0 | 0 | 0 | 0 | 0 | 0 | 0 | 0 | 0 | 3 | 0 | 0 | 0 | 0 |
| Giant foxtail | 5 | 10 | 8 | 10 | 9 | 4 | 10 | - | 10 | 10 | 4 | 8 | 10 | 10 |
| Lambsquarters | 0 | 0 | 0 | 0 | 0 | - | 0 | - | 0 | 0 | 0 | 0 | 6 | 0 |
| Morningglory | 0 | 0 | 0 | 0 | 0 | 0 | 0 | - | 0 | 0 | 0 | 0 | 0 | 0 |
| Pigweed | 0 | 0 | 0 | 0 | 0 | 0 | 0 | - | 0 | 0 | 0 | 0 | 0 | 0 |
| Rape | 0 | 0 | 0 | 0 | 0 | 0 | 0 | - | 0 | 0 | 0 | 0 | 7 | 0 |
| Ryegrass | 0 | - | 0 | 10 | 0 | 0 | 0 | - | 4 | 0 | 0 | 0 | 0 | 2 |
| Sorghum | 10 | 10 | 9 | 10 | 10 | 2 | 10 | - | 10 | 10 | 10 | 3 | 10 | 10 |
| Soybean | 0 | 0 | 0 | 0 | 0 | 0 | 0 | - | 0 | 0 | 0 | 0 | 0 | 0 |
| Speedwell | 0 | 0 | 0 | 0 | 0 | 0 | 2 | - | 0 | 0 | 0 | 0 | 5 | 0 |
| Sugar beet | 0 | 0 | 0 | 0 | 0 | 0 | 0 | - | 0 | 0 | 0 | 0 | 9 | 0 |
| Velvetleaf | 0 | 0 | 0 | 0 | 0 | 0 | 0 | - | 0 | 0 | 0 | 0 | 0 | 0 |
| Wheat | 8 | 10 | 7 | 10 | 9 | 0 | 4 | - | 10 | 10 | 5 | 0 | 2 | 7 |
| Wild buckwheat | - | 0 | 0 | 0 | 0 | 0 | 0 | - | 0 | 0 | 0 | 0 | 8 | 0 |
| Wild oat | 0 | 10 | 0 | 7 | 0 | 5 | 3 | - | 3 | 1 | 0 | 0 | 9 | 0 |
| Barnyardgrass | 10 | 10 | 10 | 10 | 10 | 10 | 10 | 10 | 10 | 10 | 9 | 0 | 10 | 6 |
| Rice Japonica | 0 | 7 | 5 | 10 | 9 | 0 | 0 | 10 | 10 | 10 | 3 | 0 | 0 | 4 |
| Umbrella sedge | 3 | 0 | 0 | 0 | 0 | 0 | 0 | 0 | 0 | 0 | 0 | 0 | 0 | 0 |

| Table B | COMPOUND | | | | | | | |
|---|---|---|---|---|---|---|---|---|
| Rate (250 g/ha) | 5 | 6 | 8 | 10 | 15 | 48 | 52 | 53 |
| PREEMERGENCE | | | | | | | | |
| Barley Igri | 0 | 0 | 0 | 0 | 0 | 0 | 0 | 0 |
| Bedstraw | 0 | 0 | 0 | 0 | 0 | 0 | 0 | 0 |
| Blackgrass | 4 | 7 | 10 | 10 | 10 | 10 | 9 | 0 |
| Chickweed | 0 | 0 | 0 | 0 | - | 0 | 0 | 0 |
| Corn | 0 | 0 | 0 | 4 | 0 | 4 | 2 | 2 |
| Cotton | 0 | 0 | 0 | 0 | 0 | 0 | 0 | 0 |
| Crabgrass | 10 | 10 | 10 | 10 | 10 | 10 | 8 | 8 |
| Downy brome | 0 | 0 | 0 | 0 | 0 | 0 | 0 | 0 |
| Duck salad | - | - | - | - | - | - | - | - |
| Giant foxtail | 5 | 10 | 10 | 10 | 9 | 10 | 10 | 5 |
| Lambsquarters | 0 | 3 | 0 | 0 | 0 | 0 | 0 | 0 |
| Morningglory | 0 | 0 | 0 | 0 | 0 | 0 | 0 | 0 |
| Pigweed | 0 | 0 | 0 | 0 | 0 | 0 | 0 | 0 |
| Rape | 0 | 0 | 0 | 0 | 0 | 0 | 0 | 0 |
| Ryegrass | 0 | 0 | 7 | 0 | 4 | 0 | 0 | 0 |
| Sorghum | 2 | 0 | 2 | 10 | 2 | 6 | 4 | 2 |
| Soybean | 0 | 0 | 0 | 0 | 0 | 0 | 0 | 0 |
| Speedwell | 2 | 4 | 0 | 0 | - | 0 | 0 | 0 |
| Sugar beet | 0 | 4 | 0 | 0 | 0 | 0 | 0 | 0 |
| Velvetleaf | 0 | 0 | 0 | 0 | 0 | 0 | 0 | 0 |
| Wheat | 0 | 0 | 0 | 4 | 0 | 0 | 0 | 0 |
| Wild buckwheat | 0 | 9 | 0 | 0 | - | 0 | - | - |
| Wild oat | 0 | 0 | 0 | 0 | 0 | 0 | 0 | 0 |
| Barnyardgrass | - | - | - | - | - | - | - | - |
| Rice Japonica | - | - | - | - | - | - | - | - |
| Umbrella sedge | - | - | - | - | - | - | - | - |

Table B

| Rate (125 g/ha) | COMPOUND 5 | 6 | 8 | 10 | 15 | 19 | 22 | 23 | 24 | 26 | 28 | 32 | 36 | 37 | 38 | 39 | 40 | 41 | 42 | 43 | 46 | 47 | 48 | 52 | 53 | 55 | 56 | 58 | 59 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley Igri | 8 | 10 | 9 | 10 | 9 | 10 | 10 | 10 | 10 | 10 | 10 | 0 | 10 | 10 | 10 | 6 | 0 | 9 | 10 | 9 | 10 | 0 | 10 | 8 | 0 | 10 | 10 | 10 | 10 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 9 | 10 | 9 | 10 | 10 | 10 | 10 | 10 | 10 | 3 | 10 | 0 | 10 | 10 | 10 | 10 | 10 | 9 | 10 | 10 | 10 | 0 | 10 | 8 | 0 | 10 | 10 | 9 | 10 |
| Chickweed | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 2 | 10 | 2 | 10 | 2 | 10 | 10 | 10 | 10 | 0 | 10 | - | 10 | 10 | 2 | 10 | 5 | 5 | 10 | 2 | 2 | 2 | 3 | 0 | 0 | 10 | 3 | 10 | 10 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 9 | 10 | 8 | 10 | 9 | 10 | 10 | 10 | 10 | 0 | 10 | 10 | 10 | 10 | 10 | 9 | 10 | 10 | 10 | 10 | 10 | 9 | 9 | 0 | 0 | 10 | 10 | 10 | 10 |
| Downy brome | 0 | 10 | 0 | 7 | 0 | 10 | 10 | 10 | - | 0 | 10 | 0 | 10 | 0 | 8 | 0 | 0 | 0 | 5 | 0 | 0 | 0 | 0 | 0 | 0 | 10 | 0 | 10 | 10 |
| Duck salad | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 3 | 0 | 0 | 0 | 0 | 0 | 0 |
| Giant foxtail | 5 | 10 | 8 | 10 | 9 | 10 | 10 | 10 | 10 | 0 | 10 | 10 | 10 | 10 | 10 | 10 | 10 | 10 | 10 | 10 | 10 | 7 | 7 | 2 | 8 | 10 | - | 10 | 10 |
| Lambsquarters | 0 | 0 | 0 | 0 | 0 | 0 | 0 | 4 | 0 | 0 | 0 | 0 | 0 | 0 | 0 | 0 | 0 | 0 | 0 | 0 | 3 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Ryegrass | 0 | 9 | 0 | 10 | 0 | 9 | 10 | 10 | - | 0 | 10 | 0 | 10 | 0 | 8 | 0 | 0 | 0 | 4 | 0 | 0 | 0 | 0 | 0 | 0 | 10 | 0 | 10 | 6 |
| Sorghum | 10 | 10 | 9 | 10 | 10 | 10 | 10 | 10 | 10 | 0 | 10 | 10 | 10 | 10 | 10 | 10 | 10 | 10 | 10 | 10 | 10 | 7 | 9 | 10 | 3 | 10 | 10 | 10 | 10 |
| Soybean | 0 | 0 | 0 | 0 | 0 | 0 | 0 | 2 | 2 | 0 | 0 | 0 | 0 | 0 | 0 | 0 | 0 | 0 | 0 | 0 | 0 | 0 | 0 | 0 | 0 | 0 | 0 | 0 | 0 |
| Speedwell | 0 | 0 | 0 | 0 | 0 | 9 | 0 | 0 | 4 | 0 | 0 | 0 | 5 | 0 | 0 | 0 | 0 | 0 | 2 | 0 | 0 | 0 | 0 | 0 | 0 | 0 | 0 | 0 | 0 |
| Sugar beet | 0 | 0 | 0 | 0 | 0 | 2 | 0 | 0 | 0 | 0 | 0 | 0 | 0 | 0 | 0 | 0 | 0 | 0 | 0 | 0 | 5 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 6 | 10 | 5 | 10 | 9 | 10 | 10 | 10 | 10 | 0 | 10 | 0 | 10 | 10 | 10 | 9 | 7 | 9 | 10 | 10 | 10 | 0 | 10 | 2 | 0 | 10 | 10 | 10 | 10 |
| Wild buckwheat | 0 | 0 | 0 | 0 | 0 | 0 | 0 | 0 | 6 | - | 0 | 0 | 0 | 0 | 0 | 0 | 0 | 0 | 0 | 0 | 6 | 0 | 0 | 0 | 0 | 0 | 0 | 0 | 0 |
| Wild oat | 0 | 10 | 0 | 7 | 0 | 9 | 9 | 10 | 10 | 5 | 10 | 0 | 7 | 3 | 5 | 0 | 0 | 2 | 5 | 0 | 0 | 0 | 0 | 0 | 0 | 5 | 1 | 10 | 8 |
| Barnyardgrass | 10 | 10 | 10 | 10 | 10 | 10 | 10 | 10 | - | 6 | 10 | 10 | 10 | 10 | 10 | 10 | 10 | 10 | 10 | 10 | 10 | 10 | 10 | 9 | 0 | 10 | 9 | 10 | 10 |
| Rice Japonica | 0 | 1 | 3 | 10 | 9 | 9 | 10 | 10 | 10 | 0 | 10 | 0 | 10 | 10 | 10 | 7 | 10 | 10 | 9 | 6 | 3 | 1 | 9 | 1 | 0 | 10 | 9 | 10 | 10 |
| Umbrella sedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

Table B

| | COMPOUND | | | | | | | | | | | | | | | | | | | |
|---|---|---|---|---|---|---|---|---|---|---|---|---|---|---|---|---|---|---|---|---|
| Rate (125 g/ha) | 60 | 64 | 67 | 68 | 69 | 73 | 79 | 85 | 86 | 87 | 88 | 91 | 92 | 94 | 95 | 96 | 98 | 99 | 101 | 103 |
| POSTEMERGENCE | | | | | | | | | | | | | | | | | | | | |
| Barley Igri | 5 | 10 | 10 | 5 | 9 | 0 | 0 | 7 | 1 | 3 | 10 | 10 | 10 | 10 | 6 | 6 | 10 | 9 | 2 | 4 |
| Bedstraw | 0 | 0 | 0 | 0 | 0 | 0 | 0 | 0 | 0 | 0 | 0 | - | 0 | 0 | 0 | 0 | 0 | 0 | 0 | 0 |
| Blackgrass | 9 | 7 | 10 | 10 | 10 | 10 | 0 | 10 | 0 | 10 | 10 | 10 | 10 | 10 | 9 | 10 | 10 | 9 | 3 | 0 |
| Chickweed | 0 | 0 | 0 | 0 | 0 | 0 | 0 | 0 | 0 | 0 | 0 | 0 | 0 | 0 | 0 | 0 | 0 | 0 | 0 | 0 |
| Corn | 10 | 1 | 10 | 10 | 10 | 1 | 9 | 5 | - | 9 | 10 | 10 | 10 | 10 | 10 | 10 | 10 | 10 | 10 | 1 |
| Cotton | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 10 | 10 | 10 | 10 | 10 | 10 | 9 | 10 | 10 | 10 | 10 | 10 | 10 | 10 | 10 | 10 | 10 | 10 | 7 | 9 |
| Downy brome | 0 | 0 | 4 | 0 | 9 | 0 | 2 | 0 | 0 | 0 | 9 | 10 | 10 | 10 | 0 | 8 | 7 | 5 | 4 | 2 |
| Duck salad | 0 | 4 | 0 | 0 | 0 | 0 | 0 | 0 | 0 | 0 | 0 | 0 | 0 | 0 | 0 | 0 | 0 | 0 | 0 | 0 |
| Giant foxtail | 10 | 10 | 10 | 10 | 10 | 10 | 6 | 10 | 10 | 10 | 10 | 10 | 10 | 10 | 10 | 10 | 10 | 10 | 9 | 10 |
| Lambsquarters | 0 | 0 | 0 | 0 | 0 | 0 | 0 | 0 | 0 | 0 | 0 | 0 | 3 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 |
| Ryegrass | 0 | 0 | 0 | 0 | 5 | 0 | 0 | 0 | 0 | 0 | 6 | 9 | 10 | 6 | 0 | 6 | 9 | 3 | 2 | 2 |
| Sorghum | 10 | 10 | 10 | 10 | 10 | 10 | 10 | 10 | 10 | 10 | 10 | 10 | 10 | 10 | 10 | 10 | 10 | 10 | 10 | 10 |
| Soybean | 0 | 0 | 0 | 0 | 0 | 0 | 0 | 0 | 0 | 0 | 0 | 0 | 0 | 0 | 0 | 0 | 0 | 0 | 0 | 0 |
| Speedwell | 0 | 0 | 0 | 0 | 0 | 0 | 0 | 0 | 0 | 0 | 0 | 0 | 4 | 0 | 0 | 0 | 0 | 0 | 0 | 0 |
| Sugar beet | 0 | 0 | 0 | 0 | 0 | 0 | 0 | 0 | 0 | 4 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 |
| Wheat | 6 | 10 | 10 | 10 | 9 | 0 | 2 | 10 | 7 | 10 | 10 | 10 | 10 | 10 | 8 | 8 | 10 | 9 | 1 | 0 |
| Wild buckwheat | 0 | 0 | - | 0 | 0 | 0 | 0 | 0 | 0 | 0 | 0 | 0 | 0 | 0 | 0 | 0 | 0 | 0 | 0 | 0 |
| Wild oat | 0 | 2 | 4 | 0 | 7 | 3 | 2 | 0 | 0 | 0 | 7 | 9 | 9 | 7 | 2 | 0 | 5 | 4 | 0 | 7 |
| Barnyardgrass | 10 | 10 | 10 | 10 | 10 | 10 | 9 | 10 | 10 | 10 | 10 | 10 | 10 | 10 | 9 | 10 | 10 | 10 | 10 | 6 |
| Rice Japonica | 10 | 10 | 10 | 10 | 10 | 4 | 0 | 10 | 6 | 10 | 9 | 9 | 10 | 10 | 9 | 9 | 10 | 10 | 1 | 0 |
| Umbrella sedge | 0 | 2 | 0 | 0 | 0 | 0 | 0 | 0 | 0 | 0 | 0 | 0 | 0 | 0 | 0 | 0 | 0 | 0 | 0 | 0 |

Table B

| Rate (125 g/ha) | COMPOUND 5 | 6 | 8 | 10 | 15 | 19 | 22 | 23 | 24 | 28 | 41 | 42 | 43 | 46 | 47 | 48 | 52 | 53 |
|---|---|---|---|---|---|---|---|---|---|---|---|---|---|---|---|---|---|---|
| PREEMERGENCE | | | | | | | | | | | | | | | | | | |
| Barley Igri | 0 | 0 | 0 | 0 | 0 | 0 | 0 | 0 | 0 | 0 | 0 | 0 | 0 | 0 | 0 | 0 | 0 | 0 |
| Bedstraw | 0 | 0 | 0 | 0 | 0 | 0 | 3 | 0 | 0 | 0 | 0 | 0 | 0 | 0 | 0 | 0 | 0 | 0 |
| Blackgrass | 2 | 3 | 10 | 9 | 10 | 8 | 10 | 8 | 6 | 10 | 6 | 10 | 5 | 9 | 2 | 8 | 5 | 0 |
| Chickweed | 0 | 0 | 0 | 0 | 0 | 0 | 0 | 0 | 0 | 0 | 0 | 0 | 0 | 0 | 0 | 0 | 0 | 0 |
| Corn | 0 | 0 | 0 | 3 | 0 | 0 | 2 | 4 | 4 | 2 | 2 | 3 | 0 | 0 | 0 | 4 | 0 | 0 |
| Cotton | 0 | 0 | 0 | 0 | 0 | 0 | 0 | 0 | 0 | 0 | 0 | 0 | 0 | 0 | 0 | 0 | 0 | 0 |
| Crabgrass | 7 | 9 | 10 | 10 | 10 | 5 | 10 | 6 | 6 | 10 | 10 | 10 | 3 | 10 | 7 | 10 | 3 | 1 |
| Downy brome | 0 | 0 | 0 | 0 | 0 | 0 | 0 | 0 | 0 | 0 | 0 | 0 | 0 | 0 | 0 | 0 | 0 | 0 |
| Duck salad | - | - | - | - | - | - | - | - | - | - | - | - | - | - | - | - | - | - |
| Giant foxtail | 3 | 4 | 9 | 10 | 9 | 9 | 10 | 10 | 10 | 10 | 7 | 3 | 4 | 8 | 9 | 10 | 4 | 2 |
| Lambsquarters | 0 | 3 | 0 | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | - | 0 | 0 | 0 | 0 | 0 |
| Pigweed | 0 | 0 | 0 | 0 | 0 | 0 | 0 | 0 | 3 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 |
| Ryegrass | 0 | 0 | 3 | 0 | 0 | 0 | 0 | 0 | 0 | 0 | 0 | 0 | 0 | 0 | 0 | 0 | 0 | 0 |
| Sorghum | 2 | 0 | 0 | 7 | 0 | 4 | 9 | 3 | 4 | 5 | 4 | 2 | 0 | 2 | 0 | 4 | 2 | 0 |
| Soybean | 0 | 0 | 0 | 0 | 0 | 3 | 0 | 0 | 0 | 0 | 0 | 0 | 0 | 0 | 0 | 0 | 0 | 0 |
| Speedwell | 0 | 0 | 0 | 0 | 0 | 0 | 0 | 0 | - | 2 | 0 | 0 | 0 | 0 | 0 | 0 | - | 0 |
| Sugar beet | 0 | 0 | 0 | 0 | 0 | 2 | 0 | 1 | 0 | 0 | 0 | 0 | 0 | 0 | 0 | 0 | 0 | 0 |
| Velvetleaf | 0 | 0 | 0 | 0 | 0 | 0 | 0 | 0 | 2 | 0 | 0 | 0 | 0 | 0 | 0 | 0 | 0 | 0 |
| Wheat | 0 | 0 | 0 | 3 | 0 | 0 | 0 | 0 | 0 | 0 | 0 | 0 | 0 | 0 | 0 | 0 | 0 | 0 |
| Wild buckwheat | 0 | 0 | 0 | 0 | - | 0 | 0 | 9 | 5 | 0 | 0 | - | - | 0 | 0 | 0 | - | 0 |
| Wild oat | 0 | 0 | 0 | 0 | 0 | 0 | 0 | 0 | 0 | 0 | 0 | 0 | 0 | 2 | 0 | 0 | 0 | 0 |
| Barnyardgrass | - | - | - | - | - | - | - | - | - | - | - | - | - | - | - | - | - | - |
| Rice Japonica | - | - | - | - | - | - | - | - | - | - | - | - | - | - | - | - | - | - |
| Umbrella sedge | - | - | - | - | - | - | - | - | - | - | - | - | - | - | - | - | - | - |

Table B

COMPOUND

| Rate (62 g/ha) | 5 | 6 | 8 | 10 | 11 | 15 | 19 | 22 | 23 | 24 | 26 | 28 | 30 | 31 | 32 | 35 | 36 | 37 | 38 | 39 | 40 | 41 | 42 | 43 | 46 | 47 | 48 | 52 | 53 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley Igri | 8 | 10 | 9 | 10 | 0 | 9 | 10 | 10 | 10 | 10 | 5 | 10 | 10 | 10 | 0 | 10 | 10 | 10 | 10 | 2 | 0 | 8 | 10 | 8 | 9 | 0 | 10 | 6 | 0 |
| Bedstraw | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 |
| Blackgrass | 6 | 9 | 9 | 10 | 9 | 10 | 10 | 10 | 10 | 10 | 3 | 10 | 9 | 9 | 0 | 9 | 8 | 10 | 10 | 9 | 9 | 9 | 10 | 10 | 9 | 0 | 10 | 0 | 0 |
| Chickweed | 0 | 0 | 0 | 0 | - | 0 | 0 | 0 | 0 | 0 | 0 | 0 | - | - | - | 0 | 0 | 0 | 0 | 0 | 0 | 0 | 0 | 0 | 0 | 0 | 0 | 0 | 0 |
| Corn | 2 | 10 | 2 | 10 | 10 | 2 | 8 | 10 | 10 | 10 | 0 | 10 | 10 | 10 | 0 | 10 | 10 | 10 | 2 | 10 | 5 | 5 | 7 | 2 | 2 | 2 | 2 | 0 | 0 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 7 | 10 | 7 | 10 | 10 | 9 | 9 | 10 | 10 | 10 | 0 | 10 | 10 | 10 | 10 | 10 | 10 | 10 | 10 | 5 | 10 | 9 | 10 | 10 | 10 | 9 | 8 | 0 | 0 |
| Downy brome | 0 | 5 | 0 | 6 | 0 | 0 | 10 | 10 | 10 | 10 | 0 | 10 | 8 | 8 | 0 | 10 | 7 | 0 | 8 | 0 | 0 | 0 | 0 | 0 | 0 | 0 | 0 | 0 | 0 |
| Duck salad | 0 | 0 | 0 | 0 | 0 | 0 | 2 | 0 | 0 | 0 | 0 | 0 | 2 | 0 | 0 | 0 | 0 | 0 | 0 | 0 | 0 | 0 | 0 | 0 | 0 | 0 | 0 | 0 | 0 |
| Giant foxtail | 5 | 10 | 8 | 10 | 10 | 6 | 10 | 10 | 10 | 10 | 0 | 10 | 10 | 10 | 10 | 10 | 10 | 10 | 10 | 10 | 7 | 9 | 10 | 10 | 10 | 5 | 7 | 0 | 3 |
| Lambsquarters | 0 | 0 | 0 | 0 | 0 | 0 | 0 | - | 0 | 0 | - | 0 | 0 | 0 | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Ryegrass | 0 | 0 | 0 | 7 | 0 | 0 | 9 | 10 | 8 | 10 | 0 | 10 | 5 | 10 | 0 | 10 | 5 | 0 | 1 | 0 | 0 | 0 | 0 | 0 | 0 | 0 | 0 | 0 | 0 |
| Sorghum | 10 | 10 | 9 | 10 | 10 | 7 | 10 | 10 | 10 | 10 | 0 | 10 | 10 | 10 | 10 | 10 | 10 | 10 | 10 | 10 | 10 | 10 | 10 | 10 | 10 | 7 | 9 | 9 | 0 |
| Soybean | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 |
| Speedwell | 0 | 0 | 0 | 0 | 0 | 0 | 7 | 0 | 0 | 0 | 0 | 0 | - | 4 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 6 | 9 | 5 | 10 | 0 | 9 | 10 | 10 | 10 | 10 | 0 | 10 | 10 | 10 | 0 | 10 | 10 | 10 | 10 | 9 | 7 | 8 | 10 | 8 | 9 | 0 | 10 | 0 | 0 |
| Wild buckwheat | 0 | - | 0 | 0 | 0 | 0 | - | 0 | 0 | - | 0 | 0 | 0 | 0 | 0 | 0 | 0 | 0 | 0 | 0 | 0 | 0 | 0 | 0 | 0 | 0 | 0 | 0 | 0 |
| Wild oat | 0 | 10 | 0 | 6 | 6 | 0 | 6 | 9 | 10 | 10 | - | 10 | 5 | 7 | 0 | 5 | 2 | 2 | 0 | 0 | 0 | 0 | 5 | 0 | 0 | 0 | 0 | 0 | 0 |
| Barnyardgrass | 10 | 10 | 10 | 10 | 10 | 10 | 10 | 10 | 10 | 10 | 2 | 10 | 10 | 10 | 10 | 10 | 10 | 10 | 10 | 7 | 10 | 10 | 10 | 10 | 10 | 10 | 10 | 8 | 0 |
| Rice Japonica | 0 | 0 | 0 | 10 | 2 | 5 | 3 | 9 | 9 | 10 | 0 | 10 | 10 | 10 | 0 | 10 | 10 | 9 | 10 | 5 | 10 | 8 | 9 | 5 | 1 | 0 | 9 | 0 | 0 |
| Umbrella sedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

Table B

| | COMPOUND | | | | | | | | | | | | | | | | | |
|---|---|---|---|---|---|---|---|---|---|---|---|---|---|---|---|---|---|---|
| Rate (62 g/ha) | 55 | 56 | 58 | 59 | 60 | 62 | 63 | 64 | 67 | 68 | 70 | 71 | 72 | 73 | 76 | 78 | 113 | 114 |
| POSTEMERGENCE | | | | | | | | | | | | | | | | | | |
| Barley Igri | 10 | 10 | 10 | 10 | 5 | - | 10 | 10 | 10 | 3 | 10 | 7 | 10 | 0 | 10 | 0 | 6 | 10 |
| Bedstraw | 0 | 0 | 0 | 0 | 0 | 0 | 0 | 0 | 0 | 0 | 0 | 0 | 0 | 0 | 0 | 0 | 0 | 0 |
| Blackgrass | 10 | 8 | 9 | 10 | 9 | 10 | 10 | 7 | 9 | 9 | 10 | 10 | 10 | 10 | 10 | 0 | 5 | 9 |
| Chickweed | 0 | 0 | 0 | 0 | 0 | 0 | 0 | 0 | 0 | 0 | 0 | 0 | 0 | 0 | 0 | 0 | 0 | 0 |
| Corn | 10 | 0 | 10 | 10 | 10 | 10 | 10 | 1 | 10 | 10 | 3 | 4 | 10 | 1 | 10 | 6 | 10 | 10 |
| Cotton | 0 | 0 | 0 | 0 | 0 | 0 | 0 | 0 | 0 | 0 | 0 | 0 | 0 | 0 | 0 | 0 | 0 | 0 |
| Crabgrass | 10 | 10 | 10 | 10 | 10 | 10 | 10 | 10 | 10 | 8 | 9 | 10 | 8 | 7 | 10 | 5 | 8 | 10 |
| Downy brome | 10 | 0 | 10 | 5 | 0 | 0 | 7 | 0 | 0 | 0 | 0 | 0 | 7 | 0 | 4 | 0 | 0 | 10 |
| Duck salad | 0 | 0 | 0 | 0 | 0 | 0 | 0 | 0 | 0 | 0 | 0 | 0 | 0 | 0 | 2 | 0 | 0 | 1 |
| Giant foxtail | 10 | 10 | 10 | 10 | 10 | 10 | 10 | 10 | 10 | 10 | 10 | 10 | 10 | 5 | 10 | 10 | 9 | 10 |
| Lambsquarters | 0 | 0 | 0 | 0 | - | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 |
| Ryegrass | 10 | 0 | 10 | 3 | 0 | 5 | 6 | 0 | 0 | 0 | 4 | 0 | 1 | 0 | 4 | 0 | 0 | 9 |
| Sorghum | 10 | 10 | 10 | 10 | 10 | 10 | 10 | 10 | 10 | 10 | 10 | 10 | 10 | 10 | 10 | 10 | 9 | 10 |
| Soybean | 0 | 0 | 0 | 0 | 0 | 0 | 0 | 0 | 0 | 0 | 0 | 0 | 0 | 0 | 0 | 0 | 0 | 0 |
| Speedwell | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | - | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 |
| Wheat | 10 | 10 | 10 | 10 | 1 | 10 | 10 | 10 | 10 | 10 | 10 | 10 | 10 | 0 | 10 | 0 | 6 | 9 |
| Wild buckwheat | 0 | 0 | 0 | 0 | 0 | 0 | 0 | 0 | 0 | 0 | - | 0 | 0 | 0 | 0 | 0 | 0 | 0 |
| Wild oat | - | 0 | 10 | 7 | 0 | 10 | 7 | 0 | 3 | 0 | 0 | 0 | 4 | 0 | 2 | 3 | 0 | 9 |
| Barnyardgrass | 10 | 8 | 10 | 10 | 10 | 10 | 10 | 10 | 10 | 9 | 10 | 10 | 10 | 10 | 10 | 10 | 3 | 10 |
| Rice Japonica | 10 | 7 | 10 | 9 | 10 | 10 | 10 | 9 | 10 | 9 | 10 | 10 | 10 | 0 | 9 | 0 | 3 | 10 |
| Umbrella sedge | 0 | 0 | 0 | 0 | 0 | 0 | 3 | 0 | 0 | 0 | 0 | 0 | 0 | 0 | 0 | 0 | 0 | 0 |

Table B

| | COMPOUND | | | | | | | | | | | | | | | | | |
|---|---|---|---|---|---|---|---|---|---|---|---|---|---|---|---|---|---|---|
| Rate (62 g/ha) | 5 | 6 | 8 | 10 | 15 | 19 | 22 | 23 | 24 | 28 | 41 | 42 | 43 | 46 | 47 | 48 | 52 | 53 |
| PREEMERGENCE | | | | | | | | | | | | | | | | | | |
| Barley Igri | 0 | 0 | 0 | 0 | 0 | 0 | 0 | 0 | 0 | 0 | 0 | 0 | 0 | 0 | 0 | 0 | 0 | 0 |
| Bedstraw | 0 | 0 | 0 | 0 | 0 | 0 | 0 | 0 | 0 | 0 | 0 | 0 | 0 | 0 | 0 | 0 | 0 | 0 |
| Blackgrass | 0 | 0 | 7 | 8 | 10 | 4 | 10 | 7 | 5 | 6 | 2 | 3 | 4 | 7 | 0 | 4 | 0 | 0 |
| Chickweed | 0 | 0 | 0 | 0 | 0 | 0 | 0 | 0 | 0 | 0 | 0 | 0 | 0 | 0 | 0 | 0 | 0 | 0 |
| Corn | 0 | 0 | 0 | 0 | 0 | 0 | 0 | 2 | 0 | 0 | 0 | 1 | 0 | 0 | 0 | 3 | 0 | 0 |
| Cotton | 0 | 0 | 0 | 0 | 0 | 0 | 0 | 0 | 0 | 0 | 0 | 0 | 0 | 0 | 0 | 0 | 0 | 0 |
| Crabgrass | 2 | 3 | 7 | 10 | 8 | 3 | 5 | 3 | 6 | 10 | 3 | 4 | 2 | 4 | 2 | 4 | 2 | 0 |
| Downy brome | 0 | 0 | 0 | 0 | 0 | 0 | 0 | 0 | 0 | 0 | 0 | 0 | 0 | 0 | 0 | 0 | 0 | 0 |
| Duck salad | - | - | - | - | - | - | - | - | - | - | - | - | - | - | - | - | - | - |
| Giant foxtail | 0 | 2 | 5 | 10 | 9 | 5 | 5 | 6 | 9 | 10 | 3 | 2 | 0 | 4 | 3 | 10 | 2 | 0 |
| Lambsquarters | 0 | 0 | - | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 |
| Ryegrass | 0 | 0 | 0 | 0 | 0 | 0 | 0 | 0 | 0 | 0 | 0 | 0 | 0 | 0 | 0 | 0 | 0 | 0 |
| Sorghum | 0 | 0 | 0 | 3 | 0 | 2 | 4 | 0 | 2 | 4 | 2 | 0 | 0 | 0 | 0 | 2 | 0 | 0 |
| Soybean | 0 | 0 | 0 | 0 | 0 | 2 | 0 | 0 | 0 | 0 | 0 | 0 | 0 | 0 | 0 | 0 | 0 | 0 |
| Speedwell | - | - | 0 | 0 | 0 | 0 | 0 | 0 | 0 | 0 | 0 | 0 | - | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 |
| Wheat | 0 | 0 | 0 | 0 | 0 | 0 | 0 | 0 | 0 | 0 | 0 | 0 | 0 | 0 | 0 | 0 | 0 | 0 |
| Wild buckwheat | 0 | 0 | - | 0 | 0 | 0 | 0 | 7 | 4 | 0 | 0 | - | 0 | 0 | 0 | 0 | 0 | 0 |
| Wild oat | 0 | 0 | 0 | 0 | 0 | 0 | 0 | 0 | 0 | 0 | 0 | 0 | 0 | 0 | 0 | 0 | 0 | 0 |
| Barnyardgrass | - | - | - | - | - | - | - | - | - | - | - | - | - | - | - | - | - | - |
| Rice Japonica | - | - | - | - | - | - | - | - | - | - | - | - | - | - | - | - | - | - |
| Umbrella sedge | - | - | - | - | - | - | - | - | - | - | - | - | - | - | - | - | - | - |

Table B

| Rate (31 g/ha) | COMPOUND 5 | 6 | 8 | 10 | 11 | 15 | 19 | 22 | 23 | 24 | 26 | 28 | 30 | 31 | 32 | 35 | 36 | 37 | 38 | 39 | 40 | 41 | 42 | 43 | 46 | 47 | 48 | 52 | 53 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley Igri | 5 | 10 | 5 | 7 | 0 | 7 | 10 | 10 | 10 | 10 | 0 | 10 | 10 | 10 | 0 | 10 | 8 | 10 | 10 | 1 | 0 | 3 | 10 | 2 | 8 | 0 | 9 | 2 | 0 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 6 | 4 | 8 | 10 | 0 | 10 | 10 | 10 | 10 | 10 | 0 | 9 | 6 | 9 | 0 | 9 | - | 8 | 6 | 3 | 9 | 4 | 10 | 9 | 9 | 0 | 2 | 0 | 0 |
| Chickweed | 0 | 0 | 0 | 0 | 0 | 0 | 0 | 0 | 0 | 0 | 0 | 0 | - | - | 0 | - | 0 | 0 | 0 | 0 | 0 | 0 | 0 | 0 | 0 | 0 | 0 | 0 | 0 |
| Corn | 2 | 10 | 2 | 10 | 10 | 2 | 8 | 10 | 10 | 10 | 0 | 10 | 10 | 10 | 0 | 10 | 10 | 10 | 0 | 9 | 2 | 5 | 6 | 2 | 1 | 1 | 1 | 0 | 0 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 5 | 10 | 7 | 10 | 10 | 8 | 8 | 10 | 8 | 9 | 0 | 10 | 10 | 10 | 3 | 10 | 8 | 10 | 10 | 5 | 9 | 9 | 10 | 9 | 10 | 8 | 8 | 0 | 0 |
| Downy brome | 0 | 3 | 0 | 3 | 0 | 0 | 5 | 5 | 6 | 5 | 0 | 10 | 8 | 8 | 0 | 7 | 0 | 0 | 1 | 0 | 0 | 0 | 0 | 0 | 0 | 0 | 0 | 0 | 0 |
| Duck salad | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Giant foxtail | 5 | 10 | 8 | 10 | 10 | 6 | 10 | 10 | 10 | 10 | 0 | 10 | 10 | 10 | 8 | 10 | 10 | 10 | 10 | 10 | 7 | 9 | 10 | 9 | 8 | 4 | 6 | 0 | 0 |
| Lambsquarters | 0 | 0 | - | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Ryegrass | 0 | 0 | 0 | 3 | 0 | 0 | 5 | 6 | 8 | 6 | 0 | 9 | 5 | 7 | 0 | 8 | 0 | 0 | 1 | 0 | 0 | 0 | 0 | 0 | 0 | 0 | 0 | 0 | 0 |
| Sorghum | 10 | 10 | 9 | 10 | 10 | 7 | 10 | 10 | 10 | 10 | 0 | 10 | 10 | 10 | 10 | 10 | 10 | 10 | 10 | 10 | 10 | 10 | 10 | 10 | 10 | 7 | 9 | 9 | 0 |
| Soybean | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 |
| Speedwell | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 5 | 9 | 5 | 10 | 0 | 8 | 9 | 10 | 10 | 10 | 0 | 10 | 10 | 10 | 0 | 10 | 10 | 10 | 10 | 9 | 1 | 4 | 10 | 5 | 9 | 0 | 10 | 0 | 0 |
| Wild buckwheat | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | - | - | 0 | 0 | 0 |
| Wild oat | 0 | 9 | 0 | 6 | 0 | 0 | 3 | 8 | 10 | 10 | 3 | 10 | 5 | 4 | 0 | 5 | 0 | 2 | 0 | 0 | 0 | 0 | 0 | 0 | 0 | 0 | 0 | 0 | 0 |
| Barnyardgrass | 10 | 10 | 10 | 10 | 10 | 10 | 10 | 10 | 10 | 10 | 0 | 10 | 10 | 10 | 10 | 10 | 10 | 10 | 9 | 2 | 6 | 10 | 10 | 10 | 10 | 10 | 10 | 7 | 0 |
| Rice Japonica | 0 | 0 | 0 | 10 | 0 | 4 | 0 | 5 | 3 | 8 | 0 | 10 | 10 | 9 | 0 | 10 | 9 | 9 | 9 | 2 | 10 | 5 | 9 | 4 | 1 | 0 | 9 | 0 | 0 |
| Umbrella sedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

Table B

| | COMPOUND | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| Rate (31 g/ha) | 55 | 56 | 58 | 59 | 60 | 62 | 63 | 64 | 67 | 68 | 69 | 70 | 71 | 72 | 73 | 76 | 79 | 85 | 86 | 87 | 88 | 91 | 92 | 94 | 95 | 96 | 98 | 99 | 101 |
| POSTEMERGENCE | | | | | | | | | | | | | | | | | | | | | | | | | | | | | |
| Barley Igri | 10 | 9 | 10 | 7 | 5 | 10 | 10 | 7 | 7 | 0 | 8 | - | 6 | 10 | 0 | 9 | 0 | 3 | 1 | 3 | 10 | 10 | 10 | 4 | 1 | 5 | 3 | 8 | 0 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 10 | 8 | 8 | 9 | 8 | 9 | 10 | 3 | 8 | 9 | 10 | 9 | 7 | 9 | 3 | 10 | 0 | 10 | 0 | 10 | 9 | 7 | 9 | 9 | 6 | 9 | 10 | 8 | 0 |
| Chickweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 10 | 0 | 10 | 10 | 10 | 10 | 10 | - | 10 | 10 | 10 | 3 | 4 | 10 | 1 | 10 | 4 | 1 | - | 6 | 10 | 9 | 10 | 10 | 10 | 10 | 10 | 10 | 10 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 10 | 10 | 10 | 10 | 8 | 10 | 10 | 10 | 10 | 5 | 10 | 9 | 10 | 7 | 7 | 10 | 7 | 9 | 10 | 10 | 10 | 10 | 10 | 8 | 10 | 10 | 10 | 10 | 6 |
| Downy brome | 10 | 0 | 10 | 0 | 0 | 0 | 2 | 0 | 0 | 0 | 9 | 0 | 0 | 4 | 0 | 0 | 0 | 0 | 0 | 0 | 7 | 6 | 9 | 2 | 0 | 2 | 2 | 2 | 2 |
| Duck salad | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 |
| Giant foxtail | 10 | 5 | 10 | 10 | 10 | 10 | 10 | 10 | 10 | 10 | 10 | 10 | 10 | 10 | 4 | 10 | 5 | 10 | 9 | 10 | 10 | 10 | 10 | 10 | 8 | 10 | 10 | 10 | 9 |
| Lambsquarters | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Ryegrass | 10 | 0 | 10 | 0 | 0 | 5 | - | 0 | 0 | 0 | 0 | 0 | 0 | 0 | 0 | 0 | 0 | 0 | 0 | 0 | 2 | 8 | 5 | 0 | 0 | 3 | 2 | 1 | 0 |
| Sorghum | 10 | 10 | 10 | 10 | 10 | 10 | 10 | 4 | 10 | 10 | 10 | 10 | 10 | 10 | 10 | 10 | 10 | 10 | 10 | 10 | 10 | 10 | 10 | 10 | 10 | 10 | 10 | 10 | 10 |
| Soybean | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Speedwell | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 10 | 10 | 10 | 10 | 0 | 10 | 10 | 10 | 10 | 10 | 9 | 10 | 10 | 10 | 0 | 10 | 0 | 10 | 0 | 9 | 6 | 10 | 10 | 6 | 0 | 6 | 6 | 8 | 0 |
| Wild buckwheat | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 |
| Wild oat | 5 | 0 | 10 | 0 | 0 | 9 | 5 | 0 | 0 | 0 | 0 | 0 | 0 | 4 | 0 | 0 | 0 | 0 | 0 | 0 | 7 | 6 | 4 | 5 | 0 | 0 | 2 | 2 | 0 |
| Barnyardgrass | 10 | 8 | 10 | 6 | 10 | 10 | 10 | 10 | 10 | 5 | 10 | 10 | 10 | 10 | 10 | 10 | 0 | 10 | 10 | 10 | 10 | 10 | 10 | 9 | 9 | 10 | 10 | 10 | 10 |
| Rice Japonica | 10 | 6 | 9 | 4 | 3 | 10 | 9 | 6 | 10 | 8 | 10 | 10 | 9 | 10 | 0 | 6 | 0 | 10 | 6 | 10 | 9 | 9 | 10 | 8 | 4 | 9 | 10 | 10 | 0 |
| Umbrella sedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

| Table B | COMPOUND |
|---|---|
| Rate (31 g/ha) | 103 |
| POSTEMERGENCE | |
| Barley Igri | 3 |
| Bedstraw | 0 |
| Blackgrass | 0 |
| Chickweed | 0 |
| Corn | - |
| Cotton | 0 |
| Crabgrass | 5 |
| Downy brome | 0 |
| Duck salad | 0 |
| Giant foxtail | 7 |
| Lambsquarters | 0 |
| Morningglory | 0 |
| Pigweed | 0 |
| Rape | 0 |
| Ryegrass | 0 |
| Sorghum | 10 |
| Soybean | 0 |
| Speedwell | 0 |
| Sugar beet | 0 |
| Velvetleaf | 0 |
| Wheat | 0 |
| Wild buckwheat | 0 |
| Wild oat | 3 |
| Barnyardgrass | 4 |
| Rice Japonica | 0 |
| Umbrella sedge | 0 |

| Table B | COMPOUND | | | | | | | | | | | | | | | | | |
|---|---|---|---|---|---|---|---|---|---|---|---|---|---|---|---|---|---|---|
| Rate (31 g/ha) | 5 | 6 | 8 | 10 | 15 | 19 | 22 | 23 | 24 | 28 | 41 | 42 | 43 | 46 | 47 | 48 | 52 | 53 |
| PREEMERGENCE | | | | | | | | | | | | | | | | | | |
| Barley Igri | 0 | 0 | 0 | 0 | 0 | 0 | 0 | 0 | 0 | 0 | 0 | 0 | 0 | 0 | 0 | 0 | 0 | 0 |
| Bedstraw | 0 | 0 | 0 | - | 0 | 0 | 0 | 0 | 0 | 0 | 0 | 0 | 0 | 0 | 0 | 0 | 0 | 0 |
| Blackgrass | 0 | - | 0 | 0 | 5 | 0 | 3 | 7 | 5 | 6 | 0 | 0 | 4 | 6 | 0 | 0 | 0 | 0 |
| Chickweed | 0 | 0 | 0 | 0 | 0 | 0 | 0 | 0 | 0 | 0 | 0 | 0 | 0 | 0 | 0 | 0 | 0 | 0 |
| Corn | 0 | 0 | 0 | 0 | 0 | 0 | 0 | 0 | 0 | 0 | 0 | 0 | 0 | 0 | 0 | 2 | 0 | 0 |
| Cotton | 0 | 0 | 0 | 0 | 0 | 0 | 0 | 0 | 0 | 0 | 0 | 0 | 0 | 0 | 0 | 0 | 0 | 0 |
| Crabgrass | 0 | 0 | 4 | 7 | 3 | 0 | 2 | 2 | 2 | 4 | 0 | 0 | 0 | 2 | 0 | 2 | 0 | 0 |
| Downy brome | 0 | 0 | 0 | 0 | 0 | 0 | 0 | 0 | 0 | 0 | 0 | 0 | 0 | 0 | 0 | 0 | 0 | 0 |
| Duck salad | - | - | - | - | - | - | - | - | - | - | - | - | - | - | - | - | - | - |
| Giant foxtail | 0 | 0 | 2 | 4 | 3 | 3 | 2 | 4 | 4 | 3 | 0 | 0 | 0 | 2 | 0 | 2 | 0 | 0 |
| Lambsquarters | 0 | - | 0 | 0 | 0 | 0 | 0 | 0 | 0 | 0 | 0 | 0 | - | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 |
| Ryegrass | 0 | 0 | 0 | 0 | 0 | 0 | 0 | 0 | 0 | 0 | 0 | 0 | 0 | 0 | 0 | 0 | 0 | 0 |
| Sorghum | 0 | 0 | 0 | 0 | 0 | 0 | 2 | 0 | 0 | 0 | 0 | 0 | 0 | 0 | 0 | 0 | 0 | 0 |
| Soybean | 0 | 0 | 0 | 0 | 0 | 0 | 0 | 0 | 0 | 0 | 0 | 0 | 0 | 0 | 0 | 0 | 0 | 0 |
| Speedwell | - | 0 | 0 | 0 | - | 0 | 0 | 0 | - | 0 | 0 | 0 | 0 | 0 | 0 | 0 | 0 | 0 |
| Sugar beet | 0 | 0 | 0 | 0 | - | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 |
| Wheat | 0 | 0 | 0 | 0 | 0 | 0 | 0 | 0 | 0 | 0 | 0 | 0 | 0 | 0 | 0 | 0 | 0 | 0 |
| Wild buckwheat | 0 | 0 | 0 | 0 | - | 0 | - | - | - | 0 | 0 | - | 0 | 0 | 0 | 0 | 0 | 0 |
| Wild oat | 0 | 0 | 0 | 0 | 0 | 0 | 0 | 0 | 0 | 0 | 0 | 0 | 0 | 0 | 0 | 0 | 0 | 0 |
| Barnyardgrass | - | - | - | - | - | - | - | - | - | - | - | - | - | - | - | - | - | - |
| Rice Japonica | - | - | - | - | - | - | - | - | - | - | - | - | - | - | - | - | - | - |
| Umbrella sedge | - | - | - | - | - | - | - | - | - | - | - | - | - | - | - | - | - | - |

Table B

| Rate (16 g/ha) POSTEMERGENCE | 6 | 10 | 11 | 19 | 22 | 23 | 24 | 28 | 30 | 31 | 35 | 38 | 39 | 40 | 41 | 42 | 43 | 46 | 47 | 55 | 58 | 59 | 60 | 62 | 63 | 64 | 67 | 68 | 70 |
|---|---|---|---|---|---|---|---|---|---|---|---|---|---|---|---|---|---|---|---|---|---|---|---|---|---|---|---|---|---|
| Barley Igri | 7 | 7 | 0 | 6 | 10 | 10 | 9 | 10 | 9 | 10 | 5 | 9 | 0 | 0 | 0 | 9 | 0 | 5 | 0 | 10 | 10 | 2 | 1 | 10 | 8 | 6 | 6 | 0 | - |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 0 | 10 | 0 | 8 | 10 | 10 | 10 | 9 | 3 | 7 | 9 | 6 | 0 | 6 | 4 | 10 | 7 | 6 | 0 | 10 | - | 2 | - | 9 | 10 | 0 | - | 0 | 8 |
| Chickweed | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 | 0 | 0 | 0 |
| Corn | 10 | 10 | 4 | 2 | 10 | 10 | 10 | 10 | 10 | 10 | 10 | 0 | 7 | - | 3 | 2 | 1 | 1 | 1 | 10 | 10 | 10 | 10 | 10 | 10 | 0 | 10 | 4 | 2 |
| Cotton | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 |
| Crabgrass | 8 | 10 | 10 | 8 | 10 | 8 | 8 | 9 | 9 | 8 | 8 | 8 | 5 | 9 | 9 | 10 | 9 | 10 | 8 | 10 | 10 | 6 | 8 | 10 | 10 | 10 | 10 | 0 | 9 |
| Downy brome | 0 | 2 | 0 | 0 | 5 | 0 | 3 | 7 | 8 | 8 | 4 | 0 | 0 | 0 | 0 | 0 | 0 | 0 | 0 | 7 | 10 | 0 | 0 | 0 | 2 | 0 | 0 | 0 | 0 |
| Duck salad | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Giant foxtail | 8 | 10 | 9 | 8 | 10 | 10 | 10 | 10 | 10 | 10 | 10 | 10 | 7 | 7 | 6 | 10 | 8 | 8 | 4 | 10 | 10 | 10 | 10 | 10 | 10 | 9 | 10 | 4 | 10 |
| Lambsquarters | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Ryegrass | 0 | 0 | 0 | 0 | 5 | 8 | 4 | 4 | 3 | 2 | 4 | 0 | 0 | 0 | 0 | 0 | 0 | 0 | 0 | 7 | 6 | 0 | 0 | 4 | 3 | 0 | 0 | 0 | 0 |
| Sorghum | 10 | 10 | 10 | 10 | 10 | 10 | 10 | 10 | 10 | 10 | 10 | 10 | 10 | 10 | 9 | 10 | 10 | 10 | 5 | 10 | 10 | 10 | 10 | 10 | 10 | 4 | 10 | 10 | 10 |
| Soybean | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Speedwell | 0 | 0 | 0 | 0 | 0 | 0 | 0 | 0 | 0 | 0 | 0 | 0 | 0 | 0 | 0 | - | - | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - |
| Wheat | 4 | 10 | 0 | 8 | 10 | 9 | 10 | 10 | 10 | 10 | 10 | 9 | 2 | 0 | 3 | - | 5 | - | 0 | 10 | 10 | 5 | 0 | 10 | 10 | 6 | 10 | 9 | 10 |
| Wild buckwheat | 0 | 0 | 0 | - | 0 | 0 | 0 | 0 | 0 | 0 | - | 0 | 0 | 0 | 0 | 0 | - | 0 | 0 | 0 | 0 | 0 | 0 | 0 | 0 | 0 | 0 | 0 | 0 |
| Wild oat | 8 | 6 | 0 | 0 | 7 | 9 | 9 | 10 | 0 | 3 | 0 | 0 | 0 | 0 | 0 | 0 | 0 | 0 | 0 | - | 9 | 0 | 0 | 9 | 4 | 0 | 0 | 0 | 0 |
| Barnyardgrass | 8 | 10 | 10 | 6 | 10 | 10 | 10 | 10 | 10 | 10 | 10 | 9 | 0 | 0 | 10 | 10 | 10 | 10 | 10 | 10 | 10 | 0 | 8 | 10 | 10 | 10 | 10 | 5 | 10 |
| Rice Japonica | 0 | 10 | 0 | 0 | 0 | 2 | 0 | 7 | 9 | 9 | 9 | 0 | 0 | 3 | 1 | 9 | 3 | 0 | 0 | 9 | 9 | 3 | 0 | 9 | 9 | 5 | 9 | 4 | 10 |
| Umbrella sedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

(Column headings: COMPOUND)

| Table B | COMPOUND | | | | | | |
|---|---|---|---|---|---|---|---|
| Rate (16 g/ha) | 71 | 72 | 73 | 76 | 78 | 113 | 114 |
| POSTEMERGENCE | | | | | | | |
| Barley Igri | 6 | 10 | 0 | 8 | 0 | 5 | 9 |
| Bedstraw | 0 | 0 | 0 | 0 | 0 | 0 | 0 |
| Blackgrass | 7 | 8 | 0 | 9 | 0 | 3 | 8 |
| Chickweed | 0 | 0 | 0 | 0 | 0 | 0 | 0 |
| Corn | 3 | 10 | 0 | 10 | 2 | 10 | 10 |
| Cotton | 0 | 0 | 0 | 0 | 0 | 0 | 0 |
| Crabgrass | 10 | 4 | 7 | 10 | 2 | 8 | 10 |
| Downy brome | 0 | 3 | 0 | 0 | 0 | 0 | 9 |
| Duck salad | 0 | 0 | 0 | 0 | 0 | 0 | 0 |
| Giant foxtail | 10 | 10 | 4 | 10 | 2 | 7 | 10 |
| Lambsquarters | 0 | 0 | 0 | 0 | 0 | 0 | 0 |
| Morningglory | 0 | 0 | 0 | 0 | 0 | 0 | 0 |
| Pigweed | 0 | 0 | 0 | 0 | 0 | 0 | 0 |
| Rape | 0 | 0 | 0 | 0 | 0 | 0 | 0 |
| Ryegrass | 0 | 0 | 0 | 0 | 0 | 0 | 8 |
| Sorghum | 10 | 10 | 10 | 10 | 10 | 4 | 10 |
| Soybean | 0 | 0 | 0 | 0 | 0 | 0 | 0 |
| Speedwell | 0 | 0 | 0 | 0 | 0 | 0 | 0 |
| Sugar beet | 0 | 0 | 0 | 0 | 0 | 0 | 0 |
| Velvetleaf | 0 | 0 | 0 | 0 | 0 | 0 | 0 |
| Wheat | 10 | 10 | 0 | 5 | 0 | 3 | 9 |
| Wild buckwheat | 0 | 0 | 0 | 0 | 0 | 0 | 0 |
| Wild oat | 0 | 4 | 0 | 0 | 0 | 0 | 8 |
| Barnyardgrass | 10 | 10 | 10 | 7 | 3 | 0 | 10 |
| Rice Japonica | 6 | 10 | 0 | 2 | 0 | 2 | 9 |
| Umbrella sedge | 0 | 0 | 0 | 0 | 0 | 0 | 0 |

| Table B | COMPOUND | | | | | | | | | | | |
|---|---|---|---|---|---|---|---|---|---|---|---|---|
| Rate (16 g/ha) | 6 | 10 | 19 | 22 | 23 | 24 | 28 | 41 | 42 | 43 | 46 | 47 |
| PREEMERGENCE | | | | | | | | | | | | |
| Barley Igri | 0 | 0 | 0 | 0 | 0 | 0 | 0 | 0 | 0 | 0 | 0 | 0 |
| Bedstraw | 0 | 0 | 0 | 0 | 0 | 0 | 0 | 0 | 0 | 0 | 0 | 0 |
| Blackgrass | 0 | 0 | - | 3 | 0 | 2 | 4 | 0 | 0 | 2 | 0 | 0 |
| Chickweed | 0 | 0 | 0 | 0 | 0 | 0 | 0 | 0 | 0 | 0 | 0 | 0 |
| Corn | 0 | 0 | 0 | 0 | 0 | 0 | 0 | 0 | 0 | 0 | 0 | 0 |
| Cotton | 0 | 0 | 0 | 0 | 0 | 0 | 0 | 0 | 0 | 0 | 0 | 0 |
| Crabgrass | 0 | 5 | 0 | 0 | 0 | 0 | 4 | 0 | 0 | 0 | 0 | 0 |
| Downy brome | 0 | 0 | 0 | 0 | 0 | 0 | 0 | 0 | 0 | 0 | 0 | 0 |
| Duck salad | - | - | - | - | - | - | - | - | - | - | - | - |
| Giant foxtail | 0 | 0 | 0 | 0 | 3 | 2 | 2 | 0 | 0 | 0 | 0 | 0 |
| Lambsquarters | 0 | 0 | 0 | 0 | 0 | - | 0 | 0 | 0 | 0 | 0 | - |
| Morningglory | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 |
| Ryegrass | 0 | 0 | 0 | 0 | 0 | 0 | 0 | 0 | 0 | 0 | 0 | 0 |
| Sorghum | 0 | 0 | 0 | 0 | 0 | 0 | 0 | 0 | 0 | 0 | 0 | 0 |
| Soybean | 0 | 0 | 0 | 0 | 0 | 0 | 0 | 0 | 0 | 0 | 0 | 0 |
| Speedwell | - | 0 | 0 | 0 | - | 0 | 0 | 0 | 0 | 0 | 0 | 0 |
| Sugar beet | 0 | 0 | 0 | - | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 |
| Wheat | 0 | 0 | 0 | 0 | 0 | 0 | 0 | 0 | 0 | 0 | 0 | 0 |
| Wild buckwheat | 0 | 0 | - | 0 | 7 | - | 0 | 0 | 0 | - | 0 | 0 |
| Wild oat | 0 | 0 | 0 | 0 | 0 | 0 | 0 | 0 | 0 | 0 | 0 | 0 |
| Barnyardgrass | - | - | - | - | - | - | - | - | - | - | - | - |
| Rice Japonica | - | - | - | - | - | - | - | - | - | - | - | - |
| Umbrella sedge | - | - | - | - | - | - | - | - | - | - | - | - |

Table B

COMPOUND

| Rate (8 g/ha) | 11 | 19 | 22 | 23 | 24 | 28 | 30 | 31 | 35 | 41 | 42 | 43 | 46 | 47 | 62 | 63 | 69 | 70 | 71 | 72 | 76 | 79 | 85 | 86 | 87 | 88 | 91 | 92 | 94 |
|---|---|---|---|---|---|---|---|---|---|---|---|---|---|---|---|---|---|---|---|---|---|---|---|---|---|---|---|---|---|
| POSTEMERGENCE | | | | | | | | | | | | | | | | | | | | | | | | | | | | | |
| Barley Igri | 0 | 0 | 10 | 7 | 8 | 9 | 2 | 7 | 0 | 0 | 9 | 0 | 2 | 0 | 10 | 5 | 4 | 9 | 5 | 6 | 2 | 0 | 0 | 0 | 1 | 4 | 3 | 4 | 0 |
| Bedstraw | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Blackgrass | 0 | 8 | 10 | 9 | 10 | 9 | 3 | 5 | 0 | 4 | 9 | 3 | 2 | 0 | 9 | 10 | 9 | 8 | 6 | 2 | 5 | 0 | 4 | 0 | 0 | 9 | 7 | 9 | 5 |
| Chickweed | 0 | 0 | 0 | 0 | 0 | 0 | - | - | 0 | 0 | 0 | 0 | 0 | 0 | 0 | 0 | 0 | 0 | 0 | 0 | 0 | 0 | 0 | 0 | 0 | 0 | 0 | 0 | 0 |
| Corn | 3 | 2 | 10 | 10 | 10 | 10 | 10 | 10 | 2 | 2 | 1 | 1 | 1 | 1 | 10 | 10 | 6 | 1 | 2 | 10 | 10 | 1 | - | 0 | 2 | 9 | 2 | 9 | 4 |
| Cotton | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Crabgrass | 5 | 7 | 7 | 7 | 6 | 9 | 5 | 8 | 3 | 9 | 10 | 8 | 9 | 6 | 10 | 10 | 10 | 5 | 10 | 4 | 4 | 4 | 8 | 3 | 7 | 9 | 9 | 10 | 5 |
| Downy brome | 0 | 0 | 0 | 0 | 0 | 2 | 4 | 1 | 0 | 0 | 0 | 0 | 0 | 0 | 0 | 0 | 4 | 0 | 0 | 3 | 0 | 0 | 0 | 0 | 0 | 0 | 4 | 0 | 0 |
| Duck salad | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Giant foxtail | 4 | 8 | 10 | 10 | 10 | 10 | 10 | 10 | 4 | 6 | 9 | 8 | 6 | 4 | 10 | 10 | 8 | 5 | 10 | 10 | 5 | 4 | 9 | 8 | 7 | 9 | 9 | 10 | 7 |
| Lambsquarters | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Ryegrass | 0 | 0 | 5 | 0 | 3 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 |
| Sorghum | 6 | 10 | 10 | 10 | 10 | 10 | 10 | 10 | 4 | 9 | 10 | 10 | 6 | 5 | 10 | 10 | 10 | 10 | 10 | 10 | 4 | 6 | 10 | 10 | 10 | 10 | 10 | 10 | 10 |
| Soybean | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Speedwell | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | - | - | 0 | - | 0 | 0 | 0 | 0 | 0 | 0 | 0 | 0 | - | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Wheat | 0 | 8 | 10 | 4 | 9 | 10 | 10 | 10 | 2 | 0 | 9 | 4 | 9 | 0 | 10 | 4 | 3 | - | 10 | 10 | 0 | 0 | 3 | 0 | 9 | 0 | 0 | 4 | 0 |
| Wild buckwheat | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 | 0 | 0 |
| Wild oat | 0 | 0 | 5 | 0 | 6 | 4 | 0 | 0 | 0 | 0 | 0 | 0 | 0 | 0 | 7 | 0 | 0 | 0 | 0 | 0 | 0 | 0 | 0 | 0 | 0 | 4 | 0 | 0 | 0 |
| Barnyardgrass | 10 | 4 | 10 | 10 | 10 | 10 | 10 | 10 | 10 | 10 | 10 | 10 | 10 | 10 | 10 | 10 | 6 | 10 | 10 | 10 | 3 | 0 | 10 | 10 | 10 | 10 | 10 | 8 | 4 |
| Rice Japonica | 0 | 0 | 0 | 0 | 0 | 0 | 3 | 3 | 0 | 0 | 0 | 1 | 0 | 0 | 3 | 0 | 0 | 10 | 4 | 7 | 2 | 0 | 6 | 6 | 10 | 2 | 2 | 0 | 0 |
| Umbrella sedge | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

| Table B | COMPOUND | | | | | |
|---|---|---|---|---|---|---|
| Rate (8 g/ha) | 95 | 96 | 98 | 99 | 101 | 103 |
| POSTEMERGENCE | | | | | | |
| Barley Igri | 0 | 2 | 0 | 4 | 0 | 0 |
| Bedstraw | 0 | 0 | 0 | 0 | 0 | 0 |
| Blackgrass | 0 | 8 | 9 | 8 | 0 | 0 |
| Chickweed | 0 | 0 | 0 | 0 | 0 | 0 |
| Corn | 2 | 0 | 9 | 10 | 2 | 0 |
| Cotton | 0 | 0 | 0 | 0 | 0 | 0 |
| Crabgrass | 5 | 5 | 10 | 9 | 2 | 0 |
| Downy brome | 0 | 0 | 0 | 0 | 0 | 0 |
| Duck salad | 0 | 0 | 0 | 0 | 0 | 0 |
| Giant foxtail | 6 | 5 | 7 | 10 | 3 | 2 |
| Lambsquarters | 0 | 0 | 0 | 0 | 0 | 0 |
| Morningglory | 0 | 0 | 0 | 0 | 0 | 0 |
| Pigweed | 0 | 0 | 0 | 0 | 0 | 0 |
| Rape | 0 | 0 | 0 | 0 | 0 | 0 |
| Ryegrass | 0 | 2 | 0 | 0 | 0 | 0 |
| Sorghum | 10 | 10 | 10 | 10 | 6 | 4 |
| Soybean | 0 | 0 | 0 | 0 | 0 | 0 |
| Speedwell | 0 | 0 | 0 | 0 | 0 | 0 |
| Sugar beet | 0 | 0 | 0 | 0 | 0 | 0 |
| Velvetleaf | 0 | 0 | 0 | 0 | 0 | 0 |
| Wheat | 0 | 0 | 2 | 6 | 0 | 0 |
| Wild buckwheat | 0 | 0 | 0 | 0 | 0 | 0 |
| Wild oat | 0 | 0 | 0 | 0 | 0 | 0 |
| Barnyardgrass | 5 | 5 | 10 | 10 | 10 | 0 |
| Rice Japonica | 0 | 0 | 6 | 9 | 0 | 0 |
| Umbrella sedge | 0 | 0 | 0 | 0 | 0 | 0 |

| Table B | COMPOUND | | | | | | | | | |
|---|---|---|---|---|---|---|---|---|---|---|
| Rate (8 g/ha) | 19 | 22 | 23 | 24 | 28 | 41 | 42 | 43 | 46 | 47 |
| PREEMERGENCE | | | | | | | | | | |
| Barley Igri | 0 | 0 | 0 | 0 | 0 | 0 | 0 | 0 | 0 | 0 |
| Bedstraw | 0 | 0 | 0 | 0 | 0 | 0 | 0 | 0 | 0 | 0 |
| Blackgrass | 0 | 3 | 0 | 0 | 0 | 0 | 0 | 0 | 0 | 0 |
| Chickweed | 0 | 0 | 0 | 0 | 0 | 0 | 0 | 0 | 0 | 0 |
| Corn | 0 | 0 | 0 | 0 | 0 | 0 | 0 | 0 | 0 | 0 |
| Cotton | 0 | 0 | 0 | 0 | 0 | 0 | 0 | 0 | 0 | 0 |
| Crabgrass | 0 | 0 | 0 | 0 | 4 | 0 | 0 | 0 | 0 | 0 |
| Downy brome | 0 | 0 | 0 | 0 | 0 | 0 | 0 | 0 | 0 | 0 |
| Duck salad | - | - | - | - | - | - | - | - | - | - |
| Giant foxtail | 0 | 0 | 0 | 0 | 0 | 0 | 0 | 0 | 0 | 0 |
| Lambsquarters | 0 | 0 | 0 | 0 | 0 | 0 | 0 | 0 | 0 | 0 |
| Morningglory | 0 | 0 | 0 | 0 | 0 | 0 | 0 | 0 | 0 | 0 |
| Pigweed | 0 | 0 | 0 | 0 | 0 | 0 | 0 | 0 | 0 | 0 |
| Rape | 0 | 0 | 0 | 0 | 0 | 0 | 0 | 0 | 0 | 0 |
| Ryegrass | 0 | 0 | 0 | 0 | 0 | 0 | 0 | 0 | 0 | 0 |
| Sorghum | 0 | 0 | 0 | 0 | 0 | 0 | 0 | 0 | 0 | 0 |
| Soybean | 0 | 0 | 0 | 0 | 0 | 0 | 0 | 0 | 0 | 0 |
| Speedwell | 0 | 0 | - | 0 | 0 | 0 | 0 | 0 | 0 | 0 |
| Sugar beet | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 |
| Wheat | 0 | 0 | 0 | 0 | 0 | 0 | 0 | 0 | 0 | 0 |
| Wild buckwheat | 0 | - | 7 | 0 | 0 | 0 | 0 | - | - | 0 |
| Wild oat | 0 | 0 | 0 | 0 | 0 | 0 | 0 | 0 | 0 | 0 |
| Barnyardgrass | - | - | - | - | - | - | - | - | - | - |
| Rice Japonica | - | - | - | - | - | - | - | - | - | - |
| Umbrella sedge | - | - | - | - | - | - | - | - | - | - |

| Table B | COMPOUND | | | |
|---|---|---|---|---|
| Rate (4 g/ha) | 63 | 78 | 113 | 114 |
| POSTEMERGENCE | | | | |
| Barley Igri | 0 | 0 | 4 | 5 |
| Bedstraw | 0 | 0 | 0 | 0 |
| Blackgrass | 2 | 0 | 2 | 8 |
| Chickweed | 0 | 0 | 0 | 0 |
| Corn | 3 | 0 | 4 | 10 |
| Cotton | 0 | 0 | 0 | 0 |
| Crabgrass | 8 | 0 | 6 | 9 |
| Downy brome | 0 | 0 | 0 | 3 |
| Duck salad | 0 | 0 | 0 | 0 |
| Giant foxtail | 8 | 0 | 5 | 10 |
| Lambsquarters | 0 | 0 | 0 | 0 |
| Morningglory | 0 | 0 | 0 | 0 |
| Pigweed | 0 | 0 | 0 | 0 |
| Rape | 0 | 0 | 0 | 0 |
| Ryegrass | 0 | 0 | 0 | 3 |
| Sorghum | 10 | 2 | 3 | 10 |
| Soybean | 0 | 0 | 0 | 0 |
| Speedwell | 0 | 0 | 0 | 0 |
| Sugar beet | 0 | 0 | 0 | 0 |
| Velvetleaf | 0 | 0 | 0 | 0 |
| Wheat | 4 | 0 | 0 | 7 |
| Wild buckwheat | 0 | 0 | 0 | 0 |
| Wild oat | 0 | 0 | 0 | 3 |
| Barnyardgrass | 9 | 0 | 0 | 10 |
| Rice Japonica | 2 | 0 | 2 | 0 |
| Umbrella sedge | 0 | 0 | 0 | 0 |

| Table B | COMPOUND | | | | | | | | | | | | | |
|---|---|---|---|---|---|---|---|---|---|---|---|---|---|---|
| Rate (2 g/ha) | 69 | 79 | 85 | 86 | 87 | 88 | 91 | 92 | 94 | 95 | 96 | 98 | 99 | 101 |
| POSTEMERGENCE | | | | | | | | | | | | | | |
| Barley Igri | 0 | 0 | 0 | 0 | 0 | 0 | 0 | 0 | 0 | 0 | 0 | 0 | 0 | 0 |
| Bedstraw | 0 | 0 | 0 | 0 | 0 | 0 | 0 | 0 | 0 | 0 | 0 | 0 | 0 | 0 |
| Blackgrass | 5 | 0 | 0 | 0 | 0 | 0 | 0 | 0 | 0 | 0 | 2 | 0 | 3 | 0 |
| Chickweed | 0 | 0 | 0 | 0 | 0 | 0 | 0 | 0 | 0 | 0 | 0 | 0 | 0 | 0 |
| Corn | 3 | 1 | 0 | 0 | 0 | 4 | 1 | 3 | 0 | 2 | 0 | 4 | 2 | 1 |
| Cotton | 0 | 0 | 0 | 0 | 0 | 0 | 0 | 0 | 0 | 0 | 0 | 0 | 0 | 0 |
| Crabgrass | 3 | 2 | 3 | 0 | 4 | 3 | 2 | 3 | 1 | 0 | 0 | 3 | 7 | 0 |
| Downy brome | 0 | 0 | 0 | 0 | 0 | 0 | 0 | 0 | 0 | 0 | 0 | 0 | 0 | 0 |
| Duck salad | 0 | 0 | 0 | 0 | 0 | 0 | 0 | 0 | 0 | 0 | 0 | 0 | 0 | 0 |
| Giant foxtail | 4 | 2 | 5 | 2 | 3 | 4 | 4 | 4 | 0 | 2 | 0 | 2 | 5 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 |
| Rape | 0 | 0 | 0 | 0 | 0 | 0 | 0 | 0 | 0 | 0 | 0 | 0 | 0 | 0 |
| Ryegrass | 0 | 0 | 0 | 0 | 0 | 0 | 0 | 0 | 0 | 0 | 0 | 0 | 0 | 0 |
| Sorghum | 3 | 2 | 3 | 2 | 10 | 2 | 9 | 3 | 2 | 4 | 3 | 4 | 9 | 3 |
| Soybean | 0 | 0 | 0 | 0 | 0 | 0 | 0 | 0 | 0 | 0 | 0 | 0 | 0 | 0 |
| Speedwell | 0 | 0 | 0 | 0 | 0 | 0 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 |
| Wheat | 0 | 0 | 0 | 0 | 2 | 0 | 0 | 2 | 0 | 0 | 0 | 0 | 4 | 0 |
| Wild buckwheat | 0 | 0 | 0 | 0 | 0 | 0 | 0 | 0 | 0 | 0 | 0 | 0 | 0 | 0 |
| Wild oat | 0 | 0 | 0 | 0 | 0 | 0 | 0 | 0 | 0 | 0 | 0 | 0 | 0 | 0 |
| Barnyardgrass | 2 | 0 | 1 | 5 | 10 | 8 | 10 | 2 | 0 | 0 | 0 | 3 | 1 | 0 |
| Rice Japonica | 0 | 0 | 2 | 0 | 9 | 0 | 0 | 0 | 0 | 0 | 0 | 1 | 3 | 0 |
| Umbrella sedge | 0 | 0 | 0 | 0 | 0 | 0 | 0 | 0 | 0 | 0 | 0 | 0 | 0 | 0 |

| Table B | COMPOUND | |
|---|---|---|
| Rate (1 g/ha) | 63 | 114 |
| POSTEMERGENCE | | |
| Barley Igri | 0 | 0 |
| Bedstraw | 0 | 0 |
| Blackgrass | 0 | 2 |
| Chickweed | 0 | 0 |
| Corn | 0 | 5 |
| Cotton | 0 | 0 |
| Crabgrass | 2 | 2 |
| Downy brome | 0 | 0 |
| Duck salad | 0 | 0 |
| Giant foxtail | 2 | 4 |
| Lambsquarters | 0 | 0 |
| Morningglory | 0 | 0 |
| Pigweed | 0 | 0 |
| Rape | 0 | 0 |
| Ryegrass | 0 | 0 |
| Sorghum | 3 | 5 |
| Soybean | 0 | 0 |
| Speedwell | 0 | 0 |
| Sugar beet | 0 | 0 |
| Velvetleaf | 0 | 0 |
| Wheat | 0 | 2 |
| Wild buckwheat | 0 | 0 |
| Wild oat | 0 | 0 |
| Barnyardgrass | 2 | 9 |
| Rice Japonica | 0 | 0 |
| Umbrella sedge | 0 | 0 |

TEST C

Seeds, tubers, or plant parts of alfalfa (Medicago sativa), annual bluegrass (Poa annua), bermudagrass (Cynodon dactylon), broadleaf signalgrass (Brachiaria plantaginea), dallisgrass (Paspalum Dilatatum), goosegrass (Eleusine indica), guineagrass (Panicum maximum), itchgrass (Rottboellia exaltata), johnsongrass (Sorghum halepense), large crabgrass (Digitaria sanguinalis), KY bluegrass (Poa pratensis), peanuts (Arachis hypogaea), P. J. legume (Pueraria javanica), pitted morningglory (Ipomoea lacunosa), purple nutsedge (Cyperus rotundus), purslane (Portulaca oleracea), ragweed (Ambrosia elatior), sandbur (Cenchrus echinatus), smooth crabgrass (Digitaria ischaemum), TX panicum (Panicum Texas), and yellow nutsedge (Cyperus esculentus) were planted into greenhouse pots of flats containing greenhouse planting medium. Plant species were grown grown in separate pots or individual compartments. Preemergence applications were made within one day of planting the seed or plant part. Postemergence applications were applied

when the plants were in the two to four leaf stage (three to twenty cm). Test chemicals were dissolved in a non-phytotoxic solvent and applied preemergence and postemergence to the plants. Untreated control plants and treated plants were placed in the greenhouse and visually evaluated for injury 13 to 21 days after herbicide application. Plant response ratings, summarized in Table C, are based on a 0 to 10 scale where 0 is no injury and 10 is complete control. A dash (-) response means no test result.

Table C

| | COMPOUND | | | | | | | | | | | |
|---|---|---|---|---|---|---|---|---|---|---|---|---|
| Rate (250 g/ha) | 1 | 2 | 3 | 4 | 5 | 6 | 7 | 8 | 9 | 10 | 14 | 15 |
| POSTEMERGENCE | | | | | | | | | | | | |
| Alfalfa | 0 | 0 | 0 | 0 | 0 | 0 | 0 | 0 | 0 | 0 | 0 | 0 |
| Ann. Bluegrass | 0 | 0 | 0 | 0 | 0 | 0 | 0 | 0 | 0 | 0 | 0 | 0 |
| Bermudagrass | 5 | 10 | 10 | 10 | 4 | 10 | 10 | 9 | 9 | 10 | 10 | 10 |
| Brdlf sgnlgrass | 0 | 10 | 1 | 10 | 2 | 10 | 10 | 2 | 2 | 10 | 10 | 2 |
| Dallis Grass | 0 | 9 | 9 | 4 | 0 | 9 | 5 | 0 | 0 | 0 | 2 | 0 |
| Goosegrass | 0 | 10 | 9 | 10 | 5 | 10 | 10 | 9 | 0 | 10 | 10 | 0 |
| Guineagrass | 0 | 10 | 10 | 10 | 7 | 10 | 10 | 10 | 3 | 10 | 10 | 7 |
| Itch Grass | 0 | 10 | 9 | 10 | 10 | 10 | 10 | 10 | 8 | 10 | 10 | 10 |
| Johnsongrass | 0 | 10 | 10 | 10 | 10 | 10 | 10 | 10 | 6 | 10 | 10 | 10 |
| Large crabgrass | 0 | 10 | 9 | 10 | 4 | 10 | 7 | 8 | 3 | 8 | 10 | 5 |
| P.J.Legume | 8 | 0 | 0 | 0 | 3 | 5 | 0 | 0 | 0 | 0 | 0 | 3 |
| Peanuts | 0 | 0 | 0 | 0 | 0 | 0 | 0 | 0 | 0 | 0 | 0 | 0 |
| Pitted mrnglory | 0 | 0 | 0 | 0 | 0 | 0 | 0 | 0 | 0 | 0 | 0 | 0 |
| Purple nutsedge | 0 | 0 | 0 | 0 | 0 | 0 | 0 | 0 | 0 | 0 | 0 | 0 |
| Purslane | 0 | 0 | 0 | 0 | 10 | 10 | 0 | 0 | 0 | 0 | 0 | 0 |
| Ragweed | 0 | 0 | 0 | 0 | 0 | 10 | 0 | 0 | 0 | 0 | 0 | 0 |
| Sandbur | 10 | 9 | 10 | 9 | 5 | 10 | 10 | 10 | 9 | 10 | 10 | 10 |
| Smooth crbgrass | 0 | 10 | 0 | 7 | 0 | 8 | 3 | 7 | 0 | 0 | 2 | 7 |
| TX panicum | 0 | 10 | 9 | 10 | 0 | 10 | 10 | 5 | 0 | 7 | 10 | 4 |
| Yellow nutsedge | 0 | 0 | 0 | 0 | 0 | 0 | 0 | 0 | 0 | 0 | 0 | 0 |

| Table C | COMPOUND | | | | | | | | | | | |
|---|---|---|---|---|---|---|---|---|---|---|---|---|
| Rate (250 g/ha) | 1 | 2 | 3 | 4 | 5 | 6 | 7 | 8 | 9 | 10 | 14 | 15 |
| PREEMERGENCE | | | | | | | | | | | | |
| Alfalfa | 0 | 0 | 0 | 0 | 0 | 0 | 0 | 0 | 0 | 0 | 0 | 0 |
| Ann. Bluegrass | 0 | 0 | 0 | 0 | 0 | 0 | 0 | 0 | 0 | 0 | 0 | 0 |
| Bermudagrass | 7 | 10 | 10 | 9 | 9 | 0 | 10 | 10 | 9 | 10 | 10 | 0 |
| Brdlf sgnlgrass | 0 | 9 | 0 | 7 | 0 | 0 | 10 | 8 | 0 | 9 | 10 | 0 |
| Dallis Grass | 0 | 0 | 0 | 0 | 0 | 0 | 0 | 0 | 0 | 0 | 0 | 0 |
| Goosegrass | 0 | 9 | 9 | 10 | 10 | 9 | 9 | 9 | 8 | 9 | 10 | 0 |
| Guineagrass | 0 | 9 | 0 | 7 | 0 | 0 | 9 | 6 | 0 | 9 | 2 | 0 |
| Itch Grass | 0 | 3 | 0 | 0 | 0 | 0 | 0 | 0 | 0 | 0 | 0 | 0 |
| Johnsongrass | 0 | 0 | 0 | 0 | 0 | 0 | 0 | 0 | 0 | 0 | 0 | 0 |
| Large crabgrass | 0 | 0 | 0 | 6 | 0 | 0 | 0 | 3 | 0 | 3 | 0 | 0 |
| P.J.Legume | 0 | 0 | 0 | 0 | 0 | 0 | 0 | 0 | 0 | 0 | 0 | 0 |
| Peanuts | 0 | 0 | 0 | 0 | 0 | 0 | 0 | 0 | 0 | 0 | 1 | 0 |
| Pitted mrnglory | 2 | 0 | 1 | 0 | 0 | 0 | 0 | 0 | 0 | 0 | 0 | 0 |
| Purple nutsedge | 0 | 0 | 0 | 0 | 0 | 0 | 0 | 0 | 0 | 0 | 0 | 0 |
| Purslane | 0 | 0 | 0 | 0 | 0 | 0 | 0 | 0 | 0 | 0 | 0 | 0 |
| Ragweed | 0 | 0 | 0 | 2 | 0 | 0 | 0 | 0 | 0 | 0 | 0 | 0 |
| Sandbur | 2 | 0 | 0 | 0 | 2 | 0 | 0 | 0 | 0 | 0 | 0 | 8 |
| Smooth crbgrass | 0 | 10 | 0 | 3 | 0 | 0 | 2 | 9 | 0 | 8 | 0 | 0 |
| TX panicum | 0 | 3 | 0 | 0 | 0 | 0 | 2 | 0 | 0 | 7 | 0 | 0 |
| Yellow nutsedge | 0 | 0 | 0 | 0 | 0 | 0 | 0 | 0 | 0 | 0 | 0 | 0 |

Table C

| Rate (125 g/ha) | COMPOUND 1 | 2 | 3 | 4 | 5 | 6 | 7 | 8 | 9 | 10 | 14 | 15 |
|---|---|---|---|---|---|---|---|---|---|---|---|---|
| POSTEMERGENCE | | | | | | | | | | | | |
| Alfalfa | 0 | 0 | 0 | 0 | 0 | 0 | 2 | 0 | 0 | 0 | 0 | 0 |
| Ann. Bluegrass | 0 | 0 | 0 | 0 | 0 | 0 | 0 | 0 | 0 | 0 | 0 | 0 |
| Bermudagrass | 0 | 10 | 9 | 10 | 7 | 10 | 10 | 0 | 3 | 8 | 10 | 10 |
| Brdlf sgnlgrass | 0 | 10 | 5 | 10 | 0 | 10 | 10 | 9 | 0 | 10 | 10 | 0 |
| Dallis Grass | 0 | 7 | 1 | 2 | 0 | 3 | 4 | 0 | 0 | 3 | 2 | 0 |
| Goosegrass | 0 | 9 | 10 | 9 | 2 | 9 | 10 | 5 | 0 | 9 | 10 | 0 |
| Guineagrass | 0 | 10 | 9 | 9 | 4 | 10 | 9 | 10 | 9 | 9 | 10 | 4 |
| Itch Grass | 0 | 10 | 9 | 10 | 10 | 10 | 10 | 10 | 10 | 10 | 10 | 7 |
| Johnsongrass | 0 | 10 | 10 | 10 | 10 | 10 | 10 | 10 | 10 | 10 | 10 | 10 |
| Large crabgrass | 0 | 10 | 7 | 9 | 2 | 9 | 10 | 0 | 3 | 6 | 9 | 5 |
| P.J.Legume | 5 | 0 | 0 | 0 | 0 | 0 | 0 | 0 | 2 | 5 | 0 | 0 |
| Peanuts | 0 | 0 | 0 | 0 | 0 | 0 | 0 | 0 | 0 | 0 | 0 | 0 |
| Pitted mrnglory | 0 | 0 | 0 | 0 | 0 | 0 | 0 | 0 | 0 | 0 | 0 | 0 |
| Purple nutsedge | 0 | 0 | 0 | 0 | 0 | 0 | 0 | 0 | 0 | 0 | 0 | 0 |
| Purslane | 0 | 0 | 0 | 0 | 0 | 10 | 10 | 10 | 0 | 0 | 0 | 0 |
| Ragweed | 0 | 0 | 0 | 0 | 0 | 10 | 0 | 0 | 0 | 0 | 0 | 0 |
| Sandbur | 0 | 9 | 9 | 10 | 5 | 10 | 10 | 5 | 9 | 10 | 10 | 10 |
| Smooth crbgrass | 0 | 2 | 1 | 6 | 0 | 5 | 10 | 0 | 0 | 2 | 5 | 0 |
| TX panicum | 0 | 8 | 7 | 10 | 0 | 0 | 10 | 2 | 0 | 9 | 10 | 0 |
| Yellow nutsedge | 0 | 0 | 0 | 0 | 0 | 0 | 0 | 0 | 0 | 0 | 0 | 0 |

| Table C | COMPOUND | | | | | | | | | | | |
|---|---|---|---|---|---|---|---|---|---|---|---|---|
| Rate (125 g/ha) | 1 | 2 | 3 | 4 | 5 | 6 | 7 | 8 | 9 | 10 | 14 | 15 |
| PREEMERGENCE | | | | | | | | | | | | |
| Alfalfa | 0 | 0 | 0 | 0 | 0 | 0 | 0 | 0 | 0 | 0 | 0 | 0 |
| Ann. Bluegrass | 0 | 0 | 0 | 0 | 0 | 0 | 0 | 0 | 0 | 0 | 0 | 0 |
| Bermudagrass | 7 | 9 | 0 | 9 | 9 | 0 | 8 | 9 | 0 | 9 | 9 | 8 |
| Brdlf sgnlgrass | 0 | 8 | 0 | 0 | 0 | 0 | 2 | 2 | 0 | 7 | 7 | 0 |
| Dallis Grass | 0 | 0 | 0 | 0 | 0 | 0 | 0 | 0 | 0 | 0 | 0 | 0 |
| Goosegrass | 0 | 8 | 0 | 9 | 0 | 0 | 9 | 2 | 0 | 9 | 10 | 0 |
| Guineagrass | 0 | 0 | 0 | 0 | 0 | 0 | 0 | 0 | 0 | 0 | 1 | 0 |
| Itch Grass | 0 | 0 | 0 | 0 | 0 | 0 | 0 | 0 | 0 | 0 | 0 | 0 |
| Johnsongrass | 0 | 0 | 0 | 0 | 0 | 0 | 0 | 0 | 0 | 0 | 0 | 0 |
| Large crabgrass | 0 | 0 | 0 | 0 | 0 | 0 | 0 | 0 | 0 | 0 | 0 | 0 |
| P.J.Legume | 0 | 0 | 0 | 0 | 0 | 0 | 0 | 0 | 0 | 0 | 0 | 0 |
| Peanuts | 0 | 0 | 0 | 0 | 0 | 0 | 0 | 0 | 0 | 0 | 0 | 0 |
| Pitted mrnglory | 0 | 0 | 0 | 0 | 0 | 0 | 0 | 0 | 0 | 2 | 0 | 0 |
| Purple nutsedge | 0 | 0 | 0 | 0 | 0 | 0 | 0 | 0 | 0 | 0 | 0 | 0 |
| Purslane | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 |
| Sandbur | 0 | 0 | 0 | 0 | 0 | 0 | 0 | 0 | 0 | 0 | 5 | 0 |
| Smooth crbgrass | 0 | 0 | 0 | 0 | 0 | 0 | 0 | 9 | 0 | 0 | 0 | 0 |
| TX panicum | 0 | 0 | 0 | 0 | 0 | 0 | 0 | 0 | 0 | 0 | 0 | 0 |
| Yellow nutsedge | 0 | 0 | 0 | 0 | 0 | 0 | 0 | 0 | 0 | 0 | 0 | 0 |

| Table C | COMPOUND |
|---|---|
| Rate (64 g/ha) | 10 |
| POSTEMERGENCE | |
| Alfalfa | 0 |
| Ann. Bluegrass | 0 |
| Bermudagrass | 9 |
| Brdlf sgnlgrass | 0 |
| Dallis Grass | 0 |
| Goosegrass | 0 |
| Guineagrass | 2 |
| Itch Grass | 9 |
| Johnsongrass | 5 |
| KY. Bluegrass | 0 |
| Large crabgrass | 0 |
| Peanuts | 0 |
| Pitted mrnglory | 0 |
| Purple nutsedge | 0 |
| Purslane | 0 |
| Ragweed | 0 |
| Smooth crbgrass | 0 |
| TX panicum | 2 |
| Yellow nutsedge | 0 |

| Table C | COMPOUND |
|---|---|
| Rate (32 g/ha) | 10 |
| POSTEMERGENCE | |
| Alfalfa | 0 |
| Ann. Bluegrass | 0 |
| Bermudagrass | 9 |
| Brdlf sgnlgrass | 1 |
| Dallis Grass | 0 |
| Goosegrass | 0 |
| Guineagrass | 2 |
| Itch Grass | 8 |
| Johnsongrass | 5 |
| KY. Bluegrass | 0 |
| Large crabgrass | 0 |
| Peanuts | 0 |
| Pitted mrnglory | 0 |
| Purple nutsedge | 0 |
| Purslane | 0 |
| Ragweed | 0 |
| Smooth crbgrass | 0 |
| TX panicum | 2 |
| Yellow nutsedge | 0 |

## TEST D

Plastic pots were partially filled with silt loam soil. The soil was then flooded with water, Japonica rice (Oryza sativa) sprouted seeds and 1.5 leaf transplants were planted in the soil. Seeds of barnyardgrass (Echinochloa crus-galli) were planted in saturated soil and plants grown to the 1 leaf, 2 leaf and 3 leaf stages for testing. At testing, the water level for all plantings was raised to 2 cm above the soil surface. Chemical treatments were formulated in a non-phytotoxic solvent and applied directly to the paddy water. Treated plants and controls were maintained in a greenhouse for approximately 21 days, after which all species were compared to controls and visually evaluated. Plant response ratings, summarized in Table D are reported on a 0 to 10 scale where 0 is no effect and 10 is complete control. A dash (-) response means no test result.

| Table D | COMPOUND |
|---|---|
| Rate (1000 g/ha) | 10 |
| PADDY | |
| 1-LF B.Y.Grass | 10 |
| 2-LF B.Y.Grass | 10 |
| 3-lf B.Y.Grass | 10 |
| Jap Direct Seed | 10 |
| Jap Rice Eff | 10 |

| Table D | COMPOUND | |
|---|---|---|
| Rate (500 g/ha) | 10 | 104 |
| PADDY | | |
| 1-LF B.Y.Grass | 10 | - |
| 2-LF B.Y.Grass | 10 | 0 |
| 3-lf B.Y.Grass | 10 | 0 |
| Jap Direct Seed | 10 | 0 |
| Jap Rice Eff | 10 | 0 |

| Rate (250 g/ha) | 5 | 6 | 10 | 11 | 15 | 19 | 30 | 31 | 32 | 43 | 46 | 47 | 49 | 60 | 73 | 78 | 104 |
|---|---|---|---|---|---|---|---|---|---|---|---|---|---|---|---|---|---|
| PADDY | | | | | | | | | | | | | | | | | |
| 1-LF B.Y.Grass | - | - | 10 | - | - | - | - | - | - | - | - | - | - | - | - | - | - |
| 2-LF B.Y.Grass | 10 | 10 | 10 | 10 | 10 | 10 | 10 | 10 | 5 | 10 | 10 | 4 | 10 | 9 | 10 | 5 | 0 |
| 3-lf B.Y.Grass | 10 | 10 | 10 | 10 | 10 | 10 | 10 | 10 | 5 | 10 | 10 | 8 | 10 | 10 | 9 | 0 | 0 |
| Jap Direct Seed | 4 | 10 | 10 | 0 | 8 | 10 | 10 | 10 | 2 | 8 | 6 | 4 | 10 | 10 | 4 | 0 | 0 |
| Jap Rice Eff | 3 | 0 | 10 | 4 | 9 | 10 | 10 | 10 | 0 | 6 | 4 | 1 | 10 | 9 | 2 | 0 | 0 |

Table D

COMPOUND

| Rate (125 g/ha) | 5 | 6 | 8 | 10 | 11 | 15 | 19 | 25 | 30 | 31 | 32 | 35 | 43 | 46 | 47 | 49 | 55 | 60 | 63 | 73 | 78 | 101 | 104 |
|---|---|---|---|---|---|---|---|---|---|---|---|---|---|---|---|---|---|---|---|---|---|---|---|
| PADDY | | | | | | | | | | | | | | | | | | | | | | | |
| 1-LF B.Y.Grass | - | - | 10 | 10 | - | - | - | - | - | - | - | - | - | - | - | - | - | - | - | - | - | - | - |
| 2-LF B.Y.Grass | 10 | 8 | 10 | 10 | 10 | 10 | 10 | 10 | 10 | 10 | 2 | 10 | 10 | 10 | 7 | 10 | 10 | 7 | 10 | 10 | 0 | 10 | 0 |
| 3-lf B.Y.Grass | 8 | 10 | 0 | 10 | 10 | 10 | 10 | 10 | 10 | 10 | 0 | 10 | 10 | 10 | 8 | 10 | 10 | 7 | 10 | 6 | 0 | 10 | 0 |
| Jap Direct Seed | 3 | 3 | 3 | 10 | 0 | 8 | 10 | 10 | 10 | 10 | 1 | 10 | 9 | 5 | 4 | 10 | 10 | 10 | 10 | 5 | 0 | 3 | 0 |
| Jap Rice Eff | 0 | 0 | 0 | 10 | 1 | 8 | 10 | 9 | 9 | 9 | 0 | 9 | 3 | 3 | 0 | 10 | 10 | 10 | 10 | 3 | 0 | 4 | 0 |

| Rate (64 g/ha) | 2 | 3 | 4 | 5 | 6 | 7 | 8 | 10 | 11 | 14 | 15 | 19 | 22 | 23 | 24 | 25 | 28 | 30 | 31 | 32 | 35 | 41 | 42 | 43 | 46 | 47 | 49 | 55 | 60 |
|---|---|---|---|---|---|---|---|---|---|---|---|---|---|---|---|---|---|---|---|---|---|---|---|---|---|---|---|---|---|
| PADDY | | | | | | | | | | | | | | | | | | | | | | | | | | | | | |
| 1-LF B.Y.Grass | - | - | - | - | - | - | 10 | 10 | - | - | - | - | - | - | - | - | - | - | - | - | - | - | - | - | - | - | - | - | - |
| 2-LF B.Y.Grass | 10 | 10 | 10 | 10 | 9 | 10 | 8 | 10 | 10 | 10 | 10 | 10 | 10 | 10 | 10 | 10 | 10 | 10 | 10 | 0 | 10 | 10 | 10 | 10 | 10 | 7 | 10 | 10 | 6 |
| 3-lf B.Y.Grass | 10 | 10 | 10 | 8 | 2 | 10 | 0 | 10 | 5 | 10 | 10 | 10 | 10 | 10 | 10 | 10 | 10 | 10 | 10 | 0 | 10 | 10 | 10 | 10 | 10 | 5 | 10 | 10 | 0 |
| Jap Direct Seed | 10 | 10 | 10 | 3 | 2 | 10 | 4 | 10 | 0 | 10 | 5 | 9 | 10 | 10 | 8 | 8 | 9 | 10 | 10 | 0 | 10 | 5 | 6 | 6 | 5 | 3 | 10 | 10 | 9 |
| Jap Rice Eff | 9 | 9 | 10 | 0 | 0 | 9 | 0 | 9 | 1 | 10 | 5 | 10 | 5 | 10 | 10 | 8 | 9 | 8 | 8 | 0 | 8 | 4 | 9 | 0 | 4 | 0 | 9 | 9 | 9 |

| Table D | COMPOUND | | | | | | | | |
|---|---|---|---|---|---|---|---|---|---|
| Rate (64 g/ha) | 63 | 64 | 70 | 71 | 72 | 73 | 78 | 101 | 104 |
| PADDY | | | | | | | | | |
| 1-LF B.Y.Grass | - | - | - | - | - | - | - | - | - |
| 2-LF B.Y.Grass | 10 | 10 | 10 | 10 | 10 | 10 | 0 | 10 | 0 |
| 3-lf B.Y.Grass | 10 | 10 | 10 | 10 | 10 | 7 | 0 | 10 | 0 |
| Jap Direct Seed | 9 | 9 | 10 | 6 | 9 | 4 | 0 | 2 | 0 |
| Jap Rice Eff | 9 | 6 | 9 | 9 | 10 | 1 | 0 | 1 | 0 |

Table D

| | COMPOUND | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| Rate (32 g/ha) | 2 | 3 | 4 | 5 | 6 | 7 | 8 | 10 | 11 | 14 | 15 | 19 | 22 | 23 | 24 | 25 | 28 | 30 | 31 | 32 | 35 | 41 | 42 | 43 | 46 | 47 | 49 | 55 | 60 |
| PADDY | | | | | | | | | | | | | | | | | | | | | | | | | | | | | |
| 1-LF B.Y.Grass | - | - | - | - | - | - | 10 | 10 | - | - | - | - | - | - | - | - | - | - | - | - | - | - | - | - | - | - | - | - | - |
| 2-LF B.Y.Grass | 10 | 10 | 10 | 10 | 4 | 10 | 9 | 10 | 8 | 10 | 10 | 9 | 9 | 10 | 10 | 10 | 10 | 10 | 10 | 0 | 10 | 10 | 10 | 10 | 10 | 3 | 10 | 10 | 3 |
| 3-1f B.Y.Grass | 10 | 10 | 10 | 10 | 2 | 10 | 0 | 10 | 5 | 10 | 10 | 5 | 2 | 10 | 9 | 10 | 10 | 10 | 10 | 0 | 10 | 10 | 10 | 10 | 10 | 4 | 10 | 8 | 0 |
| Jap Direct Seed | 10 | 5 | 7 | 2 | 0 | 5 | 2 | 10 | 0 | 10 | 4 | 8 | 3 | 6 | 6 | 3 | 4 | 10 | 5 | 0 | 8 | 4 | 4 | 5 | 4 | 3 | 10 | 9 | 7 |
| Jap Rice Eff | 4 | 4 | 9 | 0 | 0 | 1 | 0 | 8 | 0 | 9 | 2 | 10 | 3 | 8 | 4 | 1 | 3 | 2 | 2 | 0 | 7 | 2 | 5 | 0 | 0 | 0 | 8 | 9 | 4 |

| Table D | COMPOUND | | | | | | | | | |
|---|---|---|---|---|---|---|---|---|---|---|
| Rate (32 g/ha) | 63 | 64 | 70 | 71 | 72 | 73 | 78 | 91 | 101 | 104 |
| PADDY | | | | | | | | | | |
| 1-LF B.Y.Grass | - | - | - | - | - | - | - | - | - | - |
| 2-LF B.Y.Grass | 10 | 10 | 10 | 10 | 10 | 10 | 0 | 10 | 9 | 0 |
| 3-lf B.Y.Grass | 10 | 4 | 10 | 10 | 10 | 4 | 0 | 10 | 10 | 0 |
| Jap Direct Seed | 2 | 5 | 4 | 4 | 9 | 2 | 0 | 10 | 0 | 0 |
| Jap Rice Eff | 3 | 1 | 8 | 5 | 10 | 1 | 0 | 9 | 0 | 0 |

Table D

| | COMPOUND | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| Rate (16 g/ha) | 2 | 3 | 4 | 5 | 6 | 7 | 8 | 10 | 11 | 14 | 15 | 19 | 22 | 23 | 24 | 25 | 28 | 30 | 31 | 32 | 35 | 41 | 42 | 43 | 46 | 47 | 49 | 55 | 60 |
| PADDY | | | | | | | | | | | | | | | | | | | | | | | | | | | | | |
| 1-LF B.Y.Grass | - | - | - | - | - | - | 9 | 10 | - | - | - | - | - | - | - | - | - | - | - | - | - | - | - | - | - | - | - | - | - |
| 2-LF B.Y.Grass | 10 | 10 | 10 | 10 | 1 | 10 | 4 | 10 | 8 | 10 | 10 | 1 | 1 | 10 | 8 | 10 | 10 | 10 | 10 | 0 | 10 | 10 | 10 | 10 | 10 | 0 | 10 | 4 | 1 |
| 3-lf B.Y.Grass | 10 | 10 | 10 | 10 | 1 | 10 | 0 | 10 | 5 | 10 | 9 | 3 | 0 | 9 | 2 | 8 | 10 | 10 | 10 | 0 | 10 | 10 | 10 | 10 | 10 | 3 | 10 | 0 | - |
| Jap Direct Seed | 4 | 4 | 3 | 0 | 0 | 3 | 1 | 6 | 0 | 5 | 1 | 2 | 1 | 3 | 3 | 1 | 2 | 3 | 2 | 0 | 2 | 2 | 2 | 3 | 3 | 0 | 7 | 3 | 0 |
| Jap Rice Eff | 1 | 0 | 5 | 1 | 0 | 1 | 0 | 7 | 0 | 5 | 4 | 9 | 0 | 5 | 5 | 0 | 1 | 0 | 0 | 0 | 1 | 2 | 3 | 0 | 0 | 0 | 3 | 1 | 2 |

| | | | | | | | | | | | | | | | | | | | | | | | | | |
|---|---|---|---|---|---|---|---|---|---|---|---|---|---|---|---|---|---|---|---|---|---|---|---|---|---|
| Rate (8 g/ha) | 2 | 3 | 4 | 7 | 8 | 10 | 14 | 22 | 23 | 24 | 25 | 28 | 35 | 41 | 42 | 43 | 55 | 63 | 64 | 70 | 71 | 72 | 73 | 91 | 101 |
| PADDY | | | | | | | | | | | | | | | | | | | | | | | | | |
| 1-LF B.Y.Grass | - | - | - | - | 6 | 10 | - | - | - | - | - | - | - | - | - | - | - | - | - | - | - | - | - | - | - |
| 2-LF B.Y.Grass | 10 | 10 | 8 | 4 | 7 | 10 | 10 | 0 | 10 | 0 | 9 | 10 | 10 | 10 | 10 | 10 | 0 | 9 | 6 | 10 | 10 | 10 | 10 | 10 | 2 |
| 3-lf B.Y.Grass | 10 | 10 | 10 | 10 | 0 | 9 | 10 | 0 | 5 | 0 | 4 | 9 | 10 | 8 | 10 | 10 | 0 | 1 | 0 | 9 | 5 | 10 | 0 | 9 | 0 |
| Jap Direct Seed | 0 | 2 | 0 | 0 | 0 | 3 | 2 | 0 | 2 | 1 | 0 | 0 | 1 | 2 | 1 | 0 | 1 | 0 | 3 | 2 | 1 | 4 | 0 | 8 | 0 |
| Jap Rice Eff | 0 | 0 | 0 | 0 | 0 | 1 | 1 | 0 | 0 | 0 | 0 | 1 | 0 | 2 | 2 | 0 | 0 | 0 | 0 | 1 | 2 | 1 | 0 | 5 | 0 |

| Table D | COMPOUND | | | | | | | | |
|---|---|---|---|---|---|---|---|---|---|
| Rate (16 g/ha) | 63 | 64 | 70 | 71 | 72 | 73 | 78 | 91 | 101 |
| PADDY | | | | | | | | | |
| 1-LF B.Y.Grass | - | - | - | - | - | - | - | - | - |
| 2-LF B.Y.Grass | 9 | 9 | 10 | 10 | 10 | 10 | 0 | 10 | 9 |
| 3-lf B.Y.Grass | 2 | 2 | 10 | 10 | 10 | 10 | 0 | 10 | 2 |
| Jap Direct Seed | 1 | 4 | 2 | 2 | 4 | 1 | 0 | 10 | 0 |
| Jap Rice Eff | 0 | 0 | 2 | 1 | 9 | 1 | 0 | 9 | 0 |

| Table D | COMPOUND | | | | | | | | | | | | | | | | |
|---|---|---|---|---|---|---|---|---|---|---|---|---|---|---|---|---|---|
| Rate (4 g/ha) | 2 | 3 | 4 | 7 | 14 | 22 | 23 | 24 | 28 | 41 | 42 | 43 | 64 | 70 | 71 | 72 | 91 |
| PADDY | | | | | | | | | | | | | | | | | |
| 2-LF B.Y.Grass | 4 | 10 | 7 | 3 | 9 | 0 | 1 | 0 | 9 | 3 | 7 | 4 | 5 | 7 | 9 | 10 | 9 |
| 3-lf B.Y.Grass | 8 | 4 | 9 | 7 | 8 | 0 | 0 | 0 | 2 | 4 | 4 | 4 | 0 | 3 | 0 | 9 | 2 |
| Jap Direct Seed | 0 | 0 | 0 | 0 | 1 | 0 | 1 | 0 | 0 | 1 | 1 | 0 | 3 | 1 | 1 | 3 | 1 |
| Jap Rice Eff | 0 | 0 | 0 | 0 | 1 | 0 | 1 | 0 | 0 | 0 | 1 | 0 | 0 | 1 | 1 | 1 | 2 |

| Rate (2 g/ha) | 91 |
|---|---|
| PADDY | |
| 2-LF B.Y.Grass | 2 |
| 3-lf B.Y.Grass | 0 |
| Jap Direct Seed | 1 |
| Jap Rice Eff | 1 |

## TEST E

Plastic pots were partially filled with silt loam soil. The soil was then saturated with water. Japonica rice (Oryza sativa) seedlings at the 2.0 to 2.5 leaf stage, seeds selected from barnyardgrass (Echinochloa crus-galli), duck salad (Heteranthera limosa), umbrella sedge (Cyperus difformis), and tubers selected from waterchestnut (Eleocharis spp.), were planted into this soil. After planting, water levels were raised to 3 cm above the soil surface and maintained at this level throughout the test. Chemical treatments were formulated in a non-phytotoxic solvent and applied directly to the paddy water. Treated plants and controls were maintained in a greenhouse for approximately 21 days, after which all species were compared to controls and visually evaluated. Plant response ratings, summarized in Table E, are reported on a 0 to 10 scale where 0 is no effect and 10 is complete control. A dash (-) response means no test result.

| Table E | COMPOUND |
|---|---|
| Rate (32 g/ha) | 10 |
| PADDY | |
| Barnyardgrass | 10 |
| Duck salad | 0 |
| Japonica rice | 2 |
| Umbrella sedge | 0 |
| Waterchestnut | 0 |
| | |
| Rate (16 g/ha) | 10 |
| PADDY | |
| Barnyardgrass | 10 |
| Duck salad | 0 |
| Japonica rice | 2 |
| Umbrella sedge | 0 |
| Waterchestnut | 0 |
| | |
| Rate (8 g/ha) | 10 |
| PADDY | |
| Barnyardgrass | 7 |
| Duck salad | 0 |
| Japonica rice | 1 |
| Umbrella sedge | 0 |
| Waterchestnut | 0 |

| Table E | COMPOUND |
|---|---|
| Rate (4 g/ha) | 10 |
| PADDY | |
| Barnyardgrass | 4 |
| Duck salad | 0 |
| Japonica rice | 0 |
| Umbrella sedge | 0 |
| Waterchestnut | 0 |
| | |
| Rate (2 g/ha) | 10 |
| PADDY | |
| Barnyardgrass | 1 |
| Duck salad | 0 |
| Japonica rice | 0 |
| Umbrella sedge | 0 |
| Waterchestnut | 0 |

## TEST F

Seeds of barnyardgrass (Echinochloa crus-galli), black nightshade (Solanum ptycanthum dunal), cocklebur (Xanthium pensylvanicum), common ragweed (Ambrosia elatior), corn (Zea mays), cotton (Gossypium hirsutam), crabgrass (Digitaria spp.), fall panicum (Panicum dichotomiflorum), giant foxtail (Setaria faberii), green foxtail (Setaria viridis), jimsonweed (Datura stramomium), johnson grass (Sorghum halepense), morningglory (Ipomoea spp.), signalgrass (Brachiaria platyphylla), soybean (Glycine max), velvetleaf (Abutilon theophrasti), wild proso (Pancium miliaceum) and purple nutsedge (Cyperus rotundus) tubers were planted into a silt loam soil. These crops and weeds were grown in the greenhouse until the plants ranged in height from two to eighteen cm (one to four leaf stage), then treated postemergence with the test chemicals dissolved in a non-phytotoxic solvent. Pots receiving these postemergence treatments were placed in the greenhouse and maintained according to routine greenhouse procedures.

Treated plants and untreated controls were maintained in the greenhouse approximately 21 days, then the treated plants were compared to controls and visually evaluated. Plant response ratings, summarized in Table F, are reported on a 0 to 10 scale where 0 is no effect and 10 is complete control. A dash (-) response means no test result.

| Table F | COMPOUND | |
|---|---|---|
| Rate (250 g/ha) | 32 | 56 |
| POSTEMERGENCE | | |
| Barnyardgrass | 10 | 10 |
| Corn G4689A | 3 | 4 |
| Cotton | 0 | 0 |
| Crabgrass | 10 | 10 |
| Fall Panicum | 10 | 9 |
| Giant Foxtail | 10 | 10 |
| Green Foxtail | 10 | 10 |
| Johnson Grass | 10 | 10 |
| Soybean | 1 | 1 |
| Wild Proso | 10 | 10 |

| Rate (125 g/ha) | 32 | 38 | 56 | 64 | 86 | 103 |
|---|---|---|---|---|---|---|
| POSTEMERGENCE | | | | | | |
| Barnyardgrass | 10 | 10 | 10 | 2 | 10 | 7 |
| Corn G4689A | 2 | 3 | 2 | 6 | 4 | 0 |
| Cotton | 0 | 0 | 0 | 0 | 0 | 0 |
| Crabgrass | 10 | 10 | 10 | 10 | 10 | 10 |
| Fall Panicum | 9 | 10 | 7 | 9 | 10 | 7 |
| Giant Foxtail | 10 | 10 | 10 | 10 | 10 | 10 |
| Green Foxtail | 10 | 10 | 10 | 10 | 10 | 7 |
| Johnson Grass | 10 | 10 | 10 | 10 | 10 | 10 |
| Signalgrass | - | - | - | - | 10 | 10 |
| Soybean | 1 | 2 | - | 2 | 0 | 0 |
| Wild Proso | 10 | 7 | 10 | 10 | 10 | 6 |

| Table F | COMPOUND | | | | | | | | |
|---|---|---|---|---|---|---|---|---|---|
| Rate (62 g/ha) | 32 | 38 | 42 | 43 | 46 | 56 | 64 | 86 | 103 |
| POSTEMERGENCE | | | | | | | | | |
| Barnyardgrass | 10 | 10 | - | 10 | 10 | 9 | 1 | 10 | 0 |
| Corn G4689A | 1 | 2 | 9 | 5 | 9 | 0 | 1 | 1 | 0 |
| Cotton | 0 | 0 | 0 | 0 | 0 | 0 | 0 | 0 | 0 |
| Crabgrass | 10 | 10 | 7 | 10 | 9 | 10 | 7 | 10 | 7 |
| Fall Panicum | 8 | 10 | 9 | 10 | 10 | 6 | 6 | 10 | 2 |
| Giant Foxtail | 9 | 10 | 10 | 10 | 10 | 9 | 10 | 10 | 9 |
| Green Foxtail | 9 | 10 | 10 | 10 | 10 | 10 | 10 | 10 | 6 |
| Johnson Grass | 10 | 10 | 10 | 10 | 10 | 10 | 10 | 10 | 6 |
| Nutsedge | - | - | 0 | 0 | 0 | - | - | - | - |
| Signalgrass | - | - | 10 | 10 | 10 | - | - | 10 | 8 |
| Soybean | 0 | 1 | 0 | 1 | 1 | 0 | 2 | 0 | 0 |
| Wild Proso | 8 | 9 | - | 10 | 10 | 8 | 10 | 10 | 4 |

| Table F | COMPOUND | | | | | | | | | | |
|---|---|---|---|---|---|---|---|---|---|---|---|
| Rate (31 g/ha) | 10 | 32 | 38 | 42 | 43 | 46 | 56 | 64 | 71 | 86 | 103 |
| POSTEMERGENCE | | | | | | | | | | | |
| Barnyardgrass | 10 | 8 | 9 | - | 10 | 10 | 8 | 1 | 10 | 10 | 0 |
| Black Nightshae | 0 | - | - | - | - | - | - | - | - | - | - |
| Cocklebur | 0 | - | - | - | - | - | - | - | - | - | - |
| Common Ragweed | 0 | - | - | - | - | - | - | - | - | - | - |
| Corn G4689A | 10 | 0 | 1 | 8 | 5 | 4 | 0 | 0 | 4 | 1 | 0 |
| Cotton | 0 | 0 | 0 | 0 | 0 | 0 | 0 | 0 | 0 | 0 | 0 |
| Crabgrass | 9 | 8 | 9 | 7 | 10 | 8 | 9 | 5 | 10 | 10 | 5 |
| Fall Panicum | - | 5 | 8 | 9 | 8 | 10 | 4 | 2 | 10 | 10 | 1 |
| Giant Foxtail | 9 | 7 | 10 | 9 | 10 | 10 | 4 | 6 | 10 | 10 | 8 |
| Green Foxtail | - | 7 | 10 | 10 | 10 | 10 | 5 | 9 | 10 | 9 | 6 |
| Jimson weed | 0 | - | - | - | - | - | - | - | - | - | - |
| Johnson Grass | 10 | 6 | 10 | 10 | 10 | 10 | 7 | 7 | 10 | 10 | 6 |
| Morningglory | 0 | - | - | - | - | - | - | - | - | - | - |
| Nutsedge | 0 | - | - | 0 | 0 | 0 | - | - | - | - | - |
| Signalgrass | - | - | - | 10 | 10 | 10 | - | - | - | 10 | 4 |
| Soybean | 0 | 0 | 0 | 0 | 1 | 1 | 0 | 2 | 2 | 0 | 0 |
| Velvetleaf | 0 | - | - | - | - | - | - | - | - | - | - |
| Wild Proso | - | 4 | 8 | - | 10 | 10 | 6 | 10 | 10 | 10 | 1 |

| Table F | COMPOUND | | | | | | | | | | |
|---|---|---|---|---|---|---|---|---|---|---|---|
| Rate (16 g/ha) | 10 | 32 | 38 | 42 | 43 | 46 | 56 | 64 | 71 | 86 | 103 |
| POSTEMERGENCE | | | | | | | | | | | |
| Barnyardgrass | 10 | 5 | 8 | - | 9 | 10 | 5 | 0 | 10 | 10 | 0 |
| Black Nightshae | 0 | - | - | - | - | - | - | - | - | - | - |
| Cocklebur | 0 | - | - | - | - | - | - | - | - | - | - |
| Common Ragweed | 0 | - | - | - | - | - | - | - | - | - | - |
| Corn G4689A | 10 | 0 | 0 | 1 | 3 | 0 | 0 | 0 | 3 | 1 | 0 |
| Cotton | 0 | 0 | 0 | 0 | 0 | 0 | 0 | 0 | 0 | 0 | 0 |
| Crabgrass | 8 | 6 | 7 | 7 | 8 | 8 | 6 | 5 | 9 | 6 | 0 |
| Fall Panicum | - | 3 | 6 | 9 | 8 | 10 | 2 | 2 | 10 | 9 | 0 |
| Giant Foxtail | 9 | 5 | 9 | 9 | 10 | 10 | 2 | 2 | 10 | 9 | 6 |
| Green Foxtail | - | 5 | 9 | 9 | 10 | 10 | 3 | 6 | 10 | 8 | 4 |
| Jimson weed | 0 | - | - | - | - | - | - | - | - | - | - |
| Johnson Grass | 10 | 4 | 7 | 10 | 10 | 10 | 4 | 6 | 10 | 10 | 2 |
| Morningglory | 0 | - | - | - | - | - | - | - | - | - | - |
| Nutsedge | 0 | - | - | 0 | 0 | 0 | - | - | - | - | - |
| Signalgrass | - | - | - | 10 | 10 | 9 | - | - | - | 10 | 0 |
| Soybean | 0 | 0 | 0 | 0 | 0 | 1 | 0 | 2 | 1 | 0 | 0 |
| Velvetleaf | 0 | - | - | - | - | - | - | - | - | - | - |
| Wild Proso | - | 1 | 4 | - | 8 | 8 | 2 | 6 | 10 | 10 | 0 |

| Table F | COMPOUND | | | | | | | | |
|---|---|---|---|---|---|---|---|---|---|
| Rate (8 g/ha) | 10 | 38 | 42 | 43 | 46 | 64 | 71 | 86 | 103 |
| POSTEMERGENCE | | | | | | | | | |
| Barnyardgrass | 10 | 4 | - | 5 | 6 | 0 | 10 | 10 | 0 |
| Black Nightshae | 0 | - | - | - | - | - | - | - | - |
| Cocklebur | 0 | - | - | - | - | - | - | - | - |
| Common Ragweed | 0 | - | - | - | - | - | - | - | - |
| Corn G4689A | 10 | 0 | 1 | 0 | 0 | 0 | 1 | - | 0 |
| Cotton | 0 | 0 | 0 | 0 | 0 | 0 | 0 | 0 | 0 |
| Crabgrass | 6 | 5 | 6 | 6 | 6 | 0 | 9 | 1 | 0 |
| Fall Panicum | - | 3 | 9 | 5 | 10 | 0 | 9 | 5 | 0 |
| Giant Foxtail | 8 | 4 | 8 | 9 | 10 | 0 | 10 | 8 | 1 |
| Green Foxtail | - | 5 | 8 | 8 | 10 | 0 | 10 | 8 | 1 |
| Jimson weed | 0 | - | - | - | - | - | - | - | - |
| Johnson Grass | 10 | 4 | 9 | 9 | 7 | 0 | 9 | 9 | 0 |
| Morningglory | 0 | - | - | - | - | - | - | - | - |
| Nutsedge | 0 | - | 0 | 0 | 0 | - | - | - | - |
| Signalgrass | - | - | 10 | 10 | 9 | - | - | 9 | 0 |
| Soybean | 0 | 0 | 0 | 0 | 1 | 0 | - | 0 | 0 |
| Velvetleaf | 0 | - | - | - | - | - | - | - | - |
| Wild Proso | - | 2 | - | 6 | 6 | 0 | 8 | 7 | 0 |

| Table F | COMPOUND | | | | |
|---|---|---|---|---|---|
| Rate (4 g/ha) | 10 | 42 | 43 | 46 | 71 |
| POSTEMERGENCE | | | | | |
| Barnyardgrass | 9 | - | 2 | 1 | 7 |
| Black Nightshae | 0 | - | - | - | - |
| Cocklebur | 0 | - | - | - | - |
| Common Ragweed | 0 | - | - | - | - |
| Corn G4689A | 7 | 0 | 0 | 0 | 0 |
| Cotton | 0 | 0 | 0 | 0 | 0 |
| Crabgrass | 4 | 2 | 1 | 0 | 6 |
| Fall Panicum | - | 2 | 1 | 7 | 6 |
| Giant Foxtail | 6 | 5 | 2 | 4 | 6 |
| Green Foxtail | - | 0 | 5 | 4 | 7 |
| Jimson weed | 0 | - | - | - | - |
| Johnson Grass | 8 | 1 | 4 | 0 | 5 |
| Morningglory | 0 | - | - | - | - |
| Nutsedge | 0 | 0 | 0 | 0 | - |
| Signalgrass | - | 3 | 5 | - | - |
| Soybean | 0 | 0 | 0 | 0 | 0 |
| Velvetleaf | 0 | - | - | - | - |
| Wild Proso | - | - | 2 | 1 | 3 |

| Table F | COMPOUND | |
|---|---|---|
| Rate (2 g/ha) | 10 | 71 |
| POSTEMERGENCE | | |
| Barnyardgrass | 0 | 4 |
| Black Nightshae | 0 | - |
| Cocklebur | 0 | - |
| Common Ragweed | 0 | - |
| Corn G4689A | 0 | 0 |
| Cotton | 0 | 0 |
| Crabgrass | 1 | 3 |
| Fall Panicum | - | 2 |
| Giant Foxtail | 0 | 4 |
| Green Foxtail | - | 4 |
| Jimson weed | 0 | - |
| Johnson Grass | 0 | 2 |
| Morningglory | 0 | - |
| Nutsedge | 0 | - |
| Soybean | 0 | 0 |
| Velvetleaf | 0 | - |
| Wild Proso | - | 1 |

## TEST G

Compounds evaluated in this test were formulated in a non-phytoxic solvent and applied to the soil surface before plant seedlings emerged (preemergence application) and to plants that were in the one-to-four leaf stage (postemergence application). A sandy loam soil was used for the preemergence test while a mixture of sandy loam soil and greenhouse potting mix in a 60:40 ratio was used for the postemergence test. Test compounds were applied within approximately one day after planting seeds for the preemergence test. Plantings of these crops and weed species were adjusted to produce plants of appropriate size for the postemergence test. All plant species were grown using normal greenhouse practices. Crop and weed species include winter barley (Hordeum vulgare cv. 'Igri'), blackgrass (Alopecurus myosuroides), chickweed (Stellaria media), downy brome (Bromus tectorum), field violet (Viola arvensis), galium (Galium aparine), green foxtail (Setaria viridis), kochia (Kochia scoparia), lambsquarters (Chenopodium album), Persian speedwell (Veronica persica), rape (Brassica napus cv. 'Jet Neuf'), ryegrass (Lolium multiflorum), sugar beet (Beta vulgaris cv. 'US1'), sunflower (Helianthus annuus cv. 'Russian Giant'), spring wheat (Triticum aestivum cv. 'ERA'), winter wheat (Triticum aestivum cv. 'Talent'), wild buckwheat (Polygonum convolvulus), wild mustard (Sinapis arvensis), wild oat (Avena fatua), and wild radish (Raphanus raphanistrum). Blackgrass, galium and wild oat were treated at two growth stages. The first stage (1) was when the plants had two to three leaves. The second stage (2) was when the plants had approximately four leaves or in the initial stages of tillering. Treated plants and untreated controls were maintained in a greenhouse for approximately 21 to 28 days, after which all treated plants were compared to untreated controls and visually evaluated. Plant response ratings, summarized in Table G, are based upon a 0 to 10 scale where 0 is no effect and 10 is complete control. A dash response (-) means no test result.

| Table G | COMPOUND | |
|---|---|---|
| Rate (500 g/ha) | 11 | 26 |
| POSTEMERGENCE | | |
| Blackgrass (1) | 2 | 3 |
| Blackgrass (2) | 3 | 2 |
| Chickweed | 0 | 0 |
| Downy brome | 8 | 0 |
| Field violet | 0 | 0 |
| Galium (1) | 0 | 0 |
| Galium (2) | 0 | 0 |
| Green foxtail | 10 | 8 |
| Kochia | 0 | 0 |
| Lambsquarters | - | - |
| Persn Speedwell | 0 | 0 |
| Rape | 0 | 0 |
| Ryegrass | 2 | 0 |
| Sugar beet | 0 | 0 |
| Sunflower | 0 | 0 |
| Wheat (Spring) | 10 | 9 |
| Wheat (Winter) | 10 | 8 |
| Wild buckwheat | 0 | 0 |
| Wild mustard | 0 | 0 |
| Wild oat (1) | 10 | 10 |
| Wild oat (2) | 10 | 10 |
| Wild radish | 0 | - |
| Winter Barley | 10 | 10 |

| Table G | COMPOUND | | |
|---|---|---|---|
| Rate (500 g/ha) | 11 | 22 | 26 |
| PREEMERGENCE | | | |
| Blackgrass (1) | 0 | 10 | 0 |
| Blackgrass (2) | 0 | 10 | 0 |
| Chickweed | 0 | 0 | 0 |
| Downy brome | 0 | 7 | 0 |
| Field violet | 0 | - | 0 |
| Galium (1) | 0 | 0 | 0 |
| Galium (2) | 0 | 0 | 0 |
| Green foxtail | 0 | 10 | 0 |
| Kochia | 0 | 0 | 0 |
| Lambsquarters | - | 3 | 0 |
| Persn Speedwell | 0 | 0 | 0 |
| Rape | 0 | 0 | 0 |
| Ryegrass | 0 | 10 | 0 |
| Sugar beet | 0 | 5 | 0 |
| Sunflower | 0 | 0 | 0 |
| Wheat (Spring) | 0 | 9 | 0 |
| Wheat (Winter) | 0 | 10 | 0 |
| Wild buckwheat | 0 | 0 | 0 |
| Wild mustard | 0 | 0 | 0 |
| Wild oat (1) | 0 | 7 | 0 |
| Wild oat (2) | 0 | 8 | 0 |
| Wild radish | 0 | 3 | 0 |
| Winter Barley | 0 | 9 | 0 |

| Table G | COMPOUND | | |
|---|---|---|---|
| Rate (250 g/ha) | 10 | 11 | 26 |
| POSTEMERGENCE | | | |
| Blackgrass (1) | 10 | 3 | 0 |
| Blackgrass (2) | 10 | 3 | 0 |
| Chickweed | 0 | 0 | 0 |
| Downy brome | 10 | 4 | 0 |
| Field violet | 0 | 0 | 0 |
| Galium (1) | 0 | 0 | 0 |
| Galium (2) | 0 | 0 | 0 |
| Green foxtail | 10 | 10 | 3 |
| Kochia | 0 | 0 | 0 |
| Lambsquarters | 0 | 0 | - |
| Persn Speedwell | 0 | 0 | 0 |
| Rape | 0 | 0 | 0 |
| Ryegrass | 10 | 2 | 0 |
| Sugar beet | 0 | 0 | 0 |
| Sunflower | 0 | 0 | 0 |
| Wheat (Spring) | 10 | 8 | 5 |
| Wheat (Winter) | 10 | 8 | 4 |
| Wild buckwheat | 0 | 0 | 0 |
| Wild mustard | 0 | 0 | 0 |
| Wild oat (1) | 10 | 10 | 10 |
| Wild oat (2) | 10 | 10 | 10 |
| Wild radish | 0 | 0 | - |
| Winter Barley | 10 | 9 | 7 |

| Table G | COMPOUND | | | | |
|---|---|---|---|---|---|
| Rate (250 g/ha) | 10 | 11 | 15 | 22 | 26 |
| PREEMERGENCE | | | | | |
| Blackgrass (1) | 10 | 0 | 0 | 10 | 0 |
| Blackgrass (2) | 10 | 0 | 0 | 10 | 0 |
| Chickweed | 0 | 0 | 0 | 0 | 0 |
| Downy brome | 6 | 0 | 0 | 3 | 0 |
| Field violet | 0 | 0 | - | - | 0 |
| Galium (1) | 0 | 0 | 0 | 0 | 0 |
| Galium (2) | 0 | 0 | 0 | 0 | 0 |
| Green foxtail | 10 | 0 | - | 10 | 0 |
| Kochia | 0 | 0 | 0 | 0 | 0 |
| Lambsquarters | 0 | - | 0 | 0 | 0 |
| Persn Speedwell | 0 | 0 | 0 | 0 | 0 |
| Rape | 0 | 0 | 0 | 0 | 0 |
| Ryegrass | 6 | 0 | 0 | 10 | 0 |
| Sugar beet | 0 | 0 | 0 | 2 | 0 |
| Sunflower | 0 | 0 | 0 | 0 | 0 |
| Wheat (Spring) | 3 | 0 | 0 | 7 | 0 |
| Wheat (Winter) | 3 | 0 | 0 | 7 | 0 |
| Wild buckwheat | 0 | 0 | 0 | 0 | 0 |
| Wild mustard | 0 | 0 | 0 | 0 | 0 |
| Wild oat (1) | 3 | 0 | 0 | 3 | 0 |
| Wild oat (2) | 4 | 0 | 0 | 4 | 0 |
| Wild radish | 0 | 0 | 0 | 0 | 0 |
| Winter Barley | 3 | 0 | 0 | 6 | 0 |

| Table G | COMPOUND | | |
|---|---|---|---|
| Rate (125 g/ha) | 10 | 11 | 26 |
| POSTEMERGENCE | | | |
| Blackgrass (1) | 10 | 0 | 0 |
| Blackgrass (2) | 10 | 0 | 0 |
| Chickweed | 0 | 0 | 0 |
| Downy brome | 10 | 2 | 0 |
| Field violet | 0 | 0 | 0 |
| Galium (1) | 0 | 0 | 0 |
| Galium (2) | 0 | 0 | 0 |
| Green foxtail | 10 | 10 | 0 |
| Kochia | 0 | 0 | 0 |
| Lambsquarters | 0 | 0 | - |
| Persn Speedwell | 0 | 0 | 0 |
| Rape | 0 | 0 | 0 |
| Ryegrass | 10 | 0 | 0 |
| Sugar beet | 0 | 0 | 0 |
| Sunflower | 0 | 0 | 0 |
| Wheat (Spring) | 10 | 5 | 2 |
| Wheat (Winter) | 10 | 6 | 2 |
| Wild buckwheat | 0 | 0 | 0 |
| Wild mustard | 0 | 0 | 0 |
| Wild oat (1) | 10 | 7 | 8 |
| Wild oat (2) | 10 | 8 | 9 |
| Wild radish | 0 | 0 | - |
| Winter Barley | 10 | 6 | 3 |

| Table G | COMPOUND | | | | |
|---|---|---|---|---|---|
| Rate (125 g/ha) | 10 | 11 | 15 | 22 | 26 |
| PREEMERGENCE | | | | | |
| Blackgrass (1) | 7 | 0 | 0 | 9 | 0 |
| Blackgrass (2) | 7 | 0 | 0 | 8 | 0 |
| Chickweed | 0 | 0 | 0 | 0 | 0 |
| Downy brome | 3 | 0 | 0 | 0 | 0 |
| Field violet | 0 | 0 | - | - | 0 |
| Galium (1) | 0 | 0 | 0 | 0 | 0 |
| Galium (2) | 0 | 0 | 0 | 0 | 0 |
| Green foxtail | 10 | 0 | - | 10 | 0 |
| Kochia | 0 | 0 | 0 | 0 | 0 |
| Lambsquarters | 0 | - | 0 | 0 | 0 |
| Persn Speedwell | 0 | 0 | 0 | 0 | 0 |
| Rape | 0 | 0 | 0 | 0 | 0 |
| Ryegrass | 4 | 0 | 0 | 6 | 0 |
| Sugar beet | 0 | 0 | 0 | 0 | 0 |
| Sunflower | 0 | 0 | 0 | 0 | 0 |
| Wheat (Spring) | 0 | 0 | 0 | 5 | 0 |
| Wheat (Winter) | 0 | 0 | 0 | 5 | 0 |
| Wild buckwheat | 0 | 0 | 0 | 0 | 0 |
| Wild mustard | 0 | 0 | 0 | 0 | 0 |
| Wild oat (1) | 0 | 0 | 0 | 2 | 0 |
| Wild oat (2) | 2 | 0 | 0 | 2 | 0 |
| Wild radish | 0 | 0 | 0 | 0 | 0 |
| Winter Barley | 0 | 0 | 0 | 2 | 0 |

| Table G | COMPOUND | | | | |
|---|---|---|---|---|---|
| Rate (64 g/ha) | 10 | 11 | 15 | 22 | 26 |
| POSTEMERGENCE | | | | | |
| Blackgrass (1) | 10 | 0 | 8 | 8 | 0 |
| Blackgrass (2) | 10 | 0 | 7 | 7 | 0 |
| Chickweed | 0 | 0 | 0 | 0 | 0 |
| Downy brome | 10 | 0 | 7 | 10 | 0 |
| Field violet | 0 | 0 | 0 | 0 | 0 |
| Galium (1) | 0 | 0 | 0 | 0 | 0 |
| Galium (2) | 0 | 0 | 0 | 0 | 0 |
| Green foxtail | 10 | 9 | 10 | 10 | 0 |
| Kochia | 0 | 0 | 0 | 0 | 0 |
| Lambsquarters | 0 | 0 | 0 | 0 | - |
| Persn Speedwell | 0 | 0 | 0 | 0 | 0 |
| Rape | 0 | 0 | 0 | 0 | 0 |
| Ryegrass | 10 | 0 | 4 | 10 | 0 |
| Sugar beet | 0 | 0 | 0 | 0 | 0 |
| Sunflower | 0 | 0 | 0 | 0 | 0 |
| Wheat (Spring) | 10 | 2 | 10 | 10 | 0 |
| Wheat (Winter) | 10 | 3 | 10 | 10 | 0 |
| Wild buckwheat | 0 | 0 | 0 | 0 | 0 |
| Wild mustard | 0 | 0 | 0 | 0 | 0 |
| Wild oat (1) | 10 | 3 | 6 | 10 | 5 |
| Wild oat (2) | 10 | 4 | 6 | 10 | 4 |
| Wild radish | 0 | 0 | 0 | 0 | - |
| Winter Barley | 10 | 2 | 10 | 10 | 0 |

| Table G | COMPOUND | | | |
|---|---|---|---|---|
| Rate (64 g/ha) | 10 | 11 | 15 | 22 |
| PREEMERGENCE | | | | |
| Blackgrass (1) | 2 | 0 | 0 | 7 |
| Blackgrass (2) | 2 | 0 | 0 | 6 |
| Chickweed | 0 | 0 | 0 | 0 |
| Downy brome | 0 | 0 | 0 | 0 |
| Field violet | 0 | 0 | - | - |
| Galium (1) | 0 | 0 | 0 | 0 |
| Galium (2) | 0 | 0 | 0 | 0 |
| Green foxtail | 5 | 0 | - | 6 |
| Kochia | 0 | 0 | 0 | 0 |
| Lambsquarters | 0 | - | 0 | 0 |
| Persn Speedwell | 0 | 0 | 0 | 0 |
| Rape | 0 | 0 | 0 | 0 |
| Ryegrass | 2 | 0 | 0 | 3 |
| Sugar beet | 0 | 0 | 0 | 0 |
| Sunflower | 0 | 0 | 0 | 0 |
| Wheat (Spring) | 0 | 0 | 0 | 2 |
| Wheat (Winter) | 0 | 0 | 0 | 2 |
| Wild buckwheat | 0 | 0 | 0 | 0 |
| Wild mustard | 0 | 0 | 0 | 0 |
| Wild oat (1) | 0 | 0 | 0 | 0 |
| Wild oat (2) | 0 | 0 | 0 | 0 |
| Wild radish | 0 | 0 | 0 | 0 |
| Winter Barley | 0 | 0 | 0 | 0 |

| Table G | COMPOUND | | | |
|---|---|---|---|---|
| Rate (32 g/ha) | 10 | 11 | 15 | 22 |
| POSTEMERGENCE | | | | |
| Blackgrass (1) | 10 | 0 | 6 | 4 |
| Blackgrass (2) | 10 | 0 | 5 | 3 |
| Chickweed | 0 | 0 | 0 | 0 |
| Downy brome | 10 | 0 | 5 | 10 |
| Field violet | 0 | 0 | 0 | 0 |
| Galium (1) | 0 | 0 | 0 | 0 |
| Galium (2) | 0 | 0 | 0 | 0 |
| Green foxtail | 10 | 4 | 10 | 10 |
| Kochia | 0 | 0 | 0 | 0 |
| Lambsquarters | 0 | 0 | 0 | 0 |
| Persn Speedwell | 0 | 0 | 0 | 0 |
| Rape | 0 | 0 | 0 | 0 |
| Ryegrass | 9 | 0 | 2 | 8 |
| Sugar beet | 0 | 0 | 0 | 0 |
| Sunflower | 0 | 0 | 0 | 0 |
| Wheat (Spring) | 10 | 0 | 8 | 10 |
| Wheat (Winter) | 10 | 0 | 9 | 10 |
| Wild buckwheat | 0 | 0 | 0 | 0 |
| Wild mustard | 0 | 0 | 0 | 0 |
| Wild oat (1) | 10 | 0 | 4 | 9 |
| Wild oat (2) | 10 | 0 | 4 | 8 |
| Wild radish | 0 | - | 0 | 0 |
| Winter Barley | 10 | 0 | 10 | 10 |

| Table G | COMPOUND | | |
|---|---|---|---|
| Rate (32 g/ha) | 10 | 15 | 22 |
| PREEMERGENCE | | | |
| Blackgrass (1) | 0 | 0 | 3 |
| Blackgrass (2) | 0 | 0 | 3 |
| Chickweed | 0 | 0 | 0 |
| Downy brome | 0 | 0 | 0 |
| Field violet | 0 | - | - |
| Galium (1) | 0 | 0 | 0 |
| Galium (2) | 0 | 0 | 0 |
| Green foxtail | 2 | - | 2 |
| Kochia | 0 | 0 | 0 |
| Lambsquarters | 0 | 0 | 0 |
| Persn Speedwell | 0 | 0 | 0 |
| Rape | 0 | 0 | 0 |
| Ryegrass | 0 | 0 | 0 |
| Sugar beet | 0 | 0 | 0 |
| Sunflower | 0 | 0 | 0 |
| Wheat (Spring) | 0 | 0 | 0 |
| Wheat (Winter) | 0 | 0 | 0 |
| Wild buckwheat | 0 | 0 | 0 |
| Wild mustard | 0 | 0 | 0 |
| Wild oat (1) | 0 | 0 | 0 |
| Wild oat (2) | 0 | 0 | 0 |
| Wild radish | 0 | 0 | 0 |
| Winter Barley | 0 | 0 | 0 |

| Table G | COMPOUND | | |
|---|---|---|---|
| Rate (16 g/ha) | 10 | 15 | 22 |
| POSTEMERGENCE | | | |
| Blackgrass (1) | 8 | 3 | 2 |
| Blackgrass (2) | 9 | 3 | 0 |
| Chickweed | 0 | 0 | 0 |
| Downy brome | 10 | 3 | 10 |
| Field violet | 0 | 0 | 0 |
| Galium (1) | 0 | 0 | 0 |
| Galium (2) | 0 | 0 | 0 |
| Green foxtail | 10 | 7 | 10 |
| Kochia | 0 | 0 | 0 |
| Lambsquarters | 0 | 0 | 0 |
| Persn Speedwell | 0 | 0 | 0 |
| Rape | 0 | 0 | 0 |
| Ryegrass | 5 | 0 | 4 |
| Sugar beet | 0 | 0 | 0 |
| Sunflower | 0 | 0 | 0 |
| Wheat (Spring) | 10 | 6 | 10 |
| Wheat (Winter) | 10 | 6 | 10 |
| Wild buckwheat | 0 | 0 | 0 |
| Wild mustard | 0 | 0 | 0 |
| Wild oat (1) | 10 | 2 | 7 |
| Wild oat (2) | 9 | 2 | 6 |
| Wild radish | 0 | 0 | 0 |
| Winter Barley | 10 | 7 | 10 |

| Table G | COMPOUND | |
|---|---|---|
| Rate (16 g/ha) | 10 | 22 |
| PREEMERGENCE | | |
| Blackgrass (1) | 0 | 0 |
| Blackgrass (2) | 0 | 0 |
| Chickweed | 0 | 0 |
| Downy brome | 0 | 0 |
| Field violet | 0 | - |
| Galium (1) | 0 | 0 |
| Galium (2) | 0 | 0 |
| Green foxtail | 0 | 0 |
| Kochia | 0 | 0 |
| Lambsquarters | 0 | 0 |
| Persn Speedwell | 0 | 0 |
| Rape | 0 | 0 |
| Ryegrass | 0 | 0 |
| Sugar beet | 0 | 0 |
| Sunflower | 0 | 0 |
| Wheat (Spring) | 0 | 0 |
| Wheat (Winter) | 0 | 0 |
| Wild buckwheat | 0 | 0 |
| Wild mustard | 0 | 0 |
| Wild oat (1) | 0 | 0 |
| Wild oat (2) | 0 | 0 |
| Wild radish | 0 | 0 |
| Winter Barley | 0 | 0 |

| Table G | COMPOUND | | |
|---|---|---|---|
| Rate (8 g/ha) | 10 | 15 | 22 |
| POSTEMERGENCE | | | |
| Blackgrass (1) | 5 | 0 | 0 |
| Blackgrass (2) | 5 | 0 | 0 |
| Chickweed | 0 | 0 | 0 |
| Downy brome | 6 | 0 | 8 |
| Field violet | 0 | 0 | 0 |
| Galium (1) | 0 | 0 | 0 |
| Galium (2) | 0 | 0 | 0 |
| Green foxtail | 8 | 2 | 6 |
| Kochia | 0 | 0 | 0 |
| Lambsquarters | 0 | 0 | 0 |
| Persn Speedwell | 0 | 0 | 0 |
| Rape | 0 | 0 | 0 |
| Ryegrass | 2 | 0 | 2 |
| Sugar beet | 0 | 0 | 0 |
| Sunflower | 0 | 0 | 0 |
| Wheat (Spring) | 7 | 3 | 9 |
| Wheat (Winter) | 8 | 2 | 8 |
| Wild buckwheat | 0 | 0 | 0 |
| Wild mustard | 0 | 0 | 0 |
| Wild oat (1) | 5 | 0 | 3 |
| Wild oat (2) | 5 | 0 | 3 |
| Wild radish | 0 | 0 | 0 |
| Winter Barley | 8 | 4 | 9 |

| Table G | COMPOUND |
|---|---|
| Rate (8 g/ha) | 10 |
| PREEMERGENCE | |
| Blackgrass (1) | 0 |
| Blackgrass (2) | 0 |
| Chickweed | 0 |
| Downy brome | 0 |
| Field violet | 0 |
| Galium (1) | 0 |
| Galium (2) | 0 |
| Green foxtail | 0 |
| Kochia | 0 |
| Lambsquarters | 0 |
| Persn Speedwell | 0 |
| Rape | 0 |
| Ryegrass | 0 |
| Sugar beet | 0 |
| Sunflower | 0 |
| Wheat (Spring) | 0 |
| Wheat (Winter) | 0 |
| Wild buckwheat | 0 |
| Wild mustard | 0 |
| Wild oat (1) | 0 |
| Wild oat (2) | 0 |
| Wild radish | 0 |
| Winter Barley | 0 |

| Table G | COMPOUND | | Table G | COMPOUND |
|---|---|---|---|---|
| Rate (4 g/ha) | 10 | 15 | Rate (4 g/ha) | 10 |
| POSTEMERGENCE | | | PREEMERGENCE | |
| Blackgrass (1) | 2 | 0 | Blackgrass (1) | 0 |
| Blackgrass (2) | 2 | 0 | Blackgrass (2) | 0 |
| Chickweed | 0 | 0 | Chickweed | 0 |
| Downy brome | 2 | 0 | Downy brome | 0 |
| Field violet | 0 | 0 | Field violet | 0 |
| Galium (1) | 0 | 0 | Galium (1) | 0 |
| Galium (2) | 0 | 0 | Galium (2) | 0 |
| Green foxtail | 3 | 0 | Green foxtail | 0 |
| Kochia | 0 | 0 | Kochia | 0 |
| Lambsquarters | 0 | 0 | Lambsquarters | 0 |
| Persn Speedwell | 0 | 0 | Persn Speedwell | 0 |
| Rape | 0 | 0 | Rape | 0 |
| Ryegrass | 0 | 0 | Ryegrass | 0 |
| Sugar beet | 0 | 0 | Sugar beet | 0 |
| Sunflower | 0 | 0 | Sunflower | 0 |
| Wheat (Spring) | 2 | 0 | Wheat (Spring) | 0 |
| Wheat (Winter) | 3 | 0 | Wheat (Winter) | 0 |
| Wild buckwheat | 0 | 0 | Wild buckwheat | 0 |
| Wild mustard | 0 | 0 | Wild mustard | 0 |
| Wild oat (1) | 2 | 0 | Wild oat (1) | 0 |
| Wild oat (2) | 2 | 0 | Wild oat (2) | 0 |
| Wild radish | 0 | 0 | Wild radish | 0 |
| Winter Barley | 2 | 2 | Winter Barley | 0 |

## Claims

1. Compounds of the Formula I

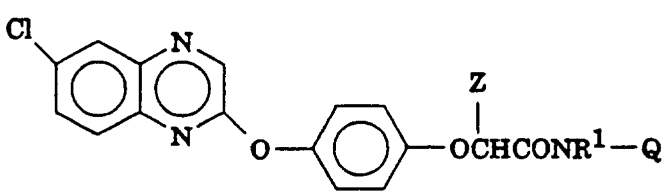

I

wherein:

Q is

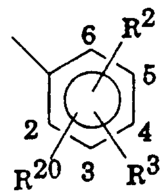 , 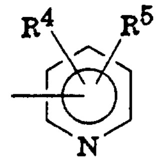 , 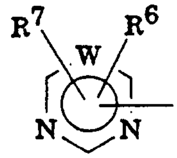 ,

Q-1 Q-2 Q-3

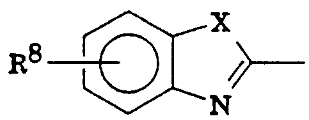 , 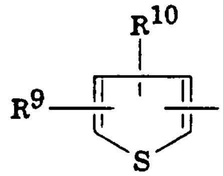 , 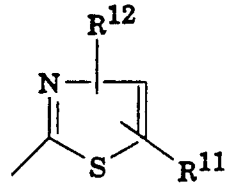 ,

Q-4 Q-5 Q-6

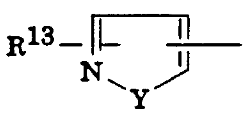 , 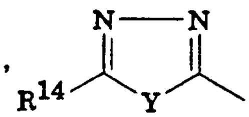 , 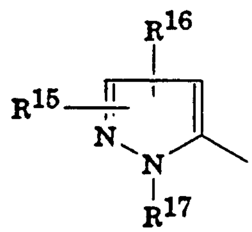 ,

Q-7 Q-8 Q-9

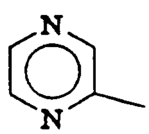 ,

Q-10

$R^1$ is H or $C_1$-$C_4$ alkyl;

$R^2$ is H; halogen; $C_1$-$C_4$ alkyl substituted with up to 3 halogens; $C_1$-$C_3$ alkoxy; $C_1$-$C_4$ haloalkoxy; CN; $CO_2H$; $CO_2(C_1$-$C_4$ alkyl);$S(O)_n(C_1$-$C_3$ alkyl); O-phenyl; $NR^{18}R^{19}$; $SO_2N(CH_3)_2$; or $CH_2CO_2(C_1$-$C_4$ alkyl);

$R^3$ is H, halogen, $C_1$-$C_4$ alkyl substituted with up to 3 halogens or $C_1$-$C_3$ alkoxy;

$R^4$ and $R^5$ are independently H; halogen; $C_1$-$C_4$ alkyl optionally substituted with up to 3 halogens; $C_1$-$C_4$ alkoxy; $C_1$-$C_4$ haloalkoxy; $CO_2H$; $CO_2(C_1$-$C_4$ alkyl); CN; O-phenyl optionally substituted with up to 3 halogens or $CF_3$; or O-benzyl;

$R^6$ and $R^7$ are independently H, $C_1$-$C_4$ alkyl, $C_1$-$C_2$ thioalkyl, $C_1$-$C_3$ alkoxy, $NR^{18}R^{19}$; Cl or Br;

$R^8$ is H, halogen, $C_1$-$C_4$ alkyl optionally substituted with up to 3 halogens, $C_1$-$C_4$ alkoxy, nitro or O-phenyl;

$R^9$ and $R^{10}$ are independently H, $C_1$-$C_4$ alkyl, $CO_2(C_1$-$C_3$ alkyl), phenyl or taken together as $-(CH_2)_4-$ to form a six-membered ring;

$R^{11}$ and $R^{12}$ are independently H; halogen; $C_1$-$C_4$ alkyl optionally substituted with up to 3 halogens or $CO_2(C_1$-$C_2$ alkyl); or phenyl optionally substituted with up to 3 halogens, $CH_3$ or $OCH_3$;

$R^{13}$ is H, $C_1$-$C_4$ alkyl, Cl or Br;

$R^{14}$ is H, $C_1$-$C_4$ alkyl optionally substituted with up to 3 halogens, $S(O)_n(C_1$-$C_4$ alkyl), S-$(O)_n$benzyl or phenyl optionally substituted with up to 3 halogens, $CH_3$ or $OCH_3$;

$R^{15}$ and $R^{16}$ are independently H, $C_1$-$C_4$ alkyl, Cl, Br or $CO_2(C_1$-$C_4$ alkyl);
$R^{17}$ is H; $C_1$-$C_4$ alkyl; or phenyl optionally substituted with up to 3 halogens, $CH_3$ or $OCH_3$;
$R^{18}$ and $R^{19}$ are independently H, $C_1$-$C_3$ alkyl or $C_1$-$C_3$ alkenyl;
$R^{18}$ and $R^{19}$ may be taken together as $-(CH_2CH_2OCH_2CH_2)-$ or $-(CH_2)_5-$;
$R^{20}$ is H or halogen;
W is CH or N;
X is O, S or NH;
Y is O or S;
Z is H or $CH_3$;
n is 0,1 or 2;

provided that:

(a) when $R^2$ is haloalkoxy or $C_1$-$C_2$ alkyl substituted with up to 3 halogens then $R^2$ is in the 2- or 3-position;
(b) when Q is Q-1 and $R^1$ is H then one of $R^2$, $R^3$, or $R^{20}$ must be other than H;
(c) when Q is Q-1 and $R^3$ and $R^{20}$ are H then $R^2$ cannot be $CF_3$;
(d) when Q is Q-1 and $R^2$, $R^3$ or $R^{20}$ is Cl then the remaining substituent cannot be $OCH_3$;
(e) when Q is Q-1 and $R^3$ and $R^{20}$ are H, then $R^2$ cannot be $SCH_3$.
(f) when $R^3$ is $C_1$-$C_2$ alkyl substituted with up to 3 halogens then $R^3$ is in the 2- or 3-position; and
(g) when Q is Q-1 and $R^2$ and $R^{20}$ are H then $R^3$ cannot be $CF_3$.

**2.** The compounds of Claim 1 wherein:
$R^1$ is H or $C_1$-$C_3$ alkyl;
$R^2$ is H; F; Cl; Br; $C_1$-$C_2$ alkyl substituted with up to 3 halogens; $C_1$-$C_2$ alkoxy; $C_1$-$C_2$ haloalkoxy; CN; $S(O)_n(C_1$-$C_2$ alkyl); $CO_2$ ($C_1$-$C_2$ alkyl); O-phenyl; $N(CH_3)_2$; or $SO_2N(CH_3)_2$;
$R^3$ is H, F, Cl, Br, $CF_3$ or $C_1$-$C_2$ alkoxy;
$R^4$ and $R^5$ are independently H, F, Cl, Br, $C_1$-$C_2$ alkyl, $CO_2$-($C_1$-$C_2$ alkyl) or CN.
$R^6$ and $R^7$ are independently H, $C_1$-$C_2$ alkyl, $C_1$-$C_2$ alkoxy, Cl or Br;
$R^8$ is H, F, Cl, or nitro;
$R^9$ and $R^{10}$ are independently H, $C_1$-$C_2$ alkyl, $CO_2CH_3$,phenyl or taken together as $-(CH_2)_4-$ to form a 6-membered ring;
$R^{11}$ and $R^{12}$ are independently H, F, Cl, $CF_3$ or phenyl optionally substituted with F, Cl, $CH_3$ or $OCH_3$;
$R^{13}$ is H, $C_1$-$C_2$ alkyl, Cl or Br;
$R^{14}$ is $C_1$-$C_4$ alkyl, $CF_3$ or $SCH_3$;
$R^{15}$ and $R^{16}$ are independently H, $C_1$-$C_2$ alkyl, Cl or Br;
$R^{17}$ is $C_1$-$C_4$ alkyl, $CF_3$ or phenyl optionally substituted with F or Cl.

**3.** The compounds of Claim 2 wherein
Q is Q-1, Q-2, Q-4, Q-6, Q-8 or Q-9.

**4.** The compounds of Claim 2 wherein
$R^1$ is H or $CH_3$;
$R^2$ is H, F, Cl, Br, $CF_3$, $C_1$-$C_2$ alkoxy, $OCF_3$, $OCH_2CF_3$, $NO_2$, $S(O)_nCH_3$, $CO_2$ ($C_1$-$C_2$ alkyl), O-phenyl or $N(CH_3)_2$;
$R^3$ is H, F, Cl or $OCH_3$;
$R^4$ is H, F, Cl, $CH_3$, $OCH_3$ or $CO_2$ ($C_1$-$C_2$ alkyl);
$R^{14}$ is $C_1$-$C_4$ alkyl or $CF_3$.

**5.** The compounds of Claim 4 wherein
Q is Q-1.

**6.** The compound of Claim 5 which is selected from the group consisting of (R)-2-[4-[(6-chloro-2-quinoxalinyl)oxy]phenoxy]-N-(2,4-difluorophenyl)propanamide, (R)-2-[4-[(6-chloro-2-quinoxalinyl)oxy]-phenoxy]-N-(3,5-dichlorophenyl)propanamide, (R)-N-(4-chloro-2-fluorophenyl)-2-[4-[(6-chloro-2-quinoxalinyl)oxy]-phenoxy]propanamide, (R)-2-[4[(6-chloro-2-quinoxalinyl)oxy]phenoxy]-N-methyl-N-phenylpropanamide, (R)-2-[4-[(6-chloro-2-quinoxalinyl)oxy]phenoxy]-N-(3,4-dichlorophenyl)propanamide, 2-[4-6-chloro-2-quinoxalinyl)oxy]phenoxy]N-(2-fluorophenyl)acetamide, N-(4-chloro-2-fluorophenyl)-2-[4-[(6-chloro-2-quinoxalinyl)oxy]phenoxy]acetamide, (R)-N-(4-bromophenyl)-2-[4-[(6-chloro-2-quinoxalinyl)oxy]-phenoxy]propanamide, N-(4-bromo-2-fluorophenyl)-2-[4-(6-chloro-2-quinoxalinyl)oxy]phenoxy]-propanamide, and N-(4-chloro-2-fluorophenyl)-2-[4-(6-chloro-2-quinoxalinyl)oxy]phenoxy]acetamide.

**7.** An agriculturally suitable composition for controlling the growth of undesired vegetation comprising an effective amount of the compounds of any of Claims 1-6 and at least one of the following: surfactant, solid or liquid diluent.

**8.** A method for controlling the growth of undesired vegetation which comprises applying to the locus to be protected an effective amount of a compound of any of Claims 1-6.

**9.** A method for controlling the growth of undesired vegetation which comprises applying to the rice or corn crop an effective amount of a compound of the following formula:

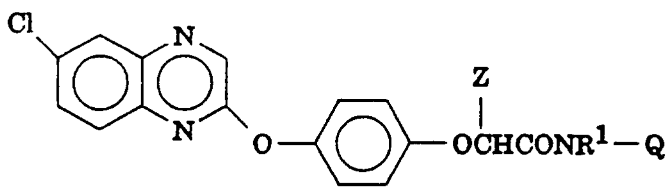

I

wherein:

Q is

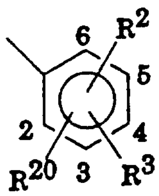
,
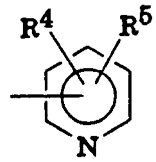
,
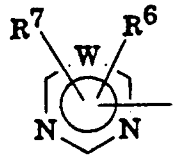
,

Q-1 Q-2 Q-3

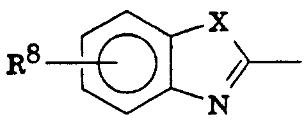
,
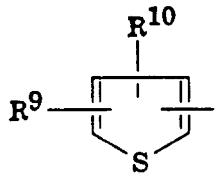
,
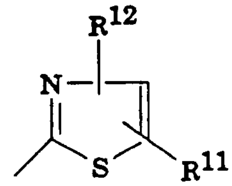
,

Q-4 Q-5 Q-6

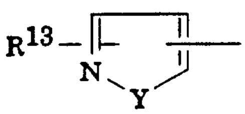
,
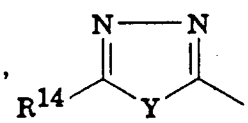
,
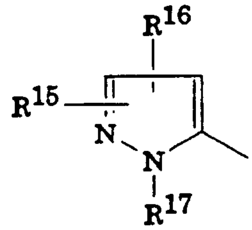
,

Q-7 Q-8 Q-9

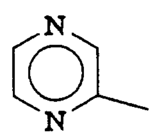
,

Q-10

$R^1$ is H or $C_1$-$C_4$ alkyl;

$R^2$ is H; halogen; $C_1$-$C_4$ alkyl optionally substituted with up to 3 halogens; $C_1$-$C_3$ alkoxy; $C_1$-$C_4$ haloalkoxy; CN; $NO_2$; $CO_2H$; $CO_2$($C_1$-$C_4$ alkyl); $S(O)_n$($C_1$-$C_3$ alkyl); O-phenyl; $NR^{18}R^{19}$; $SO_2N(CH_3)_2$; or $CH_2CO_2$($C_1$-$C_4$ alkyl);

$R^3$ is H, halogen, $C_1$-$C_4$ alkyl optionally substituted with up to 3 halogens, or $C_1$-$C_3$ alkoxy;

$R^4$ and $R^5$ are independently H; halogen; $C_1$-$C_4$ alkyl optionally substituted with up to 3 halogens; $C_1$-$C_4$ alkoxy; $C_1$-$C_4$ haloalkoxy; $CO_2H$; $CO_2$($C_1$-$C_4$ alkyl); CN; O-phenyl optionally substituted with up to 3 halogens or $CF_3$; or O-benzyl;

$R^6$ and $R^7$ are independently H, $C_1$-$C_4$ alkyl, $C_1$-$C_2$ thioalkyl, $C_1$-$C_3$ alkoxy, $NR^{18}R^{19}$; Cl or Br;

$R^8$ is H, halogen, $C_1$-$C_4$ alkyl optionally substituted with up to 3 halogens, $C_1$-$C_4$ alkoxy, nitro or O-phenyl;

$R^9$ and $R^{10}$ are independently H, $C_1$-$C_4$ alkyl, $CO_2$($C_1$-$C_3$ alkyl), phenyl or taken together as $-(CH_2)_4-$ to form a six-membered ring;

$R^{11}$ and $R^{12}$ are independently H; halogen; $C_1$-$C_4$ alkyl optionally substituted with up to 3 halogens or $CO_2$($C_1$-$C_2$ alkyl); or phenyl optionally substituted with up to 3 halogens, $CH_3$ or $OCH_3$;

$R^{13}$ is H, $C_1$-$C_4$ alkyl, Cl or Br;

$R^{14}$ is H, $C_1$-$C_4$ alkyl optionally substituted with up to 3 halogens, $S(O)_n$($C_1$-$C_4$ alkyl), S-$(O)_n$benzyl or phenyl optionally substituted with up to 3 halogens, $CH_3$ or $OCH_3$;

$R^{15}$ and $R^{16}$ are independently H, $C_1$-$C_4$ alkyl, Cl, Br or $CO_2$($C_1$-$C_4$ alkyl);

$R^{17}$ is H; $C_1$-$C_4$ alkyl; or phenyl optionally substituted with up to 3 halogens, $CH_3$ or

$OCH_3$;

$R^{18}$ and $R^{19}$ are independently H, $C_1$-$C_3$ alkyl or $C_1$-$C_3$ alkenyl;

$R^{18}$ and $R^{19}$ may be taken together as $-(CH_2CH_2OCH_2CH_2)-$ or $-(CH_2)_5-$;

$R^{20}$ is H or halogen;

W is CH or N;

X is O, S or NH;

Y is O or S;

Z is H or $CH_3$;

n is 0, 1 or 2;

provided that:

(a) when $R^2$ is haloalkoxy or $C_1$-$C_2$ alkyl substituted with up to 3 halogens then $R^2$ is in the 2 -or 3-position;

(b) when Q is Q-1 and $R^1$ is H then one of $R^2$, $R^3$ or $R^{20}$ must be other than H; and

(c) when $R^3$ is $C_1$-$C_2$ alkyl substituted with up to 3 halogens then $R^3$ is in the 2- or 3-position.

European Patent Office

# EUROPEAN SEARCH REPORT

Application Number
EP 93 10 0536

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 042 750 (E. I. DU PONT DE NEMOURS)<br>* claims *<br>--- | 1,2,7-9 | C07D241/44<br>C07D401/12<br>C07D403/12<br>C07D409/12<br>C07D411/12<br>C07D417/12<br>A01N43/60 |
| D,A | EP-A-0 046 467 (NISSAN CHEMICAL INDUSTRIES)<br>* page 4; claims; table 1 *<br>--- | 1,2,7-9 | |
| D,A | EP-A-0 205 821 (DOW CHEMICAL)<br>*PAGE 9 TO PAGE 11, PAGE 23 TO PAGE 76*<br>--- | 1,2,7-9 | |
| D,A | US-A-4 968 343 (JAMES A. TURNER ET AL.)<br>* the whole document *<br>----- | 1,2,7-9 | |

TECHNICAL FIELDS SEARCHED (Int.Cl.5)

C07D
A01N

The present search report has been drawn up for all claims

| Place of search | Date of completion of the search | Examiner |
|---|---|---|
| THE HAGUE | 4 November 1993 | FRANCOIS, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document